# EUROPÄISCHE PATENTSCHRIFT

(11) **EP 4 045 479 B1**
(45) Veröffentlichungstag und Bekanntmachung des Hinweises auf die Patenterteilung: **26.10.2022**
(21) Anmeldenummer: 21785917.2
(22) Anmeldetag: 06.10.2021
(51) Int. Cl.: C07C 51/06, C07C 57/04, C07C 67/20, C07C 69/54, C07C 231/06, C07C 231/12, C07C 303/24

(54) **OPTIMIERTES VERFAHREN ZUR HERSTELLUNG VON ALKYLMETHACRYLAT DURCH REDUZIERUNG STÖRENDER NEBENPRODUKTE**
OPTIMIZED METHOD FOR PRODUCING ALKYL METHACRYLATE BY REDUCING INTERFERING BY-PRODUCTS
PROCÉDÉ OPTIMISÉ DE FABRICATION DE MÉTHACRYLATE D'ALKYLE PAR RÉDUCTION DES SOUS-PRODUITS INTERFÉRENTS

(30) Priorität: 23.10.2020 EP 20203723
(43) Veröffentlichungstag der Anmeldung: 24.08.2022
(73) Patentinhaber: Röhm GmbH, 64295 Darmstadt (DE)
(72) Erfinder: KRILL, Steffen, 64367 Mühltal (DE); WINGS, Patrick, 50935 Köln (DE); KLASOVSKY, Florian, 45721 Haltern am See (DE); KÖNIG, Daniel, Helmut, 70499 Stuttgart (DE)
(74) Vertreter: Röhm Patent Association
(86) Internationale Anmeldenummer: PCT/EP2021/077488
(87) Internationale Veröffentlichungsnummer: WO 2022/084032

(56) Entgegenhaltungen:
- EP-A1- 1 359 137
- DE-A1-102004 006 826

## Beschreibung

Die vorliegende Erfindung betrifft ein verbessertes Verfahren zur Herstellung von Alkylmethacrylaten, insbesondere Methylmethacrylat (MMA), umfassend die Umsetzung von Acetoncyanhydrin (ACH) und Schwefelsäure in einer ersten Reaktionsstufe (Amidierung) und die Erhitzung der ersten Reaktionsmischung in einer zweiten Reaktionsstufe (Konvertierung), wobei Methacrylsäureamid (MASA) erhalten wird, sowie die anschließende Veresterung von Methacrylsäureamid (MASA) mit Alkohol und Wasser, bevorzugt Methanol und Wasser, in einer dritten Reaktionsstufe (Veresterung) unter Bildung von Alkylmethacrylat, wobei die eingesetzte Schwefelsäure eine Konzentration im Bereich von 98,0 Gew.-% bis 100,0 Gew.-% aufweist, und wobei die anschließende Aufarbeitung der dritten Reaktionsmischung mindestens zwei Destillationsschritte umfasst, bei denen die Nebenprodukte Methacrylnitril (MAN) und Aceton zumindest teilweise in der Kopf-Fraktion als wasserhaltiges Heteroazeotrop erhalten werden, wobei das wasserhaltiges Heteroazeotrop zumindest teilweise aus dem Verfahren ausgeschleust und zumindest teilweise in die dritte Reaktionsstufe zurückgeführt wird.

Insbesondere betrifft die vorliegende Erfindung ein optimiertes Verfahren zur Herstellung von Alkylmethacrylat, umfassend die spezifische Einstellung und Kontrolle der Qualität der Zwischenprodukte und Produkte, insbesondere MASA und MMA, wobei die Bildung störender Nebenprodukte, insbesondere Methacrylnitril (MAN), Aceton, Methylformiat, Propionsäuremethylester und Isobuttersäuremethylester (IBSME), in den Vorstufen und Zwischenprodukten reduziert wird, und die Ausbeute an Zwischenprodukten und Produkten verbessert wird.

### Stand der Technik

Methylmethacrylat (MMA) wird in großen Mengen zur Herstellung von Polymeren und Copolymeren mit anderen polymerisierbaren Verbindungen eingesetzt. Weiterhin ist Methylmethacrylat ein wichtiger Baustein für verschiedene Spezialester, basierend auf dem chemischen Grundkörper Methacrylsäure (MAS), die durch Umesterung vom MMA mit dem entsprechenden Alkohol hergestellt werden können oder durch Kondensation von Methylacrylsäure und einem Alkohol zugänglich sind. Hieraus ergibt sich ein großes Interesse an möglichst einfachen, wirtschaftlichen und umweltschonenden Herstellungsverfahren für diesen Ausgangsstoff.

Die Herstellung von Methacrylsäureamid durch die Amidierung (Hydrolyse) von Acetoncyanhydrin (ACH) ist ein weithin angewandtes Verfahren. Beispielsweise stellt eine derartige Amidierung, welche im Stand der Technik auch als ACH-Hydrolyse bezeichnet wird, einen wichtiger Zwischenschritt bei der Herstellung von Methylmethacrylat gemäß dem sogenannten ACH-Sulfo-Verfahren dar, wobei große Mengen an Schwefelsäure im Prozess eingesetzt werden. Die Herstellung von Methacrylsäure (MAS) und Methylmethacrylat (MMA) nach dem ACH-Sulfo-Verfahren ist allgemein bekannt und im Stand der Technik beschreiben, z.B. WO 2008/068064, WO 2013/143812, EP 0 226 724. Hierbei wird zunächst in einem ersten Schritt Acetoncyanhydrin (ACH) durch Umsetzung von Blausäure und Aceton hergestellt, welches anschließend zum Methacrylsäureamid (im Folgenden auch als Methacrylamid bezeichnet) (MASA) umgesetzt wird. Diese Schritte sind u.a. in US 7,253,307 (s. auch EP 1 359 137), EP 1 666 451 oder EP 2007 059092 dargestellt. Die Umsetzung von ACH zu MASA (Amidierung) wird in einer dem Fachmann bekannten Weise durch eine Reaktion zwischen konzentrierter Schwefelsäure und ACH bewirkt. Die Umsetzung ist exotherm, so dass bevorzugt die Reaktionswärme schnell aus dem System abgeführt wird.

Die Umsetzung zu MASA verläuft typischerweise in zwei Prozessschritten. Zunächst wird im Amidierungsschritt eine schwefelsaure, im Wesentlichen wasserfreie Lösung, enthaltend hauptsächlich alpha-Hydroxyisobuttersäureamid (alpha-Hydroxyisobutyramid) (HIBA), dessen Sulfatester alpha-Sulfoxyisobuttersäureamid (SIBA) und Methacrylsäureamid (MASA) (bzw. salzartig protoniert in Form der jeweiligen Hydrogensulfate), erhalten. Im anschließenden Schritt, der so genannten Konvertierung, wird diese Lösung typischerweise bei hohen Temperaturen von 130°C bis 200 °C und meist kurzen Verweilzeiten, z.B. von etwa 15 min oder weniger, unter β-Eliminierung von Wasser bzw. Schwefelsäure zu Methacrylsäureamid (MASA) umgesetzt. Typischerweise liegt nach der Konvertierung das Hauptprodukt MASA·H₂SO₄ mit einer Konzentration in der Lösung von etwa 30 bis 40 Gew.-% (je nach eingesetztem Schwefelsäureüberschuss) vor.

Die Schritte der Amidierung und der Konvertierung unterscheiden sich in der Regel prozesstechnisch wesentlich in der Verweilzeit und auch im eingesetzten Temperaturniveau. Im Hinblick auf die chemische Reaktion, wird die Amidierung typischerweise kürzer als die Konvertierung und typischerweise bei niedrigeren Temperaturen als die Konvertierung bzw. die nachfolgende Veresterung durchgeführt.

Das Dokument US-B 4,529,816 beschreibt ein Verfahren zur Herstellung von Alkylmethacrylaten nach dem ACH-Sulfo-Verfahren, wobei die Amidierung bei Temperaturen um 100 °C mit weit überstöchiometrischen Mengen an Schwefelsäure (molares Verhältnis von ACH : H₂SO₄ von etwa 1:1,3 bis 1:1,8) durchgeführt wird. Es wird darauf hingewiesen, dass die eingesetzte Schwefelsäure ausreichend SO₃ enthalten sollte, um mindestens eine Säurestärke von 98 %, bevorzugt mindestens 99,5 % zu gewährleisten. Insbesondere soll rauchende Schwefelsäure (Oleum) eingesetzt werden, welche bevorzugt 101 % Schwefelsäure und somit freies SO₃ aufweist. Im nachfolgenden Schritt der Veresterung wird die Reaktionsmischung mit einem Überschuss Wasser und Alkohol bei 100 bis 150 °C behandelt. US-B 4,529,816 beschreibt, dass das Nebenprodukt alpha-Hydroxyisobuttersäuremethylester (HIBSM) nach der Veresterung vom Methylmethacrylat-Produkt abgetrennt und in das Verfahren zurückgeführt wird. In ähnlicher Weise werden in dem Verfahren nach US 5,393,918 Nebenprodukte, unter anderem alpha-Hydroxyisobuttersäuremethylester (HIBSM) isoliert, dehydratisiert und in die Reaktion zurückgeführt. Nachteilig ist hierbei die Notwendigkeit der Isolierung und Rückführung des Nebenproduktes HIBSM. Es wird zudem beschrieben, dass die Konzentration der Schwefelsäure bei der Umsetzung von ACH nicht entscheidend ist.

Das Dokument DE 38 28 253 A1 beschreibt ein Verfahren zur Rückführung verbrauchter Schwefelsäure bei der Herstellung von Methacrylsäureestern nach dem ACH-Sulfo-Verfahren, wobei die verbrauchte Säure nach der Veresterung aufkonzentriert, mit frischer Säure gemischt und zurückgeführt wird. DE 38 28 253 A1 beschreibt allgemein eine Säurestärke von 96 bis 101 % bei der Umsetzung des Acetoncyanhydrin mit Schwefelsäure.

Das Dokument DE 1 618 721 beschreibt die Umsetzung von Acetoncyanhydrin (ACH) mit Schwefelsäure in zwei Stufen mit unterschiedlichem Verhältnis von Schwefelsäure zu ACH, wodurch die Viskosität der Reaktionsmischung geregelt werden soll. In dem in EP 0 226 724 beschriebenen Verfahren wird die Umsetzung in Gegenwart eines Alkan-Lösungsmittels durchgeführt, um die Viskosität der Reaktionsmischung und die Reaktionsenthalpie zu regeln und zu kontrollieren.

Das Dokument CH 239749 beschreibt ein Verfahren zur Herstellung von Methacrylamid durch Einwirkung von Schwefelsäure auf Acetoncyanhydrin bei Temperaturen von 110 bis 130 °C bzw. 115 bis 160 °C, wobei beispielsweise 100 %ige Schwefelsäure eingesetzt wird.

US-B 4,748,268 beschreibt ein Verfahren zur Veresterung von Methacrylsäure mit einem C₁-C₄ Alkohol in Gegenwart einer hochsiedenden organischen Flüssigkeit in einem Plug-Flow-Reaktor, bei dem das Reaktionsgemisch kontinuierlich fraktioniert wird, wobei der Destillatstrom einen höheren Anteil an Methacrylsäureester aufweist und der Sumpfstrom zum überwiegenden Teil in den Plug-Flow-Reaktor zurückgeführt wird.

Als Nebenprodukte bei der Amidierung und Konvertierung werden unter anderem Kohlenstoffmonoxid, Aceton, Sulfonierungsprodukte des Acetons und Cyclokondensationsprodukte von Aceton mit diversen Intermediaten gebildet. Diese genannten Nebenprodukte können meist relativ effektiv vom Alkylmethacrylat-Produkt abgetrennt werden. Darüber hinaus bilden sich allerdings auch - in Abhängigkeit der Reaktionsbedingungen - andere Nebenprodukte, deren Abtrennung vom Alkylmethacrylat, insbesondere vom Methylmethacrylat-Produkt, schwierig beziehungsweise mit erheblichem Trennaufwand verbunden ist. Beispielsweise gestaltet sich die Trennung aufgrund der Azeotrop-Siedepunkte und der Siedepunkte der spezifischen Verbindungen schwierig. Störende Nebenprodukte sind insbesondere Methacrylnitril (MAN), Aceton, Isobuttersäuremethylester (IBSME) und Propionsäuremethylester (Methylpropionat, MP) sowie Diacetyl (Di-Ac, Butan-2,3-dion). Insbesondere Methacrylnitril (MAN) und Aceton sind relevante störende Nebenprodukte, deren Aufkonzentrierung in den Rezyklier-Strömen kontrolliert werden muss, um einen gleichbleibende Produktqualität in kontinuierlichen Betrieb zu gewährleisten.

Einige dieser störenden Nebenprodukte sind maßgeblich für eine erhöhte Farbzahl im Alkylmethacrylat-Endprodukt, insbesondere MMA, verantwortlich. Die störenden niedermolekularen Nebenprodukte können zudem Probleme bei der weiteren Polymerisation und Verarbeitung der Polymere machen, z.B. durch Ausgasen bei der Extrusion oder im Spitzguss. Störende Nebenprodukte mit einer Doppelbindung werden neben dem Alkylmethacrylat ins Polymerprodukt einpolymerisiert und beeinträchtigen die Eigenschaften der Polymere, z.B. die Transparenz und das Trübungsverhalten beim Einsatz in luftfeuchter Umgebung, negativ. Um spezifikationsgerechtes Alkylmethacrylat-Endprodukt, insbesondere MMA, zu erhalten, müssen diese Nebenprodukte, wie MAN, IBSME und / oder MP, in den Reaktionsschritten reduziert oder in der Aufarbeitung entfernt werden.

Methacrylnitril (MAN) bildet sich typischerweise als Nebenprodukt während der Amidierungsreaktion aus Acetoncyanhydrin (ACH) unter Eliminierung von Wasser. 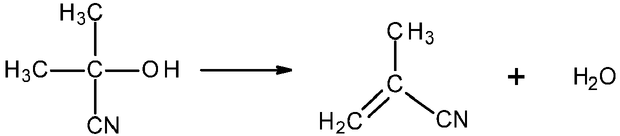 Methacrylnitril (MAN) bildet sowohl mit Methanol (MeOH) als auch mit Methylmethacrylat (MMA) ein Azeotrop bzw. hat einen ähnlichen Siedepunkt wie einige Azeotrope des Methylmethacrylats und ist daher meist schwer und nur unter erheblichem Aufwand aus dem Produkt abzutrennen. Eine vollständige Abtrennung von MAN ist mit vernünftigem Aufwand in der Regel nicht möglich.

Bei Analyse von kommerziellen MMA-Qualitäten basierend auf der ACH-Sulfo-Technologie fällt daher auf, dass eine solche Produktqualität immer mehr oder weniger geringe Spuren an MAN aufweist, üblicherweise in einer Größenordnung zwischen 10 und 500 ppm (0,05 Gew.-% neben MMA mit einem Gehalt größer gleich 99,9 Gew.-%). Auch stellt die gleichzeitige Anwesenheit von Wasser, Methanol und Aceton sowie verschiedener Nebenprodukte eine besondere Herausforderung dar, da bei destillativer Abtrennung oftmals Methanol und Aceton als Phasenvermittler die Ausbildung von reinen Trennphasen in bestimmten Konzentrationsbereichen und / oder bei bestimmten physikalischen Bedingungen (Druck, Temperatur) stören. Somit wird die Ausbildung reiner Trennphasen oftmals verhindert, so dass nach der Phasentrennungen häufig weitere Trenn- und /oder Aufarbeitungsschritte notwendig sind. Gemäß Stand der Technik werden bei der Aufarbeitung nach dem ACH-Sulfo-Verfahren meist eine organische MMA-haltige Phase und eine wässrige Methanolhaltige Phase erhalten, welche jeweils beide die genannten phasenvermittelnden Stoffe, insbesondere Aceton und Methanol, enthalten, so dass weitere aufwändige verfahrenstechnische Operationen eingesetzt werden müssen.

Bei der Amidierung werden als gewünschte Hauptprodukte der Umsetzung Sulfoxyisobuttersäureamid-Hydrogensulfat (SIBA·H₂SO₄) und Methacrylsäureamid-Hydrogensulfat (MASA·H₂SO₄) als Lösung in überschüssiger Schwefelsäure erhalten. Des Weiteren ist typischerweise in der Amidierungslösung noch alpha-Hydroxyisobuttersäureamid-Hydrogensulfat (HIBA·H₂SO₄) erhalten, beispielsweise mit einer Ausbeute bezüglich ACH von < 5 %. Bei annähernd vollständigem Umsatz von ACH kann im Amidierungsschritt eine Ausbeute an den oben beschriebenen Intermediate von typischerweise etwa 94 % bis 96 % erhalten werden. In diesem Schritt werden oftmals in erheblichen Mengen die oben beschriebenen Nebenprodukte gebildet. Grundsätzliche Nebenreaktion dieses Verfahrensschrittes ist der ACH-Zerfall in der Reaktionsmatrix, der grundsätzlich auch von der Amidierungstemperatur abhängt. Bei diesem Zerfall wird ausgehend von HCN Kohlenmonoxid gebildet, das aus der Reaktionslösung ausgast. Ebenfalls gebildetes Aceton wird sulfoniert und bildet beispielsweise Acetondisulfonsäure (ADSA) und hiervon abgeleitete Salze.

Aus der Ammoniumhydrogensulfat- und Schwefelsäure-haltigen Prozesssäure, welche oftmals in einer Schwefelsäurekontaktanlage regeneriert wird, scheiden sich zudem teerartige, feste Kondensationsprodukte ab, die eine Förderung der Prozesssäure behindern und unter erheblichem Aufwand beseitigt und entsorgt werden müssen. Zusammenhänge zwischen Teerbildung und Reaktionsbedingungen werden im Stand der Technik nicht beschrieben, und es fehlen somit beispielsweise Hinweise, wie Menge und Konsistenz der Teerbildung im Sinne einer störungsfreien Prozessführung positiv beeinflusst werden können.

Es ist außerdem bekannt, dass Hydroxyisobuttersäure ausgehend von Acetoncyanhydrin (ACH) durch Verseifung der Nitrilfunktion in Gegenwart von Mineralsäuren hergestellt werden kann. Im Stand der Technik werden Verfahren beschrieben, bei denen ACH in Gegenwart von Wasser amidiert und hydrolysiert wird, wobei die Hydroxy-Funktion im Molekülverbund zumindest in den ersten Schritten der Umsetzung erhalten bleibt, z.B. WO 2005/077878, JP H04 193845 A, JP S57 131736. Beispielsweise wird in der japanischen Patentanmeldung JP S63-61932 beschrieben, dass ACH in einem zweistufigen Prozess zur Hydroxyisobuttersäure verseift wird, wobei ACH zunächst in Gegenwart von 0,2 bis 1,0 mol Wasser und 0,5 bis 2 Äquivalenten Schwefelsäure umgesetzt wird, wobei die entsprechenden Amidsalze gebildet werden. Diese Vorschläge zu einer alternativen Amidierung in Gegenwart von Wasser führen je nachdem, ob in Gegenwart von Methanol oder ohne Methanol durchgeführt, entweder zur Entstehung von Hydroxyisobuttersäuremethylester (HIBSM) oder zur Entstehung von 2-Hydroxyisobuttersäure (HIBS). Beispielsweise erfolgt gemäß JP S57 131736 die Umsetzung von ACH mit 0,8 bis 1,25 Äquivalenten Schwefelsäure in Gegenwart von weniger als 0,8 Äquivalenten Wasser unterhalb von 60 °C und anschließend die Umsetzung mit mehr als 1,2 Äquivalenten Methanol zum HIBSM bei Temperaturen von größer als 55°C.

Keines der Verfahren im Stand der Technik beschreibt eine Möglichkeit zur Kontrolle und Reduzierung von störenden Nebenprodukten, insbesondere von Methacrylnitril (MAN) und Aceton, welche die Eigenschaften des Alkylmethacrylats und der Alkylmethacrylat-Polymere in besonderem Maße beeinträchtigen und darüber hinaus die Gesamtausbeute des Verfahrens reduzieren, da ihre effektive Abtrennung von MMA immer auch mit Isolierverlusten an MMA einhergeht.

Insbesondere enthalten kommerzielle MMA-Qualitäten mit einer Reinheit von typischerweise etwa 99,8 Gew.-% verfahrensspezifisch und typisch für die ACH-Sulfo-Technologie neben MMA auch Methacrylnitril (MAN) im Spurenbereich, was analytisch bestimmt werden kann. In der Regel ist MAN in Mengen von einigen ppm bis einigen hundert ppm vorhanden. Oftmals liegt der MAN-Gehalt in der Größenordnung von 30 bis 200 ppm und unterliegt von Charge zu Charge gewissen Schwankungen. Zusammenfassend lässt sich feststellen, dass im Stand der Technik keine Hinweise darüber gegeben werden, welche Zusammenhänge zur Entstehung der unerwünschten Nebenprodukte führen, bzw. wie diese reduziert werden können. Dem Fachmann ist zwar grundsätzlich bekannt, wie MAN destillativ von reinem MMA getrennt werden kann, allerdings ist dies nur unter Beeinträchtigung der Ausbeute möglich bzw. erfordert einen erhöhten apparativen Aufwand. Es besteht somit ein hoher Bedarf an einer technischen Möglichkeit, die Entstehung dieser störenden Nebenprodukte bereits in der Reaktion zu beeinflussen und dort zu reduzieren, so dass sich die nachfolgenden Trennschritte vereinfachen. Wünschenswert wären auch Verfahren, die es erlauben, die MAN-haltigen Stoffströme zu werthaltigen Produkten im Sinne der MMA-Produktion umzuwandeln und / oder effektiv aus dem Verfahren auszuschleusen.

### Aufgabe der Erfindung

Aufgabe der Erfindung ist es, die oben genannten Nachteile zu überwinden und ein verbessertes Verfahren zur Herstellung von Alkylmethacrylaten basierend auf dem ACH-Sulfo-Verfahren bereitzustellen, bei welchem die Menge an störenden Nebenprodukten, insbesondere an Methacrylnitril (MAN) und Aceton, reduziert werden kann. Hierdurch soll die Produktqualität des Alkylmethacrylats sowie der daraus hergestellten Polymere und Formkörper verbessert werden. Insbesondere sollen die Verarbeitbarkeit sowie die mechanischen und optischen Eigenschaften der Alkylmethacrylat-Polymere verbessert werden.

Außerdem soll im Vergleich zu bekannten Verfahren eine vergleichbare oder erhöhte Ausbeute an Alkylmethacrylat erhalten werden, wobei beispielsweise gleichzeitig Verluste an Alkylmethacrylat bei der Aufarbeitung sowie Abfallströme reduziert werden. Weitere Aufgabe der Erfindung war es, ein Verfahren zur Verfügung zu stellen, dass es erlaubt MAN-haltige Aufarbeitungsströme kontinuierlich und im Kreislaufbetrieb derart zu behandeln, dass MAN zu MASA hydrolysiert und somit wieder zum gewünschten Produkt MMA umgesetzt werden kann.

### Lösung der Aufgabe

Die Aufgabe wurde dadurch gelöst, dass im erfindungsgemäßen Verfahren einerseits die Bildung der genannten Nebenprodukte durch eine optimierte Reaktionsführung bei der Alkylmethacrylat-Synthese minimiert wird, und andererseits störende Nebenprodukte durch spezifische chemische Senken im Aufarbeitungsteil möglichst frühzeitig aus dem Verfahren abgeführt werden und somit nicht in das Endprodukt gelangen. Chemische Senken sind Reaktionen, bei denen ein Nebenprodukt bei seiner Rückführung derart stofflich umgewandelt wird, dass ein stetiges Aufschaukeln (stetige Konzentrationserhöhung) im kontinuierlichen Betrieb unterdrückt wird. Im besten Fall wird eine solche chemische Senke gefunden, die das Nebenprodukt wieder ins gewünschte Zielprodukt umwandelt.

Es wurde überraschend gefunden, dass die oben genannten Aufgaben durch das erfindungsgemäße Verfahren gelöst werden. Insbesondere wurde gefunden, dass die Menge an störenden Nebenprodukten, insbesondere Methacrylnitril (MAN), Aceton, Methylpropionat und / oder Methylisobutyrat, reduziert werden kann, wenn bei der Umsetzung von Acetoncyanhydrin (in Amidierung und Konvertierung) Schwefelsäure verwendet wird, die kein freies SO₃, insbesondere aber geringe Anteile freies Wasser enthält. Besonders vorteilhaft hat sich der Einsatz einer Schwefelsäure mit einer Konzentration im Bereich von 98,0 Gew.-% bis 100,0 Gew.-%, bevorzugt 99,0 Gew.-% bis 99,9 Gew.-%, erwiesen.

Es wurde zudem gefunden, dass die störenden Nebenprodukte, insbesondere Methacrylnitril (MAN) und Aceton, durch eine optimierte Aufarbeitung der Reaktionsmischung nach der Veresterung, umfassend eine geeignete Ausschleusung und eine optimierte Kreislaufführung von Verfahrensströmen, effektiv im erforderlichen Maß aus dem Verfahren ausgeschleust werden können. Insbesondere wurde gefunden, dass sich eine effektive Ausschleusung von Methacrylnitril (MAN) und Aceton realisieren lässt, indem in mindestens einem Azeotrop-Destillationsschritt die Nebenprodukte zumindest teilweise in der Kopf-Fraktion als wasserhaltiges Heteroazeotrop erhalten und hierüber, gegebenenfalls nach weiteren Trennschritten, zumindest teilweise aus dem Verfahren ausgeschleust werden. Gegebenenfalls kann das Heteroazeotrop enthaltend die störenden Nebenprodukte in eine wässrige und eine organische Phase aufgetrennt werden, wobei die wässrige und / oder die organische Phase zumindest teilweise aus dem Verfahren ausgeschleust werden. Hierbei ist es überraschenderweise gelungen, die störenden Nebenprodukte zusammen mit solchen Stoffströmen aus dem Verfahren zu entfernen, in denen eine Anreicherung der störenden Nebenprodukte aufgrund ihrer physikochemischen Eigenschaften (insbesondere Wasserlöslichkeit und Flüchtigkeit) nicht zu erwarten ist. Weiterhin gelingt es durch Kombination mehrfacher Destillations- und Extraktionsschritte, die störenden Nebenprodukte, insbesondere Aceton und Methacrylnitril, als Derivate aus dem Verfahren auszuschleusen (z.B. Aceton in sulfonierter Form) oder zum Zielprodukt umzuwandeln (z.B. MAN über MASA in MMA).

Insgesamt gelingt es mit Hilfe des erfindungsgemäßen Verfahrens, das technische ACH-Sulfo-Verfahren robuster, weniger störungsanfällig und mit höheren Ausbeuten durchzuführen, wobei die Abtrennung von Alkylmethacrylaten in der erforderlichen Qualität effektiv möglich ist.

### Beschreibung der Erfindung

Die vorliegende Erfindung betrifft ein Verfahren zur Herstellung von Alkylmethacrylat, bevorzugt Methylmethacrylat, umfassend
a. die Umsetzung von Acetoncyanhydrin und Schwefelsäure in ein oder mehreren Reaktoren I in einer ersten Reaktionsstufe (Amidierung) bei einer Temperatur im Bereich von 70 bis 130 °C, wobei eine erste Reaktionsmischung, enthaltend Sulfoxyisobuttersäureamid und Methacrylsäureamid, erhalten wird;
b. das Konvertieren der ersten Reaktionsmischung, umfassend das Erhitzen auf eine Temperatur im Bereich von 130 bis 200 °C, bevorzugt 130 bis 170 °C, in ein oder mehreren Reaktoren II in einer zweiten Reaktionsstufe (Konvertierung), wobei eine zweite Reaktionsmischung, enthaltend überwiegend Methacrylsäureamid und Schwefelsäure, erhalten wird;
c. die Umsetzung der zweiten Reaktionsmischung mit Alkohol und Wasser, bevorzugt Methanol und Wasser, in ein oder mehreren Reaktoren III in einer dritten Reaktionsstufe (Veresterung), wobei eine dritte Reaktionsmischung, enthaltend Alkylmethacrylat, bevorzugt Methylmethacrylat, erhalten wird;
d. und die Abtrennung von Alkylmethacrylat aus der dritten Reaktionsmischung erhalten aus der dritten Reaktionsstufe;

wobei die in der ersten Reaktionsstufe eingesetzte Schwefelsäure eine Konzentration im Bereich von 98,0 Gew.-% bis 100,0 Gew.-%, bevorzugt 99,0 Gew.-% bis 99,9 Gew.-%, aufweist;
wobei die Abtrennung von Alkylmethacrylat aus der dritten Reaktionsmischung mindestens zwei Destillationsschritte umfasst, bei denen die Nebenprodukte Methacrylnitril und Aceton zumindest teilweise in der Kopf-Fraktion als wasserhaltiges Heteroazeotrop erhalten werden,
wobei das wasserhaltige Heteroazeotrop, enthaltend Methacrylnitril und Aceton, aus mindestens einem dieser Destillationsschritte zumindest teilweise aus dem Verfahren ausgeschleust wird,
und wobei mindestens ein Strom, enthaltend Methacrylnitril und Aceton, zumindest teilweise in die dritte Reaktionsstufe zurückgeführt wird.

Im Sinne der vorliegenden Erfindung meint der Ausdruck "ppm", ohne weitere Angaben, Gew.-ppm (z.B. mg/kg).

Der Ausdruck Strom, Phase oder Fraktion enthalten ein Edukt, Produkt und / oder Nebenprodukt ist im Sinne der Erfindung so zu verstehen, dass die genannte(n) Verbindung(en) in dem jeweiligen Strom enthalten ist (sind), beispielsweise ist der überwiegende Anteil des Edukts, Produkts und / oder Nebenprodukts in dem entsprechenden Strom zu finden. Grundsätzlich können neben den genannten Verbindungen weitere Bestandteile enthalten sein. Oftmals dient die Nennung der Bestandteile der Verdeutlichung des jeweiligen Verfahrensschritts.

Der Ausdruck Brüden oder Brüdenstrom bezeichnet im Sinne der Erfindung einen gasförmigen Verfahrensstrom, beispielsweise einen gasförmigen Kopfstrom einer Destillationskolonne.. Bevorzugt wird ein gasförmiger Brüdenstrom nach Kontakt mit einer Kühleinrichtung, z.B. einem Kondensator, verflüssigt und kann dann je nach Zusammensetzung ein oder mehrere flüssige Phasen ausbilden, üblicherweise eine wässrige Phase und eine überwiegend organische Phase.

### Erste Reaktionsstufe (Amidierung)

Das erfindungsgemäße Verfahren umfasst als Schritt a die Umsetzung von Acetoncyanhydrin und Schwefelsäure in ein oder mehreren Reaktoren I in einer ersten Reaktionsstufe (Amidierung) bei einer Temperatur im Bereich von 70 bis 130°C, bevorzugt 70 bis 120 °C, besonders bevorzugt 80 bis 110 °C, wobei eine erste Reaktionsmischung, enthaltend Sulfoxyisobuttersäureamid und Methacrylsäureamid, erhalten wird.

Erfindungsgemäß weist die in der ersten Reaktionsstufe eingesetzte Schwefelsäure eine Konzentration im Bereich von 98,0 Gew.-% bis 100,0 Gew.-%, bevorzugt von 99,0 Gew.-% bis 99,9 Gew.-%, bevorzugt von 99,3 bis 99,9 Gew.-%, insbesondere bevorzugt von 99,3 bis 99,8 Gew.-% auf. Insbesondere bezieht sich die angegebene Konzentration der eingesetzten Schwefelsäure auf die Gesamtmasse des Schwefelsäure-Zuleitungsstroms (z.B. (2)). Der Einsatz einer Schwefelsäure ohne Anteil an freiem SO₃, insbesondere einer Schwefelsäure mit einem Wasseranteil von 0,1 bis 0,5 Gew.-% hat sich als besonders vorteilhaft erwiesen. Insbesondere konnte hierdurch die Amidierungsausbeute erhöht und der Anteil an Nebenprodukten, insbesondere MAN und Aceton, insbesondere bereits in der Bildungsreaktion bzw. in verschiedenen Reaktionsstufen, vermindert werden.

Dem Fachmann sind grundsätzlich Methoden zur Bestimmung des Wassergehaltes von Stoffströmen, beispielsweise von Schwefelsäure-Zuleitungsströmen, bekannt. Beispielsweise kann der Wassergehalt von Stoffströmen durch Massenbilanzen, durch Messung der Dichte oder Schallgeschwindigkeit, gaschromatographisch, durch Karl-Fischer-Titration oder mittels HPLC ermittelt werden.

Das eingesetzte Acetoncyanhydrin (ACH) kann mittels bekannter technischer Verfahren hergestellt werden (siehe beispielsweise Ullmanns Enzyklopädie der technischen Chemie, 4. Auflage, Band 7). Typischerweise werden Blausäure und Aceton in Gegenwart eines basischen Katalysators, z.B. eines Amins oder Alkalihydroxids, in einer exothermen Reaktion zu ACH umgesetzt. Eine solche Verfahrensstufe ist beispielsweise in DE 10 2006 058 250 und DE 10 2006 059 511 beschrieben.

Typischerweise entstehen bei der Umsetzung (Amidierung) von Acetoncyanhydrin (ACH) und Schwefelsäure als Hauptprodukte alpha-Hydroxyisobuttersäureamid (HIBA) bzw. dessen Hydrogensulfat (HIBA·H₂SO₄), Schwefelsäureester von alpha-Hydroxyisobuttersäureamid (Sulfoxyisobuttersäureamid (SIBA)) bzw. dessen Hydrogensulfat (SIBA·H₂SO₄) und Methacrylsäureamid-Hydrogensulfat (MASA·H₂SO₄) als Lösung in überschüssiger Schwefelsäure.

In einer bevorzugten Ausführungsform weist die Reaktionsmischung aus Acetoncyanhydrin (ACH) und Schwefelsäure in der ersten Reaktionsmischung eine Gesamtwassermenge im Bereich von 0,1 mol-% bis 20 mol-%, insbesondere 0,4 mol-% bis 10 mol-%, auf, bezogen auf das gesamte der ersten Reaktionsstufe zugeführte ACH.Bevorzugt wird in der ersten Reaktionsstufe Acetoncyanhydrin (ACH) eingesetzt, wobei das ACH bzw. die zugeführten ACH-Stoffströme (z.B. (1a) und / oder (1b)) einen Acetongehalt von kleiner oder gleich 9.000 ppm, bevorzugt von kleiner oder gleich 1.000 ppm, aufweisen, bezogen auf die Gesamtmenge an ACH, die der ersten Reaktionsstufe zugeführt wird. Bevorzugt weist das eingesetzte ACH, bzw. die zugeführten ACH-Stoffströme (z.B. (1a) und / oder (1b)) einen ACH-Anteil von größer oder gleich 98 Gew.-%, besonders bevorzugt größer oder gleich 98,5 Gew.-%, insbesondere bevorzugt größer oder gleich 99 Gew.-%, auf, bezogen auf die zugeführten ACH-Stoffströme. Typischerweise enthält der zugeführte ACH-Stoffstrom (z.B. (1a) und/oder (1b)) 98,0 bis 99,8 Gew.-%, bevorzugt 98,3 bis 99,3 Gew.-%, Acetoncyanhydrin; 0,1 bis 1,5 Gew.-%, bevorzugt 0,2 bis 1 Gew.-%, Aceton, und 0,1 bis 1,5 Gew.-%, bevorzugt 0,3 bis 1 Gew.-%, Wasser, bezogen auf den ACH-Stoffstrom.

Bevorzugt wird in der ersten Reaktionsstufe Acetoncyanhydrin (ACH) eingesetzt, wobei das ACH bzw. die zugeführten ACH-Stoffströme (z.B. (1a) und/oder (1b)) einen Wassergehalt von 0,1 mol-% bis 10 mol-%, insbesondere 0,4 mol-% bis 5 mol-%, bezogen auf das in den zugeführten ACH-Stoffströme enthaltene ACH, aufweist.

Bevorzugt wird eine ACH Qualität eingesetzt, die neben dem Reinstoff noch den Katalysator enthält, allerdings ist der Katalysator durch eine Brönstedt-Säure, bevorzugt Schwefelsäure, neutralisiert. Üblicherweise beträgt der pH des als Feedstrom eingesetzten ACH zwischen pH 2 und pH 6. Auch Spuren von HCN können im ACH vorhanden sein, allerdings wird der Gehalt an HCN so kontrolliert, dass die Konzentration an HCN im ACH 2000 ppm nicht übersteigt, bevorzugt nicht 1000 ppm übersteigt, besonders bevorzugt, wird der HCN Gehalt so kontrolliert, dass er zwischen 100 ppm und 800 ppm beträgt. Dies kann durch Strippen und Destillation erfolgen, wobei ACH als Sumpfstrom von HCN weitestgehend in den beschriebenen Grenzen befreit wird.

Bevorzugt wird die erste Reaktionsstufe mit einem Überschuss an Schwefelsäure betrieben. Die Schwefelsäure dient bevorzugt als Lösemittel. Gleichzeitig dient die Schwefelsäure als Reaktant für die Herstellung des Zwischenproduktes SIBA und als Katalysator für die Amidierung. Der Schwefelsäure-Überschuss kann insbesondere dazu dienen, die Viskosität der Reaktionsmischung niedrig zu halten, wodurch eine schnellere Abfuhr von Reaktionswärme und eine niedrigere Temperatur der Reaktionsmischung gewährleistet werden können. Dies kann insbesondere deutliche Ausbeutevorteile mit sich bringen. Obwohl Viskosität und Lösungsvermögen mit mehr Schwefelsäure verbessert werden, was letztlich eine erhöhte Ausbeute und Selektivität mit sich bringt, ist die Obergrenze der Menge an eingesetzter Schwefelsäure aus wirtschaftlichen Gründen beschränkt, da die resultierende Abfallsäuremenge recycliert oder weiterverarbeitet werden muss.

Bevorzugt werden Schwefelsäure und Acetoncyanhydrin (ACH) in der ersten Reaktionsstufe, der Amidierung, in einem molaren Verhältnis von Schwefelsäure zu ACH im Bereich von 1,2 bis 2; bevorzugt 1,25 bis 1,6; besonders bevorzugt von 1,4 bis 1,45 eingesetzt. Bevorzugt werden zwei oder mehrere Reaktoren I in der ersten Reaktionsstufe verwendet, wobei Schwefelsäure und Acetoncyanhydrin (ACH) im ersten Reaktor I in einem molaren Verhältnis von Schwefelsäure zu ACH im Bereich von 1,6 bis 3; bevorzugt 1,7 bis 2,6; besonders bevorzugt von 1,8 bis 2,3; eingesetzt werden, und wobei Schwefelsäure und Acetoncyanhydrin (ACH) im letzten Reaktor I (beispielsweise im zweiten Reaktor I) in einem molaren Verhältnis von Schwefelsäure zu ACH im Bereich von 1,2 bis 2,0; bevorzugt von 1,2 bis 1,8; insbesondere bevorzugt von 1,3 bis 1,7; eingesetzt werden. Als optimaler Kompromiss zwischen erzielbarer Ausbeute und Schwefelsäureverbrauch erweist sich ein bevorzugtes molares Verhältnis von 1,25 bis 1,6, was letztlich unter wirtschaftlichen Optimierungsaspekten bewertet werden kann. Typischerweise erhöht mehr Schwefelsäure die Kosten für diesen Einsatzstoff und den Aufwand für die Entsorgung der resultierenden Abfallsäuremischung, allerdings kann in der Regel die auf ACH bezogene Ausbeute noch einmal leicht erhöht werden.

Die Umsetzung von Acetoncyanhydrin mit Schwefelsäure in der ersten Reaktionsstufe ist exotherm. Daher ist es vorteilhaft die anfallende Reaktionswärme weitgehend oder zumindest teilweise abzuführen, beispielsweise mit Hilfe geeigneter Wärmetauscher, um eine verbesserte Ausbeute zu erhalten. Da mit sinkender Temperatur die Viskosität der Reaktionsmischung stark ansteigt und so Zirkulation, Durchfluss und Wärmeaustausch in den Reaktoren I erschwert sind, ist eine zu starke Abkühlung allerdings zu vermeiden. Darüber hinaus kann es bei niedrigen Temperaturen in der ersten Reaktionsmischung zu einer partiellen oder vollständigen Kristallisation von Inhaltsstoffen an den Wärmetauschern kommen, was zu Abrasion, beispielsweise in den Pumpengehäusen, Rohrleitungen und den Wärmetauscher-Rohren der Reaktoren I führen kann. Diese sogenannte Sulfatierung ist bevorzugt unbedingt zu vermeiden, da man gezwungen ist, die Anlage abzustellen und den Reaktor zu reinigen.

Zur Kühlung der Reaktorkreisläufe können grundsätzlich bekannte und geeignete Kühlmedien eingesetzt werden. Vorteilhaft ist der Einsatz von Kühlwasser. Typischerweise weist das Kühlmedium, insbesondere das Kühlwasser, eine Temperatur unterhalb der gewählten Verfahrensbedingungen auf. Vorteilhaft weist das Kühlmedium, insbesondere das Kühlwasser, eine Temperatur im Bereich von 20 bis 90°C, bevorzugt von 50 bis 90°C und besonders bevorzugt von 70 bis 80°C auf.

Zur Vermeidung von Unterschreitung des Kristallisationspunktes von Methacrylsäureamid wird der Wärmetauscher (Reaktorkühler) typischerweise mit einem Warmwasser-Sekundärkreislauf betrieben. Hierbei sind Temperaturdifferenzen im produktseitigen Ein-/Austritt des Apparates von etwa 1 bis 20° C, insbesondere 2 bis 10°C bevorzugt.

Die Umsetzung von Acetoncyanhydrin und Schwefelsäure in ein oder mehreren Reaktoren I in einer ersten Reaktionsstufe (Amidierung) erfolgt bei einer Temperatur im Bereich von 70 bis 130°C, bevorzugt von 80 bis 120 °C, besonders bevorzugt von 90 bis 110°C. Oftmals wird die Amidierung in der erste Reaktionsstufe im Reaktor I oder in mehreren Reaktoren I bei Normaldruck oder leichtem Unterdruck durchgeführt.

Typischerweise kann die erste Reaktionsstufe (Amidierung) batchweise und / oder kontinuierlich durchgeführt werden. Typischerweise kann die erste Reaktionsstufe in einem Rührkessel, einer Rührkesselkaskade oder einem Schlaufenreaktor bzw. einer Kombination dieser Apparate ausgeführt sein. Bevorzugt wird die erste Reaktionsstufe kontinuierlich, beispielsweise in einem oder in mehreren Schlaufenreaktoren, durchgeführt. Geeignete Reaktoren und Verfahren sind beispielsweise in WO 2013/143812 beschrieben. Vorteilhaft kann die erste Reaktionsstufe in einer Kaskade von zwei oder mehreren Schlaufenreaktoren durchgeführt werden. Insbesondere bevorzugt erfolgt die Umsetzung der ersten Reaktionsstufe in einem oder in mehreren (bevorzugt zwei) Schlaufenreaktoren.

Der erste Schlaufenreaktor wird typischerweise bei einem Kreislaufverhältnis (Verhältnis aus Umwälzvolumenstrom zu Zulaufvolumenstrom) im Bereich von 5 bis 110, bevorzugt bei 10 bis 90, besonders bevorzugt bei 10 bis 70 betrieben. In einem nachfolgenden Schlaufenreaktor liegt das Kreislaufverhältnis vorzugsweise in einem Bereich von 5 bis 100, bevorzugt von 10 bis 90, besonders bevorzugt von 10 bis 70.

Typischerweise liegt die statische Verweilzeit in den Reaktoren I, insbesondere in den Schlaufenreaktoren I, im Bereich von 5 bis 35 Minuten, bevorzugt von 8 bis 20 Minuten.

Ein geeigneter Schlaufenreaktor weist bevorzugt die folgenden Elemente auf: eine oder mehrere Zugabestellen für ACH, eine oder mehrere Zugabestellen für Schwefelsäure, einen oder mehrere Gasabscheider, einen oder mehrere Wärmetauscher, einen oder mehrere Mischer, und eine Pumpe. Die Mischer sind häufig als statische Mischer ausgeführt.

Die Zugabe des ACH kann grundsätzlich an beliebiger Stelle in den einen oder in die mehreren Rektoren I (z.B. Schlaufenreaktoren) erfolgen. Es hat sich jedoch als vorteilhaft erwiesen, wenn die Zugabe des ACH an einer gut durchmischten Stelle erfolgt. Bevorzugt erfolgt die Zugabe des ACH in ein Mischelement, beispielsweise in einen Mischer mit beweglichen Teilen oder in einen statischen Mischer.

Die Zugabe der Schwefelsäure kann grundsätzlich an beliebiger Stelle in den einen oder in die mehreren Rektoren I (z.B. Schlaufenreaktoren) erfolgen. Bevorzugt erfolgt die Zugabe der Schwefelsäure vor der Zugabe des ACH. Besonders bevorzugt erfolgt die Zugabe der Schwefelsäure auf der Saugseite der jeweiligen Reaktorpumpe. Oftmals kann hierdurch die Pumpfähigkeit der gashaltigen Reaktionsmischung verbessert werden.

Die Reaktoren I (z.B. die Schlaufenreaktoren I) umfassen bevorzugt jeweils mindestens einen Gasabscheider. Typischerweise kann über den Gasabscheider einerseits kontinuierlich Produktstrom (erste Reaktionsmischung) entnommen werden, andererseits lassen sich gasförmige Nebenprodukte abtrennen und ausschleusen. Typischerweise bildet sich als gasförmiges Nebenprodukt hauptsächlich Kohlenstoffmonoxid. Bevorzugt wird ein Teil des Abgases, welches in der Amidierung erhalten wird, zusammen mit der zweiten Reaktionsmischung, welche in der zweiten Reaktionsstufe (Konvertierung) erhalten wird, in einen Gasabscheider geführt.

In einer bevorzugten Ausführungsform umfasst die erste Reaktionsstufe die Umsetzung von Acetoncyanhydrin (ACH) und Schwefelsäure in mindestens zwei getrennten Reaktionszonen, bevorzugt in mindestens zwei Schlaufenreaktoren.

Bevorzugt erfolgt die Umsetzung von Acetoncyanhydrin (ACH) und Schwefelsäure in der Art, dass das Reaktionsvolumen auf mindestens zwei Reaktionszonen verteilt ist und die Gesamtmenge an ACH in die verschiedenen Reaktionszonen separat dosiert wird. Bevorzugt ist die Menge an ACH, die dem ersten Reaktor oder der ersten Reaktionszone zugeführt wird, größer oder gleich den Mengen an ACH, die den nachfolgenden Reaktoren oder den nachfolgenden Reaktionszonen zugeführt werden.

Vorzugsweise werden 50 bis 90 Gew.-%, bevorzugt 60 bis 75 Gew.-%, des gesamten Volumenstroms an zugeführtem ACH, in den ersten Reaktor eingeführt (z.B. (1a)). Die verbleibende Menge an zugeführtem ACH wird in den zweiten Reaktor und gegebenenfalls in weitere Reaktoren eingeführt (z.B. (1b)). Typischerweise erfolgt eine Aufteilung der Gesamtmenge an ACH auf den ersten Reaktor I (z.B. (A)) und der zweiten Reaktor I (z.B. (B)) im Massenverhältnis erster Reaktor I: zweiter Reaktor I im Bereich von 70:30 bis 80:20, bevorzugt von etwa 75:25.

Bevorzugt ist das molare Verhältnis von zugegebener Schwefelsäure zu ACH im ersten Reaktor oder in der ersten Reaktionszone größer als das entsprechende molare Verhältnis in den nachfolgenden Reaktoren oder in den nachfolgenden Reaktionszonen.

Insbesondere bevorzugt umfasst die erste Reaktionsstufe die Umsetzung von Acetoncyanhydrin (ACH) und Schwefelsäure in mindestens zwei getrennten Reaktoren, bevorzugt mindestens zwei Schlaufenreaktoren, wobei Schwefelsäure und Acetoncyanhydrin (ACH) im ersten Reaktor in einem molaren Verhältnis von Schwefelsäure zu ACH im Bereich von 1,6 bis 3; bevorzugt 1,7 bis 2,6; insbesondere bevorzugt 1,8 bis 2,3; eingesetzt werden, und wobei Schwefelsäure und Acetoncyanhydrin (ACH) im zweiten Reaktor in einem molaren Verhältnis von Schwefelsäure zu ACH im Bereich von 1,2 bis 2,0; bevorzugt 1,2 bis 1,7, insbesondere bevorzugt 1,3 bis 1,7; eingesetzt werden.

In einer besonders bevorzugten Ausführungsform erfolgt die Umsetzung in der ersten Reaktionsstufe in zwei oder mehreren Schlaufenreaktoren (z.B. (A) und (B)), wobei die Gesamtmenge an ACH in den ersten und mindestens einen weiteren Schlaufenreaktor dosiert wird. Insbesondere bevorzugt umfasst jeder Schlaufenreaktor mindestens eine Pumpe, einen mit Wasser als Medium gekühlten Wärmetauscher, eine Gastrennvorrichtung, mindestens eine Abgasleitung, verbunden mit der Gastrennvorrichtung, und mindestens eine Zuführleitung für ACH in flüssiger Form. Bevorzugt sind die mindestens zwei Schlaufenreaktoren derart miteinander verschaltet, dass die gesamte resultierende Reaktionsmischung des ersten Reaktors in die nachfolgenden Reaktoren geführt wird und die Reaktionsmischung in den nachfolgenden Reaktoren mit weiterem flüssigen ACH und optional weiteren Schwefelsäuremengen versetzt wird.

Typischerweise wird nach der ersten Reaktionsstufe (Amidierung) eine erste Reaktionsmischung erhalten, welche 5 bis 25 Gew.-% Sulfoxyisobuttersäureamid (SIBA), 5 bis 25 Gew.-% Methacrylsäureamid (MASA) sowie < 3 % Hydroxyisobuttersäureamid (HIBA), jeweils bezogen auf die gesamte Reaktionsmischung, gelöst in schwefelsaurer Reaktionsmatrix, enthält.

### Zweite Reaktionsstufe (Konvertierung)

Das erfindungsgemäße Verfahren umfasst in Schritt b das Konvertieren der ersten Reaktionsmischung, umfassend das Erhitzen auf eine Temperatur im Bereich von 130 bis 200 °C, bevorzugt 130 bis 180 °C, bevorzugt 130 bis 170 °C, besonders bevorzugt 140 bis 170 °C, in ein oder mehreren Reaktoren II in einer zweiten Reaktionsstufe (Konvertierung), wobei eine zweite Reaktionsmischung, enthaltend überwiegend Methacrylsäureamid (MASA) und Schwefelsäure, erhalten wird.

Typischerweise wird beim Erhitzen der ersten Reaktionsmischung (Konvertierung), welche eine schwefelsaure Lösung enthaltend SIBA, HIBA und MASA, überwiegend jeweils in Form der Hydrogensulfate, darstellt, auf eine Temperatur im Bereich von 130 bis 200 °C, bevorzugt 130 bis 180 °C die Menge an MASA bzw. MASA·H₂SO₄ durch Dehydratisierung des HIBA bzw. SIBA erhöht.

Insbesondere bevorzugt erfolgt die Konvertierung in der zweiten Reaktionsstufe bei einer Temperatur im Bereich von 130 bis 200 °C, bevorzugt von 130 bis 180 °C, besonders bevorzugt 140 bis 170 °C und einer Verweilzeit im Bereich von 2 bis 30 Minuten, bevorzugt 3 bis 20 Minuten, insbesondere bevorzugt 5 bis 20 Minuten.

Die Konvertierungsreaktion lässt sich typischerweise in zwei Abschnitte unterteilen, wobei im ersten Teil die Amidierungsmischung relativ schnell auf die erforderliche Temperatur der Konvertierung angehoben wird, und wobei im zweiten Teil die Mischung nach Erreichen der Reaktionstemperatur annähernd adiabatisch bis zum gewünschten Umsatz gehalten wird. Üblicherweise wird die Reaktion so optimiert, dass die Ausbeute an MASA maximiert ist, SIBA bis auf Spuren abreagiert ist und HIBA ebenfalls nur noch im Spurenbereich von einigen hundert bzw. einigen 1000 ppm vorhanden ist.

Bevorzugt erfolgt das Erhitzen in der zweiten Reaktionsstufe (Konvertierung) über einen möglichst kurzen Zeitraum. Insbesondere erfolgt das Erhitzen in der zweiten Reaktionsstufe für einen Zeitraum von 1 bis 30 Minuten, bevorzugt 1 bis 20 Minuten, besonders bevorzugt 2 bis 15 Minuten, ganz besonders bevorzugt 2 bis 10 Minuten. In einer bevorzugten Ausführungsform umfasst die zweite Reaktionsstufe (Konvertierung) das Erhitzen der Reaktionsmischung, beispielsweise in einem oder mehreren Vorheizer-Segmenten, und das Führen der Reaktionsmischung bei annähernd adiabatischen Bedingungen, beispielsweise in einem oder mehreren Verweilzeitsegmenten.

Die Konvertierung kann in bekannten Reaktoren durchgeführt werden, welche die Erzielung der genannten Temperaturen in den genannten Zeiträumen ermöglichen. Die Energiezufuhr kann hierbei in bekannter Weise, beispielsweise mittels Wasserdampfs, Heißwasser, geeigneter Wärmeträgermedien, elektrischer Energie oder elektromagnetischer Strahlung, wie Mikrowellenstrahlung, erfolgen. Bevorzugt wird die Konvertierung in der zweiten Reaktionsstufe in einem oder mehreren Wärmetauschern durchgeführt.

In einer bevorzugten Ausführungsform wird die Konvertierung in der zweiten Reaktionsstufe in einem Wärmetauscher, umfassend eine zweistufige oder mehrstufige Anordnung von Rohrwendeln, durchgeführt. Vorzugsweise liegen die mehrstufigen Rohrwendeln in einer gegenläufigen Anordnung vor.

Der Wärmetauscher kann beispielsweise mit einem oder mehreren Gasabscheidern kombiniert werden. So ist es beispielsweise möglich, die Reaktionsmischung nach Verlassen der ersten Rohrwendel des Wärmetauschers und/oder nach Verlassen der zweiten Rohrwendel des Wärmetauschers durch einen Gasabscheider zu führen. Dabei können insbesondere gasförmige Nebenprodukte aus der Reaktionsmischung abgetrennt werden.

Typischerweise wird die erste Reaktionsmischung erhalten in der ersten Reaktionsstufe vollständig in den Reaktor II der zweiten Reaktionsstufe geführt. Optional können die Schritte der Amidierung und Konvertierung alternierend durchlaufen werden, wobei die Konvertierung bevorzugt den letzten Schritt vor der anschließenden dritten Reaktionsstufe (z.B. Veresterung) darstellt. Bevorzugt umfasst diese Ausführungsform eine Zwischenkonvertierung zwischen zwei Amidierungsschritten und eine abschließende Konvertierung. Bevorzugt wird die zweite Reaktionsmischung, erhalten in der zweiten Reaktionsstufe, in einen zweiten Reaktor I für einen weiteren Amidierungsschritt geführt.

Bevorzugt wird die zweite Reaktionsmischung, welche nach der Konvertierung erhalten wird, in einen Gasabscheider (z.B. (D)) geführt, wobei gasförmige Nebenprodukte zumindest teilweise von der zweiten Reaktionsmischung abgetrennt werden können. Typischerweise wird die entgaste zweite Reaktionsmischung vollständig in die dritte Reaktionsstufe (Veresterung) geführt. Bevorzugt wird das Abgas, welches im Gasabscheider nach der Konvertierung erhalten wird, vollständig oder teilweise aus dem Verfahren ausgeschleust. Alternativ wird das Abgas, welches im Gasabscheider nach der Konvertierung erhalten wird, vollständig oder teilweise in die dritte Reaktionsstufe (Veresterung) geführt. In dieser Variante werden gasförmige organische Komponenten, die neben dem Hauptnebenprodukt der Amidierung und Konvertierung, nämlich CO, vorliegen, in die Veresterungsreaktion eingetragen; das Gas kann auf diese Art und Weise als Stripp-Medium für Roh-MMA dienen. MAN ist eine solche Komponente, die auf diesem Weg gasförmig in die Veresterungsreaktion gelangen kann und dort teilweise zu MASA abreagiert.

Insbesondere ermöglicht das erfindungsgemäße Verfahren, die Menge an störenden Nebenprodukten, bevorzugt die Mengen an MAN, Aceton, MAS und / oder HIBA, in der zweiten Reaktionsmischung (nach Amidierung und Konvertierung) zu reduzieren. Bevorzugt enthält die zweite Reaktionsmischung kleiner oder gleich 3 Gew.-%, bevorzugt kleiner oder gleich 2 Gew.-% MAS, kleiner oder gleich 1,5 Gew.-%, bevorzugt kleiner oder gleich 1 Gew.-%, HIBA und kleiner oder gleich 0,3 Gew.-% MAN, jeweils bezogen auf die gesamte zweite Reaktionsmischung.

Bevorzugt enthält die zweite Reaktionsmischung (nach Amidierung und Konvertierung) 30 bis 40 Gew.-% Methacrylsäureamid (MASA), bezogen auf die gesamte zweite Reaktionsmischung. Bevorzugt enthält die zweite Reaktionsmischung (nach Amidierung und Konvertierung) 30 bis 40 Gew.-% MASA, 0 bis 3 Gew.-% MAS und 0,2 bis 1,5 Gew.-%, bevorzugt 0,2 bis 1 Gew.-%, HIBA und 0,01 bis 0,3 Gew.-% MAN, jeweils bezogen auf die gesamte zweite Reaktionsmischung. Die zweite Reaktionsmischung (d.h. nach der Konvertierung) enthält somit eine signifikant geringere HIBA-Konzentration als die erste Reaktionsmischung (d.h. direkt nach der Amidierung).

### Dritte Reaktionsstufe (Veresterung)

Das erfindungsgemäße Verfahren umfasst in Schritt c die Umsetzung der zweiten Reaktionsmischung, enthaltend überwiegend Methacrylsäureamid, mit Alkohol und Wasser, bevorzugt mit Methanol und Wasser, in ein oder mehreren Reaktoren III in einer dritten Reaktionsstufe (Veresterung), wobei eine dritte Reaktionsmischung, enthaltend Alkylmethacrylat, erhalten wird.

Die Bedingungen der Veresterung im großtechnischen Maßstab sind dem Fachmann bekannt und beispielsweise in US 5,393,918 beschrieben.

Die Umsetzung in der dritten Reaktionsstufe (Veresterung) wird bevorzugt in einem oder mehreren geeigneten Reaktoren, beispielsweise in beheizten Kesseln durchgeführt. Insbesondere können mit Wasserdampf beheizte Kessel verwendet werden. In einer bevorzugten Ausführungsform erfolgt die Veresterung in zwei oder mehreren, beispielsweise drei oder vier, aufeinander folgenden Kesseln (Kesselkaskade).

Typischerweise wird die Veresterung bei Temperaturen im Bereich von 90 bis 180 °C, bevorzugt von 100 bis 150 °C, bei Drücken von bis zu 7 bara, bevorzugt von kleiner oder gleich 2 bara, und unter Verwendung von Schwefelsäure als Katalysator durchgeführt. Besonders bevorzugt ist es, als Katalysator die Schwefelsäure der zweiten Reaktionsmischung zu verwenden und nicht über diese Menge hinaus zusätzliche Säure zu verwenden.

Bevorzugt erfolgt die Umsetzung der zweiten Reaktionsmischung mit mindestens äquimolaren Mengen beziehungsweise einem Überschuss an Alkohol und Wasser, bevorzugt einem Überschuss an Methanol und Wasser. Die Zugabe der zweiten Reaktionsmischung, enthaltend überwiegend Methacrylsäureamid, und die Zugabe von Alkohol erfolgen bevorzugt in der Art, dass sich ein molares Verhältnis von Methacrylsäureamid zu Alkohol im Bereich von 1:0,7 bis 1:1,6 ergibt. In einer bevorzugten Ausführungsform erfolgt die Umsetzung in der dritten Reaktionsstufe in zwei oder mehreren Reaktoren III, wobei sich im ersten Reaktor III ein molares Verhältnis von Methacrylsäureamid zu Alkohol im Bereich von 1:0,7 bis 1:1,4, bevorzugt im Bereich von 1:0,9 bis 1:1,3, ergibt, und wobei sich im zweiten und den möglichen nachfolgenden Reaktoren III ein molares Verhältnis von Methacrylsäureamid zu Alkohol im Bereich von 1:1,0 bis 1:1,3 ergibt.

Bevorzugt setzt sich der in die dritte Reaktionsstufe (Veresterung) zugeführte Alkohol zusammen aus frisch ins Verfahren zugeführtem Alkohol (Frischalkohol) und aus Alkohol, welcher in zurückgeführten Strömen (Recyclingströmen) des erfindungsgemäßen Verfahrens enthalten ist. Es ist zudem möglich Alkohol im erfindungsgemäßen Verfahren zu verwenden, welcher in Recyclingströmen aus nachgeschalteten Downstream-Prozessen enthalten ist.

Der Alkohol kann insbesondere ausgewählt sein aus linearen, verzweigten, gesättigten und ungesättigten C₁-C₆-Alkoholen, bevorzugt C₁-C₄-Alkoholen. Insbesondere handelt es sich bei dem Alkohol um einen gesättigten C₁-C₄-Alkohol. Bevorzugt ist der Alkohol ausgewählt aus Methanol, Ethanol, Propanol und Butanol. Besonders bevorzugt handelt es sich bei dem Alkohol um Methanol.

Typischerweise erfolgt die Zugabe an Wasser in den Reaktor III oder in die Reaktoren III der dritten Reaktionsstufe derart, dass die Konzentration an Wasser im Bereich von 10 bis 30 Gew.-%, bevorzugt 15 bis 25 Gew.-%, bezogen jeweils auf die gesamte Reaktionsmischung im Reaktor III, liegt.

Grundsätzlich kann das in die dritte Reaktionsstufe (Veresterung) zugeführte Wasser aus einer beliebigen Quelle stammen und verschiedene organische Verbindungen enthalten, sofern keine Verbindungen enthalten sind, welche die Veresterung oder die nachfolgenden Verfahrensstufen nachteilig beeinflussen. Bevorzugt stammt das in die dritte Reaktionsstufe zugeführte Wasser aus zurückgeführten Strömen (Recyclingströmen) des erfindungsgemäßen Verfahrens, beispielsweise aus der Aufreinigung des Alkylmethacrylats. Es ist zudem möglich, Frischwasser, insbesondere vollentsalztes Wasser oder Brunnenwasser, in die dritte Reaktionsstufe (Veresterung) zuzuführen.

Die Veresterung mit Methanol liefert typischerweise eine dritte Reaktionsmischung enthaltend Alkylmethacrylat (insbesondere MMA), Hydroxyisobuttersäuremethylester (HIBSM) und weitere oben beschriebene Nebenprodukte sowie signifikante Mengen Wasser und nicht-umgesetzten Alkohol (insbesondere Methanol).

In einer bevorzugten Ausführungsform erfolgt die Veresterung in zwei oder mehreren (insbesondere drei oder vier) aufeinander folgenden Kesseln (Kesselkaskade), wobei der flüssige Überlauf und die gasförmigen Produkte aus dem ersten Kessel in den zweiten Kessel geführt werden. Typischerweise wird mit möglichen nachfolgenden Kesseln in entsprechender Weise verfahren. Insbesondere kann durch eine solche Fahrweise die Schaumbildung in den Kesseln reduziert werden. Im zweiten Kessel und in den möglichen nachfolgenden Kesseln kann ebenfalls eine Zugabe von Alkohol erfolgen. Bevorzugt ist hierbei die Menge an zugegeben Alkohol um mindestens 10 % kleiner im Vergleich zum vorhergehenden Kessel. Typischerweise kann es sich beim zugegebenen Alkohol um Frischalkohol und / oder zurückgeführte (recyclierte) Alkohol-haltige Stoffströme handeln. Die Konzentrationen an Wasser in den verschiedenen Kesseln können sich typischerweise voneinander unterscheiden. Typischerweise beträgt die Temperatur der in den ersten Kessel eingespeisten zweiten Reaktionsmischung im Bereich von 100 bis 180 °C. Typischerweise liegt die Temperatur im ersten Kessel im Bereich von 90 bis 180 °C und die Temperatur im zweiten und in den möglichen nachfolgenden Kesseln im Bereich von 100 bis 150 °C.

In einer bevorzugten Ausführungsform wird der verdampfbare Anteil der dritten Reaktionsmischung, welche in der dritten Reaktionsstufe erhalten wird, in gasförmiger Form (Brüden) aus den Reaktoren III abgeführt und der weiteren Aufarbeitung, beispielsweise einem Destillationsschritt, zugeführt. Insbesondere kann der verdampfbare Anteil der dritten Reaktionsmischung in Form eines Brüdens in den Sumpf einer nachfolgenden Destillationskolonne K1 (Rohkolonne K1) geführt werden. Wenn eine Kaskade aus mehreren Reaktoren III, z.B. mehreren Kesseln, verwendet wird, kann in jedem Kessel der verdampfbare Teil der entstehenden Reaktionsmischung als Brüdenstrom abgeführt und in die weitere Aufarbeitung geführt werden. Optional wird lediglich der im letzten Kessel entstehende verdampfbare Anteil der Reaktionsmischung (als dritte Reaktionsmischung) als Brüdenstrom abgeführt und in die weitere Aufarbeitung geführt. Dieser bei der Veresterung entstehende Brüdenstrom (dritte Reaktionsmischung) stellt typischerweise eine azeotrope Mischung dar, enthaltend Wasser, Alkylmethacrylat, Alkohol sowie die beschriebenen Nebenprodukte, z.B. Methacrylnitril und Aceton. Typischerweise weist dieser bei der Veresterung entstehende Brüdenstrom (dritte Reaktionsmischung) eine Temperatur im Bereich von 60 bis 120 °C auf, wobei die Temperatur vom eingesetzten Alkohol abhängt. Typischerweise weist dieser bei der Veresterung entstehende Brüdenstrom eine Temperatur im Bereich von 70 bis 90 °C auf, falls Methanol als Alkohol eingesetzt wird.

Vorteilhaft können ein oder mehrere Stabilisatoren in verschiedenen Stoffströmen des erfindungsgemäßen Verfahrens zugesetzt werden, um eine Polymerisation des Alkylmethacrylat zu verhindern oder zu reduzieren. Beispielsweise kann ein Stabilisator zu der dritten Reaktionsmischung, erhalten nach der Veresterung, zugegeben werden. Weiterhin vorteilhaft kann ein Stabilisator der Kopf-Fraktion des ersten Destillationsschritts K1 (Rohkolonne K1) zugegeben werden. Bevorzugt können Phenothiazin und andere adäquat wirkende Stabilisatoren beispielsweise in der ersten Reaktionsstufe (Amidierung) und / oder in der zweiten Reaktionsstufe (Konvertierung) eingesetzt werden. Bevorzugt können weiterhin phenolische Verbindungen, Chinone und Katechole in der dritten Reaktionsstufe (Veresterung) und/oder im Aufarbeitungsteil werden eingesetzt. Daneben kommen Amin-N-Oxide, wie z.B. TEMPOL bzw. Kombinationen der genannten Stabilisatoren zum Einsatz. Besonders bevorzugt sind Mischungen mindestens zweier dieser Stabilisatoren, die an verschiedenen Stellen des Verfahrens zugegeben werden.

Aus der dritten Reaktionsstufe (Veresterung) wird bevorzugt ein Abfallstrom (z.B. (11)) bestehend im Wesentlichen aus verdünnter Schwefelsäure und Ammoniumhydrogensulfat, abgeführt. Dieser Abfallstrom wird typischerweise aus dem Verfahren ausgeschleust. Bevorzugt wird dieser Abfallstrom, insbesondere zusammen mit einem oder mehreren wässrigen Abfallströmen des erfindungsgemäßen Verfahrens, einem Verfahren zur Regenerierung von Schwefelsäure oder einem Verfahren zur Gewinnung von Ammoniumsulfat zugeführt.

### Aufarbeitung der dritten Reaktionsmischung

Das erfindungsgemäße Verfahren umfasst in Schritt d die Abtrennung von Alkylmethacrylat aus der dritten Reaktionsmischung, wobei die Abtrennung (Aufarbeitung) von Alkylmethacrylat aus der dritten Reaktionsmischung mindestens zwei Destillationsschritte umfasst, bei denen die Nebenprodukte Methacrylnitril (MAN) und Aceton zumindest teilweise in der Kopf-Fraktion als wasserhaltiges Heteroazeotrop erhalten werden und insbesondere dabei vom Alkylmethacrylat zumindest teilweise abgetrennt werden, wobei das wasserhaltige Heteroazeotrop, enthaltend Methacrylnitril (MAN) und Aceton, aus mindestens einem dieser Destillationsschritte zumindest teilweise aus dem Verfahren ausgeschleust wird, und wobei mindestens ein Strom, enthaltend Methacrylnitril und Aceton, zumindest teilweise in die dritte Reaktionsstufe zurückgeführt wird.

Bevorzugt handelt es sich bei dem mindestens einen Strom, enthaltend Methacrylnitril und Aceton, welcher zumindest teilweise in die dritte Reaktionsstufe (Veresterung) zurückgeführt wird, um wasserhaltiges Heteroazeotrop, enthaltend Methacrylnitril und Aceton, aus mindestens einem der Destillationsschritte wie oben beschrieben.

Beispielsweise können die wässrige Phase und / oder die organische Phase des wasserhaltigen Heteroazeotrops aus mindestens einem Destillationsschritt und/oder Mischungen hiervon aus dem Verfahren ausgeschleust werden, optional nach weiteren Aufarbeitungsschritten, wie Kondensation, Phasentrennung, Extraktion und Waschschritten.

Bevorzugt wird mindestens eine wässrige Phase, welche mittels Kondensation und Phasentrennung des wasserhaltigen Heteroazeotrops aus mindestens einem der Destillationsschritte erhalten wird, vollständig oder teilweise, optional nach einem Extraktionsschritt, in die dritte Reaktionsstufe (Veresterung) zurückgeführt und dort mit der zweite Reaktionsmischung, enthaltend überwiegend Methacrylsäureamid und Schwefelsäure, in Kontakt gebracht.

Bevorzugt wird mindestens eine wässrige Phase, welche mittels Kondensation und Phasentrennung des wasserhaltigen Heteroazeotrops aus mindestens einem der Destillationsschritte erhalten wird, vollständig oder teilweise, optional nach einem Extraktionsschritt, aus dem Verfahren ausgeschleust.

Bevorzugt umfasst die Abtrennung von Alkylmethacrylat aus der dritten Reaktionsmischung (Schritt d) mindestens einen Phasentrennungsschritt, bei dem das wasserhaltige Heteroazeotrop aus mindestens einem der Destillationsschritte in eine wässrige Phase, enthaltend Methacrylnitril und Aceton, und eine organische Phase, enthaltend überwiegend Alkylmethacrylat, getrennt wird, wobei die wässrige Phase vollständig oder teilweise aus dem Verfahren ausgeschleust wird.

In einer weiteren bevorzugten Ausführungsform wird das wasserhaltige Heteroazeotrop aus mindestens einem der Destillationsschritte zumindest teilweise, optional nach einem Waschschritt, in Form eines gasförmigen Stroms vollständig oder teilweise aus dem Verfahren ausgeschleust. Beispielsweise kann das wasserhaltige Heteroazeotrop aus mindestens einem Destillationsschritt in Form eines Brüdenstroms abgeführt werden und optional nach weiteren Verarbeitungsschritten, beispielsweise ausgewählt aus Kondensation, Phasentrennung, Extraktion und Waschschritten, in gasförmiger Form (als Abgas-Strom) aus dem Verfahren ausgeschleust werden.

Bevorzugt umfasst die Abtrennung von Alkylmethacrylat aus der dritten Reaktionsmischung (Schritt d) mindestens einen Phasentrennungsschritt, bei dem das wasserhaltige Heteroazeotrop aus mindestens einem der Destillationsschritte in eine wässrige Phase, enthaltend Methacrylnitril und Aceton, und eine organische Phase, enthaltend überwiegend Alkylmethacrylat, getrennt wird, wobei die wässrige Phase teilweise aus dem Verfahren ausgeschleust wird und / oder teilweise in die dritte Reaktionsstufe zurückgeführt wird, und wobei die organische Phase, enthaltend überwiegend Alkylmethacrylat, vollständig oder teilweise in den mindestens einen Destillationsschritt zurückgeführt wird.

Neben den störenden Nebenprodukten MAN und Aceton enthält das wasserhaltige Heteroazeotrop, welches als Kopf-Fraktion in dem mindestens einen Destillationsschritt erhalten wird, typischerweise Alkohol, beispielsweise Methanol, Wasser, Dimethylether und Methylformiat.

Methacrylnitril (MAN) bildet sowohl mit Methanol (MeOH) als auch mit Methylmethacrylat (MMA) ein Azeotrop bzw. hat einen ähnlichen Siedepunkt wie einige Azeotrope des Methylmethacrylats und ist daher meist schwer und nur unter erheblichem Aufwand aus dem Produkt abzutrennen. Typischerweise wird das störende Nebenprodukt MAN in dem mindestens einen Destillationsschritt wie oben beschrieben daher sowohl in der Kopf-Fraktion als wasserhaltiges Heteroazeotrop als auch in der Sumpf-Fraktion erhalten.

### Rohkolonne (K1) und Vorreinigung

Bevorzugt umfasst die Abtrennung von Alkylmethacrylat in Schritt d des erfindungsgemäßen Verfahrens die Vorreinigung der dritten Reaktionsmischung, welche in der Veresterung erhalten wird. Insbesondere umfasst die Vorreinigung mindestens einen Destillationsschritt K1 (z.B. Rohkolonne (F)), mindestens einen Phasentrennungsschritt (z.B. Phasentrenner I, (G)) und mindestens einen Extraktionsschritt (z.B. Extraktionsschritt (H)). In einer weiteren Ausführungsform umfasst die Vorreinigung mindestens zwei Destillationsschritte, z.B. Rohkolonne K1 und Roh-Stripper-Kolonne K4 (z.B. (I)), und mindestens einen Phasentrennungsschritt (z.B. Phasentrenner (K)).

Bevorzugt wird die dritte Reaktionsmischung, erhalten in der dritten Reaktionsstufe kontinuierlich verdampft, wobei der hierbei entstehenden Brüdenstrom (z.B. (12) einem ersten Destillationsschritt K1 (z.B. Rohkolonne (F)) zugeführt wird, bei dem eine Kopf-Fraktion (z.B. (14a) oder (14b)), enthaltend Alkylmethacrylat, Wasser und Alkohol, und eine Sumpf-Fraktion (z.B. (13)), enthaltend schwerer siedende Komponenten, erhalten werden, und wobei die Sumpf-Fraktion vollständig oder teilweise in die dritte Reaktionsstufe zurückgeführt wird. Insbesondere stellt die Kopf-Fraktion des Destillationsschritts K1 (z.B. (14a) oder (14b)) ein wasserhaltiges Heteroazeotrop, enthaltend Methacrylnitril und Aceton, dar.

In einer bevorzugten Ausführungsform (Variante A) wird die Kopf-Fraktion des Destillationsschritts K1 (z.B. (14a)), enthaltend Alkylmethacrylat, Wasser und Alkohol, in einem Phasentrennungsschritt (Phasentrenner I, z.B. (G)) in eine organische Phase OP-1 (z.B. (15a)), enthaltend den überwiegenden Teil des Alkylmethacrylats, und in eine wässrige Phase WP-1 (z.B. (15b)), enthaltend Alkohol und weitere wasserlösliche Verbindungen, getrennt, wobei typischerweise die wässrige Phase vollständig oder teilweise in die dritte Reaktionsstufe zurückgeführt wird. Weiterhin bevorzugt wird die organische Phase OP-1, enthaltend den überwiegenden Teil des Alkylmethacrylats, einer Extraktion (z.B. (H)), bevorzugt unter Verwendung von Wasser als Extraktionsmittel, unterzogen, wobei typischerweise die wässrige Phase dieser Extraktion (z.B. 17b) vollständig oder teilweise in die dritte Reaktionsstufe (Veresterung) zurückgeführt wird.

In einer weiteren bevorzugten Ausführungsform (Variante B) wird die Kopf-Fraktion des Destillationsschritts K1 (z.B. (14b)), enthaltend Alkylmethacrylat, Wasser und Alkohol, als Brüdenstrom in einen weiteren Destillationsschritt K4 (z.B. Roh-Stripper-Kolonne (I)) geführt, bei dem ein wasserhaltiges Heteroazeotrop (z.B. (19a)), enthaltend Methacrylnitril und Aceton, als Kopf-Fraktion und eine Sumpf-Fraktion, enthaltend Alkylmethacrylat, erhalten werden. Bevorzugt wird die Kopf-Fraktion (z.B. (19a)) des Destillationsschritts K4, optional nach einem Waschschritt (z.B. (J)), bevorzugt nach einem Waschschritt mit Alkohol (z.B. Methanol), in Form eines gasförmigen Stroms (z.B. (21a)) vollständig oder teilweise aus dem Verfahren ausgeschleust. Bevorzugt wird die Sumpf-Fraktion des Destillationsschritts K4 in einem Phasentrennungsschritt (Phasentrenner II, z.B. (K)) in eine wässrige Phase WP-2 (z.B. (20b)), enthaltend Methacrylnitril und Aceton, und eine organische Phase OP-2 (z.B. (20a)), enthaltend den überwiegenden Teil des Alkylmethacrylats, getrennt. Typischerweise wird die wässrige Phase WP-2, enthaltend Methacrylnitril und Aceton, vollständig oder teilweise in die dritte Reaktionsstufe (Veresterung) zurückgeführt.

### Azeotrop-Kolonne (K2) und Reinkolonne (K3)

Bevorzugt umfasst die Abtrennung von Alkylmethacrylat aus der dritten Reaktionsmischung (Schritt d), dass eine organische Phase (z.B. (17a) aus Extraktion (H) oder (20a) aus Phasentrenner (K)), enthaltend den überwiegenden Teil des Alkylmethacrylats, in einen Destillationsschritt K2 (Azeotrop-Kolonne, z.B. (L)) geführt wird, bei dem als Kopf-Fraktion (z.B. (22a)) ein wasserhaltiges Heteroazeotrop, enthaltend Methacrylnitril und Aceton, und als Sumpf-Fraktion ein Alkylmethacrylat-Rohprodukt (z.B. (22b)) erhalten werden.

Bevorzugt wird der Destillationsschritt K2 (Azeotrop-Kolonne, z.B. (L)) im Unterdruck durchgeführt. Bevorzugt wird der organische Zulauf des Destillationsschritts K2 (z.B. (17a) oder (20a)) vorgewärmt und auf den Kopf der Destillationskolonne K2 geführt. Typischerweise kann der Kopf der Kolonne mittels eines Verdampfers indirekt mit Niederdruck-Dampf beheizt werden.

Bevorzugt wird am Kopf der Destillationskolonne K2 (Azeotrop-Kolonne, z.B. (L)) ein wasserhaltiges Heteroazeotrop (z.B. (22a)), enthaltend Alkylmethacrylat (insbesondere MMA), Wasser, Alkohol (insbesondere Methanol), Aceton, Methacrylnitril und weitere Leichtsieder, abgetrennt.

Typischerweise wird in dem Destillationsschritt K2 (Azeotrop-Kolonne, z.B. (L)) eine Sumpf-Fraktion (z.B. (22b)) erhalten, welche den überwiegenden Anteil des Alkylmethacrylats, insbesondere Methylmethacrylat, enthält, und welche nahezu frei von Leichtsiedern ist, aber mit Schwersiedern, beispielsweise Methacrylsäure (MAS) und Hydroxyisobuttersäuremethylester (HIBSM), verunreinigt ist. Bevorzugt enthält das Alkylmethacrylat-Rohprodukt (z.B. (22b)), welches als Sumpf-Fraktion des Destillationsschritts K2 (Azeotrop-Destillation, z.B. (L)) erhalten wird, mindestens 99,0 Gew.-% Alkylmethacrylat. Bevorzugt weist das Alkylmethacrylat-Rohprodukt (z.B. (22b)), welches als Sumpf-Fraktion des Destillationsschritts K2 (Azeotrop-Destillation, z.B. (L)) erhalten wird, einen MAN-Gehalt von 20 bis 2000 ppm auf.

In einer bevorzugten Ausführungsform wird die Kopf-Fraktion (z.B. (22a)) des Destillationsschritts K2 (Azeotrop-Kolonne, z.B. (L)) zunächst als Brüdenstrom in einen Kondensator (z.B. (M)) geführt und im Vakuum stufenweise kondensiert. Bevorzugt entsteht in dieser stufenweisen Kondensation in der ersten Stufe (saugseitig des Kondensators) ein zweiphasiges Kondensat I (z.B. (23a)) und in der zweiten Stufe (druckseitig des Kondensators) ein weiteres Kondensat II (z.B. 23 (d)). Bevorzugt wird das bei der stufenweisen Kondensation (insbesondere bei der druckseitigen Kondensation) entstehende Abgas (z.B. (23e) oder (23b)), optional nach einem Waschschritt (z.B. (J)), aus dem Verfahren ausgeschleust.

In einer bevorzugten Ausführungsform (Variante A) wird das zweiphasige Kondensat I (z.B. (23a)) aus der ersten Stufe der Kondensation in einen Phasentrenner (z.B. (N)) geführt und das weitere Kondensat II (z.B. 23 (d)) aus der zweiten Stufe der Kondensation als Extraktionsmittel in einem nachgelagerten Extraktionsschritt verwendet.

In einer anderen bevorzugten Ausführungsform (Variante B) werden die flüssigen Phasen der stufenweise Kondensation (z.B. (M)) vereinigt und in Form eines flüssigen zweiphasigen Stroms (z.B. (23c)) in einen Phasentrenner (z.B. (K)) geführt.

Bevorzugt wird das wasserhaltige Heteroazeotrop, welches als Kopf-Fraktion im Destillationsschritt K2 (z.B. (L)) erhalten wird, typischerweise nach der Kondensation (z.B. in (M)), in mindestens eine organische Phase OP-2, enthaltend Alkylmethacrylat, und mindestens eine wässrige Phase WP-2, enthaltend MAN, Aceton und Methanol, in einem Phasentrenner II getrennt (z.B. in Phasentrenner (N) oder (K)). Bevorzugt werden die wässrige Phase WP-2 und / oder die organische Phase OP-2 vollständig oder teilweise aus dem Verfahren ausgeschleust. Insbesondere wird die wässrige Phase WP-2 (z.B. (20 b) oder (24b)) vollständig oder teilweise in die dritte Reaktionsstufe (Veresterung) zurückgeführt (z.B. (26c) oder (20b)), typischerweise nach einer Phasentrennung (z.B. in (N) oder (K)). Insbesondere bevorzugt wird die wässrige Phase WP-2, enthaltend Methacrylnitril (MAN) und Aceton, teilweise aus dem Verfahren ausgeschleust und teilweise in die dritte Reaktionsstufe (Veresterung) zurückgeführt.

Oftmals enthält die wässrige Phase WP-2 (z.B. (20 b) oder (24b)) 10 bis 10.000 ppm MAN, bezogen auf die gesamte wässrige Phase WP-2.

Bevorzugt wird die organische Phase OP-2 des wasserhaltigen Heteroazeotrops, welches als Kopf-Fraktion im Destillationsschritt K2 erhalten wird, vollständig oder teilweise, besonders bevorzugt vollständig, in den Destillationsschritt K2 zurückgeführt (z.B. (24a) oder (20a)), typischerweise nach einer Phasentrennung (z.B. in (N) oder (K)). Insbesondere enthält die organische Phase OP-2 (z.B. (24a) oder (20a)) Alkylmethacrylat und Methacrylnitril (MAN).

Typischerweise ist der überwiegende Anteil an MAN,, welches in der Kopf-Fraktion des Destillationsschritts K2 enthalten ist, in der organischen Phase (OP-2) des Heteroazeotrops zu finden. Bevorzugt kann durch die vollständige oder teilweise Rückführung der organischen Phase des Heteroazeotrops (OP-2) in den Destillationsschritt K2 (z.B. (L)) eine Anreicherung der störenden Nebenprodukte, insbesondere MAN, und damit eine effektivere Abtrennung, z.B. über die wässrige Phase des Heteroazeotrops (WP-2) erzielt werden.

In einer bevorzugten Ausführungsform ist das Gewichtsverhältnis der Gesamtmenge an MAN, welche in das Verfahren, bevorzugt in die dritte Reaktionsstufe (Veresterung) zurückgeführt wird (z.B. über (28a)), zu der Gesamtmenge an MAN, welche aus dem Verfahren ausgeschleust wird (z.B. über (28b)), kleiner 7, bevorzugt kleiner 5, insbesondere kleiner 3. Diese Kennzahl weist auf die Wichtigkeit der Rückführung bzw. die Kreislaufführung hin, um MAN chemisch umzusetzen oder zu entfernen. Typischerweise erfolgt ansonsten erfolgt eine Erhöhung der MAN-Konzentration im Endprodukt (z.B. MMA) mit der Konsequenz, dass man einen weiteren verfahrenstechnischen Schritt, z. B. eine weitere Destillation, durchführen müsste, was nicht wünschenswert ist.

In einer bevorzugten Ausführungsform wird das Alkylmethacrylat-Rohprodukt (z.B. (22b)) aus Destillationsschritt K2 in einen weiteren Destillationsschritt K3 (Reinkolonne) geführt, bei dem das Alkylmethacrylat von schwerer-siedenden Verbindungen getrennt wird, und bei dem als Kopf-Fraktion (z.B. (25a)) ein Alkylmethacrylat-Reinprodukt erhalten wird. Bevorzugt enthält das Alkylmethacrylat-Reinprodukt (z.B. (25a)) aus Destillationsschritt K3 mindestens 99,9 Gew.-%, bevorzugt mindestens 99,95 Gew.-%, bezogen auf das Alkylmethacrylat-Reinprodukt, Alkylmethacrylat. Bevorzugt weist das Alkylmethacrylat-Reinprodukt (z.B. (25a)) aus Destillationsschritt K3 einen Gehalt an Methacrylnitril (MAN) im Bereich von 10 bis 300 ppm, bevorzugt 10 bis 100 ppm, besonders bevorzugt 10 bis 80 ppm, insbesondere bevorzugt 50 bis 80 ppm, bezogen auf das Alkylmethacrylat-Reinprodukt, auf. Bevorzugt weist das Alkylmethacrylat-Reinprodukt einen Gehalt an Aceton von kleiner oder gleich 10 ppm, bevorzugt von kleiner oder gleich 2 ppm, besonders bevorzugt von kleiner oder gleich 1 ppm, bezogen auf das Alkylmethacrylat-Reinprodukt, auf.

In einer bevorzugten Ausführungsform wird im zweiten Destillationsschritt K2 (Azeotrop-Kolonne) (z.B. (L)) als Sumpf-Fraktion ein Alkylmethacrylat-Rohprodukt (z.B. (22b)) erhalten, welches bevorzugt mindestens 99,0 Gew.-% Alkylmethacrylat enthält, wobei das Alkylmethacrylat-Rohprodukt in einem weiteren Destillationsschritt K3 (Reinkolonne) (z.B. (O)) gereinigt wird, wobei als Kopf-Fraktion (z.B. (25a)) ein Alkylmethacrylat-Reinprodukt erhalten wird, welches einen Gehalt an Methacrylnitril im Bereich von 10 bis 300 ppm, bevorzugt 10 bis 100 ppm, besonders bevorzugt 10 bis 80 ppm, insbesondere bevorzugt 50 bis 80 ppm, bezogen auf das Alkylmethacrylat-Reinprodukt, aufweist.

Bevorzugt wird das Alkylmethacrylat-Rohprodukt (z.B. (22b)) aus Destillationsschritt K2 in flüssiger Form knapp unterhalb des Siedepunkts der Zusammensetzung in den Destillationsschritt K3 (Reinkolonne) geführt. Bevorzugt erfolgt der Zulauf des Destillationsschritts K3 (z.B. (22b)) in der Mitte der Reinkolonne K3. Typischerweise erfolgt der Energieeintrag in die Destillationskolonne K3 mittels eines mit Niederdruck-Dampf beheizten Verdampfers. Bevorzugt wird der Destillationsschritt K3 (Reinkolonne, z.B. (O)), wie der Destillationsschritt K2, im Unterdruck durchgeführt.

Typischerweise wird der am Kopf der Kolonne K3 (z.B. (O)) vollständig kondensierte Destillatstrom in einen Produktstrom (z.B. (25a)) und einen Rücklaufstrom in die Kolonne aufgeteilt. Die Qualität des Alkylmethacrylat-Reinprodukts (z.B. (25a)) kann beispielsweise über das Rücklaufverhältnis gesteuert werden. Der Sumpfstrom (25b) wird bevorzugt in die Veresterung (z.B. (E)) zurückgeführt bzw. gelangt direkt oder indirekt wieder auf die Azeotropkolonne, entweder als Feedstrom oder wird dem Kondensat der Azeotropkolonne zugeführt, wobei Anteile als Kolonnenrücklauf verwendet werden können.

Typischerweise kann die Sumpf-Fraktion des Destillationsschritts K3 (Reinkolonne) (z.B. (O)) vollständig oder teilweise in die dritte Reaktionsstufe (Veresterung) zurückgeführt werden. Insbesondere kann hierdurch enthaltenes Alkylmethacrylat zurückgewonnen werden.

### Variante A

In einer bevorzugten Ausführungsform der Erfindung (auch als Variante A bezeichnet) umfasst die Abtrennung von Alkylmethacrylat aus der dritten Reaktionsmischung, dass
(i) die dritte Reaktionsmischung erhalten in der dritten Reaktionsstufe (Veresterung) zunächst in einem ersten Destillationsschritt K1 (Rohkolonne) (z.B. (F)) destilliert wird, wobei ein erstes wasserhaltiges Heteroazeotrop (z.B. (14a)), enthaltend Methacrylnitril und Aceton, als Kopf-Fraktion erhalten wird;
(ii) das erste wasserhaltige Heteroazeotrop als Kondensat in einem Phasentrennungsschritt (Phasentrenner I) (z.B. (G)) in eine wässrige Phase WP-1 (z.B. (15b)) und eine organische Phase OP-1 (z.B. (15a)), enthaltend den überwiegenden Teil des Alkylmethacrylats, getrennt wird;
(iii) die organische Phase OP-1 (z.B. (15a)), optional nach einem Extraktionsschritt, in einen zweiten Destillationsschritt K2 (Azeotrop-Kolonne) (z.B. (L)) geführt wird, wobei als Kopf-Fraktion ein zweites wasserhaltiges Heteroazeotrop (z.B. (22a)), enthaltend Methacrylnitril und Aceton, erhalten wird;
(iv) zumindest ein Teil (z.B. 23a)) des kondensierten zweiten wasserhaltigen Heteroazeotrops (z.B. (22a)) in einem Phasentrennungsschritt (Phasentrenner II) (z.B. N) in eine wässrige Phase WP-2 (z.B. (24b)), enthaltend Methacrylnitril und Aceton, und eine organische Phase OP-2 (z.B. (24a)) getrennt wird, wobei insbesondere durch zusätzliche Zugabe von Wasser (z.B. (16c)) diese Phasentrennung begünstigt wird,
   wobei die organische Phase OP-2 (z.B. (24a)) vollständig oder teilweise in den zweiten Destillationsschritt K2 zurückgeführt wird,
   und wobei die wässrige Phase WP-2 (z.B. (24b)), enthaltend Methacrylnitril und Aceton, teilweise in die dritte Reaktionsstufe (Veresterung) zurückgeführt wird und teilweise, optional nach einem Extraktionsschritt (z.B. (P)), aus dem Verfahren ausgeschleust wird.

Bevorzugt umfasst (iv), dass das kondensierte wasserhaltige Heteroazeotrop (z.B. (23a)) in einem Phasentrennungsschritt (Phasentrenner II) (z.B. N) in eine wässrige Phase WP-2 (z.B. (24b)), enthaltend Methacrylnitril und Aceton, und eine organische Phase OP-2 (z.B. (24a)) getrennt wird, wobei durch zusätzliche Zugabe von Wasser (z.B. (16c)) diese Phasentrennung begünstigt wird,

In einer bevorzugten Ausführungsform (Variante A) wird die wässrige Phase WP-1 (z.B. (15b)) vollständig oder teilweise in die dritte Reaktionsstufe (Veresterung) zurückgeführt, und die organische Phase OP-1 (z.B. (15a)), enthaltend den überwiegenden Teil des Alkylmethacrylats, wird einer Extraktion (z.B. (H)) unter Verwendung von Wasser als Extraktionsmittel unterzogen, wobei die wässrige Phase dieser Extraktion (z.B. (17b)) in die dritte Reaktionsstufe zurückgeführt wird und die organische Phase (z.B. (17a)) dieser Extraktion in den zweiten Destillationsschritt K2 (Azeotrop-Kolonne, z.B. (L)) geführt wird.

Bevorzugt kann im Phasentrennungsschritt (Phasentrenner II) (z.B. N), bei dem zumindest ein Teil (z.B. 23a)) des zweiten wasserhaltigen Heteroazeotrops (z.B. (22a)) in eine wässrige Phase WP-2 und eine organische Phase OP-2 getrennt wird, Wasser, typischerweise vollentsalztes Wasser oder Brunnenwasser, zugegeben werden, wodurch typischerweise die Phasentrennung verbessert wird.

In einer bevorzugten Ausführungsform (Variante A) wird ein Teil (z.B. (26b)) der wässrigen Phase WP-2 (z.B. (24b)), enthaltend Methacrylnitril und Aceton, einer Extraktion (z.B. (P)) unterzogen, wobei eine wässrige Phase WP-3 (z.B. (28b)) und eine organische Phase OP-3 (z.B. (28a)) erhalten werden, wobei die wässrige Phase WP-3 vollständig oder teilweise aus dem Verfahren ausgeschleust wird, und wobei die organische Phase OP-3 vollständig oder teilweise in die dritte Reaktionsstufe zurückgeführt wird. Optional kann die organische Phase OP-3 zumindest teilweise aus dem Verfahren ausgeschleust werden. Bevorzugt wird die organische Phase OP-3 zusammen mit der Abfallsäure der Veresterung (z.B. (11)) als Spaltsäure (z.B. (27)) aus dem Verfahren ausgeschleust. Insbesondere kann die wässrige Phase WP-3, z.B. zusammen mit der Abfallsäure der Veresterung (z.B. (11)), einem nachgeschalteten Verfahren zur Regenerierung von Schwefelsäure oder einem nachgeschalteten Verfahren zur Gewinnung von Ammoniumsulfat zugeführt werden (z.B. über (27)).

Typischerweise erfolgt in der oben beschriebenen Variante A die Ausschleusung von störenden Nebenprodukten, insbesondere MAN und Aceton, über einen Teil (z.B. (26b)) der wässrige Phase WP-2 (z.B. (24b)), wobei durch einen nachgeschalteten Extraktionsschritt (z.B. (P)) der Verlust an Alkylmethacrylat vermindert werden kann.

Bevorzugt wird die Kopf-Fraktion des Destillationsschritts K2 (zweites wasserhaltiges Heteroazeotrop, z.B. (22a)) zunächst als Brüdenstrom einem Kondensator (z.B. (M)) zugeführt und im Vakuum stufenweise kondensiert. Bevorzugt wird hierbei ein zweiphasiges Kondensat I (z.B. (23a)) in der ersten Stufe der Kondensation (saugseitig des Kondensators) erhalten, welches in einen Phasentrenner (z.B. (N)) geführt wird. Zudem wird bevorzugt ein weiteres Kondensat II (z.B. 23 (d)) in der zweiten Stufe der Kondensation (druckseitig des Kondensators) erhalten, welches als Extraktionsmittel bei der Extraktion (z.B. (P)) der wässrigen Phase WP-2 (z.B. (24b) oder eines Teils der wässrigen Phase WP-2 (z.B. (26b)) verwendet wird.

In einer weiteren Ausführungsform wird ein Teil (z.B. 26b)) der wässrigen Phase WP-2, enthaltend Methacrylnitril und Aceton, einer Extraktion (z.B. (P)) unterzogen, wobei eine wässrige Phase WP-3 (z.B. (28b)) und eine organische Phase OP-3 (z.B. 28a)) erhalten werden, wobei die wässrige Phase WP-3 einem weiteren Destillationsschritt K5 unterzogen wird, wobei im Destillationsschritt K5 eine Kopf-Fraktion, enthaltend Methacrylnitril, erhalten wird, welche aus dem Verfahren ausgeschleust wird, und wobei im Destillationsschritt K5 eine Sumpf-Fraktion, enthaltend Wasser, erhalten wird, welche vollständig oder teilweise in die Extraktion (z.B. (P)) zurückgeführt wird, und wobei die organische Phase OP-3 vollständig oder teilweise in die dritte Reaktionsstufe zurückgeführt wird. Typischerweise ist die wässrige Sumpf-Fraktion aus Destillationsschritt K5 weitgehend frei von Methacrylnitril. Typischerweise kann mit Hilfe des weiteren Destillationsschritts K5 der ausgeschleuste Abwasserstrom (z.B. 28b) gereinigt und die Entsorgung des Abfallstroms vereinfacht werden.

### Variante B

In einer bevorzugten Ausführungsform der Erfindung (auch als Variante B bezeichnet) umfasst die Abtrennung von Alkylmethacrylat aus der dritten Reaktionsmischung, dass
(i) die dritte Reaktionsmischung erhalten in der dritten Reaktionsstufe zunächst in einem ersten Destillationsschritt K1 (Rohkolonne) (z.B. (F)) destilliert wird, wobei ein erstes wasserhaltiges Heteroazeotrop (z.B. 14b), enthaltend Methacrylnitril und Aceton, als Kopf-Fraktion erhalten wird;
(ii) das erste wasserhaltige Heteroazeotrop als Brüdenstrom in einen weiteren Destillationsschritt K4 (Roh-Stripper, z.B. (I)) geführt wird, bei dem ein weiteres wasserhaltiges Heteroazeotrop, enthaltend Methacrylnitril und Aceton, als Kopf-Fraktion (z.B. (19a)) und eine Sumpf-Fraktion (z.B. (19b)), enthaltend Alkylmethacrylat, erhalten werden,
(iii) die Kopf-Fraktion (z.B. (19a)) aus Destillationsschritt K4, optional nach einem Waschschritt (z.B. (J)), in Form eines gasförmigen Stroms (z.B. (21a)) vollständig oder teilweise aus dem Verfahren ausgeschleust wird;
(iv) die Sumpf-Fraktion (z.B. (19b)) aus Destillationsschritt K4 in einem Phasentrennungsschritt (Phasentrenner II, z.B. (K)) in eine wässrige Phase WP-2 (z.B. (20b)), enthaltend Methacrylnitril und Aceton, und eine organische Phase OP-2 (z.B. 20a)) getrennt wird, wobei die wässrige Phase WP-2, enthaltend Methacrylnitril und Aceton, vollständig oder teilweise in die dritte Reaktionsstufe zurückgeführt wird,
(v) die organische Phase OP-2 vollständig oder teilweise in einen zweiten Destillationsschritt K2 (Azeoptrop-Kolonne, z.B. (L)) geführt wird, bei dem als Kopf-Fraktion ein zweites wasserhaltiges Heteroazeotrop, enthaltend Methacrylnitril und Aceton, erhalten wird, welches vollständig oder teilweise kondensiert wird (z.B. in (M)) und in den Phasentrennungsschritt (Phasentrenner II, z.B: K) nach (iv) geführt wird (z.B. (23c)).

Typischerweise wird im Destillationsschritt K4 (Roh-Stripper, z.B. (I)) als Kopf-Fraktion (z.B. (19a)) ein leichtsiedendes Gemisch enthaltend Methanol, Aceton, Methacrylsäureester und Wasser, und als Sumpf-Fraktion (19b) ein azeotrop-siedenden Gemisch enthaltend Alkylmethacrylat und Wasser erhalten.

In einer bevorzugten Ausführungsform (Variante B) wird der Rücklauf im Destillationsschritt K4 (Roh-Stripper) (z.B. (I)) mittels eines Partialkondensators erzeugt, welcher so eingestellt ist, dass die Kopf-Fraktion (z.B. (19a)) in Form eines Brüden aus der Kolonne K4 ausgeschleust wird und ein flüssiges Kondensat enthaltend Alkylmethacrylat in die Kolonne als Rücklauf zurück gefahren wird. Ein Teil des Rücklaufes der Destillationskolonne K4 wird bevorzugt in Form eines flüssigen Seitenstroms (z.B. (19c)) abgeführt und als Rücklauf in Destillationsschritt K1 (Roh-Kolonne, z.B. (F)) geführt.

Typischerweise ist die Sumpf-Fraktion (z.B. (19b)) des Destillationsschritts K4 (Roh-Stripper, z.B. (I)) ein azeotropes Gemisch enthaltend Alkylmethacrylat, Wasser, geringe Mengen Leichtsieder (z.B. Methanol, Aceton) und Schwersieder (z.B. Hydroxyisobuttersäureester). Bevorzugt wird die Sumpf-Fraktion aus Destillationsschritt K4 abgekühlt und in einem Phasentrenner II (z.B. (K)), bevorzugt zusammen mit einem weiteren Rücklaufstrom (z.B. 23c)), in eine organische Phase OP-2 (z.B. (20a)) und ein wässrige Phase WP-2 (z.B. (20b)) aufgetrennt. Typischerweise enthält die wässrige Phase WP-2 Wasser, Alkohol, Aceton und Alkylmethacrylat. Bevorzugt kann die wässrige Phase WP-2 (z.B. 20b)) mit Frischwasser, z.B. vollentsalztem Wasser (VE-Wasser), vermischt werden (z.B. (16b)) und in Form eines vereinigten Rücklaufstroms (z.B. (20c)) der Veresterung (z.B. (E)) zugeführt werden. Typischerweise können hierdurch der Wasserbedarf der Veresterung gedeckt und Edukte zurückgewonnen werden.

Bevorzugt wird die Kopf-Fraktion (z.B. (19a)) aus Destillationsschritt K4 als Brüdenstrom in eine Abgas-Waschkolonne (z.B. (J)) geführt und dort mit frischem Alkohol (z.B. (10b)), z.B. Methanol, als Waschmedium gewaschen. Bevorzugt wird der gewaschene Abgas-Strom (z.B. (21a)) vollständig oder teilweise aus dem Verfahren ausgeschleust. Bevorzugt wird im Sumpf der Abgas-Waschkolonne (z.B. (J)) der organische Strom (z.B. (21b)), enthaltend Methanol und Alkylmethacrylat, erhalten, welcher in die Veresterung (E) zurückgeführt wird. Dieser organische Rücklaufstrom kann hierbei auf verschiedene Veresterungsreaktoren verteilt werden.

### Weitere Schritte

In einer bevorzugten Ausführungsform umfasst das erfindungsgemäße Verfahren eine Regenerierung von Schwefelsäure, wobei ein Teil der dritten Reaktionsmischung erhalten in der dritten Reaktionsstufe und mindestens ein wässriger oder organischer Abfallstrom, enthaltend Schwefelsäure, Ammoniumhydrogensulfat und sulfonierte Aceton-Derivate, welcher aus der Ausschleusung des wasserhaltigen Heteroazeotrops, enthaltend Methacrylnitril und Aceton, resultiert, einem thermischen Regenerierungsschritt zugeführt werden, bei dem Schwefelsäure erhalten wird, welche in die erste Reaktionsstufe zurückgeführt wird. Dieses Verfahren verläuft bei Temperaturen oberhalb von 900 °C in der Gasphase und umfasst die thermische Spaltung der Hydrogensulfatsalze, die hierbei zu Stickstoff oxidiert werden.

In einer bevorzugten Ausführungsform umfasst das erfindungsgemäße Verfahren eine Gewinnung von Ammoniumsulfat, wobei ein Teil der dritten Reaktionsmischung erhalten in der dritten Reaktionsstufe und mindestens ein wässriger oder organischer Abfallstrom, enthaltend Schwefelsäure, Ammoniumhydrogensulfat und sulfonierte Aceton-Derivate, welcher aus der Ausschleusung des wasserhaltigen Heteroazeotrops, enthaltend Methacrylnitril und Aceton, resultiert, einem thermischen Regenerierungsschritt zugeführt wird, bei dem mittels Kristallisation Ammoniumsulfat erhalten wird, welches als Nebenprodukt abgetrennt wird. Hierbei muss typischerweise neutralisiert werden, was durch Zugabe von wässrigem Ammoniak oder Ammoniak selbst erfolgt. Die Aufarbeitung der Abfallsäure mittels sogenannter Nassoxidation in Gegenwart homogener Katalysatoren (z.B. Kupfersulfat) ist typischerweise eine weitere Möglichkeit der Verarbeitung und Prozessierung.

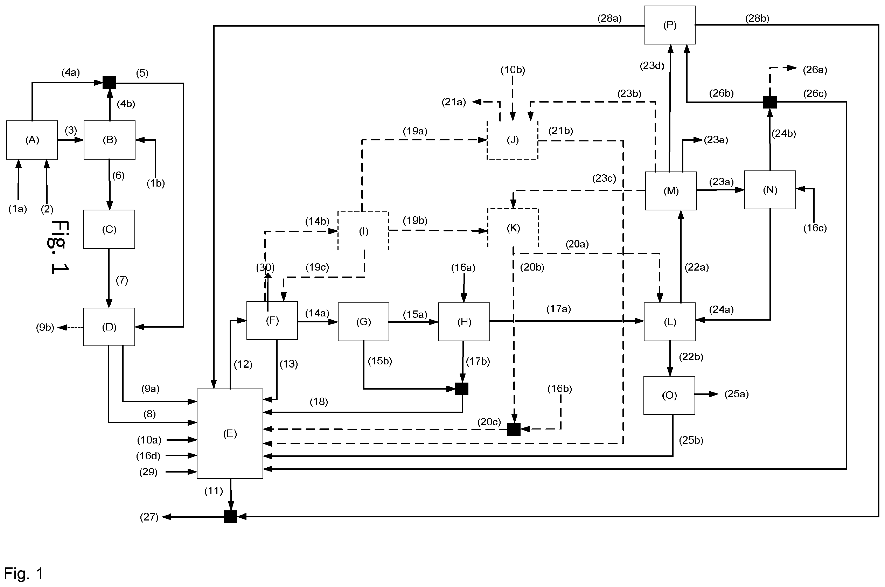

Bevorzugt wird ein Abfallstrom (z.B. (11)), bestehend im Wesentlichen aus verdünnter Schwefelsäure, welcher aus dem Reaktor III der Veresterung abgeführt wird , und / oder ein oder mehrere wässrige Abfallströme des Verfahrens (z.B. (28b) oder (26a)) einem Verfahren zur Regenerierung von Schwefelsäure oder einem Verfahren zur Gewinnung von Ammoniumsulfat zugeführt. Bevorzugt wird die Spaltsäure (27) gemäß Figuren 1-3 einem Verfahren zur Regenerierung von Schwefelsäure oder einem Verfahren zur Gewinnung von Ammoniumsulfat zugeführt.

Verfahren zur Regenerierung von Schwefelsäure und Verfahren zur Gewinnung von Ammoniumsulfat aus Spaltsäure sind dem Fachmann bekannt und beispielsweise in WO 02/23088 A1 und WO 02/23089 A1 beschrieben. Die Einbettung von Verfahren zur Regenerierung von Schwefelsäure in ein Verfahren zur Herstellung von Alkylmethacrylaten nach dem ACH-Sulfo-Verfahren ist beispielsweise in DE 10 2006 059 513 oder DE 10 2006 058 250 beschrieben.

Weiterhin ist es bevorzugt, die aus den Reaktionsstufen der Amidierung und Konvertierung anfallenden Abgase ebenfalls dem thermischen Schwefelsäure-Regenerierungsschritt zuzuführen.

### Beschreibung der Figuren

Figur 1 zeigt ein Fließschema von bevorzugten Ausführungsformen des erfindungsgemäßen Verfahrens. In Figur 1 sind die bevorzugten Elemente eines Anlagenverbunds zur kontinuierlichen Herstellung und Aufreinigung von Alkylmethacrylaten, insbesondere Methylmethacrylat (MMA), gezeigt. Der dargestellte Anlagenverbund weist verschiedene untereinander meist fluidleitenden verbundene Anlagen als Elemente dieses Verbunds auf. Zu diesem Anlagenverbund gehört die Herstellung von Methacrylsäureamid bzw. dessen schwefelsaurer Lösung bestehend aus den Verfahrensschritten Amidierung (A, B) und Konvertierung (C, D), gefolgt von einer Veresterung (E), gefolgt von einer Aufarbeitung des Reaktionsprodukts (F, G, H, I, J, K) wiederum gefolgt von einer Feinreinigung (L, M, N, O). Durchgezogene Linien beschreiben bevorzugt die Stoffstromführungen des Verfahrens nach Variante A; gestrichelte Linien beschreiben bevorzugt die Stoffstromführungen des alternativen Verfahrens nach Variante B. Eine Kombination von Apparaten und Stoffströmen der beiden Varianten ist ebenfalls möglich.

Figur 2 zeigt ein schematisches Fließbild einer ersten bevorzugten Ausführungsform des erfindungsgemäßen Verfahrens (Variante A).

Figur 3 zeigt ein schematisches Fließbild einer zweiten bevorzugten Ausführungsform des erfindungsgemäßen Verfahrens (Variante B).

In den Figuren 1 bis 3 haben die Bezugszeichen die folgenden Bedeutungen:
Apparate
   (A) Amidierungsreaktor Stufe 1
   (B) Amidierungsreaktor Stufe 2
   (C) Erhitzer
   (D) Gasabscheider / Zwischenbehälter
   (E) Veresterungsreaktor / -Kaskade
   (F) Roh-Kolonne (Kolonne K1)
   (G) Phasentrenner I
   (H) Waschkolonne (Extraktion I)
   (I) Roh-Stripper-Kolonne (Kolonne K4)
   (J) Abgas-Waschkolonne
   (K) Phasentrenner Roh-MMA
   (L) Azeotrop-Kolonne (Kolonne K2)
   (M) Kondensator / Vakuumsystem
   (N) Phasentrenner II
   (O) Rein-Kolonne (Kolonne K3)
   (P) Extraktionskolonne II (Extraktion Pumpenkondensat)
Stoffströme
   (1a) Acetoncyanhydrin-Zuleitung Stufe 1
   (1b) Acetoncyanhydrin-Zuleitung Stufe 2
   (2) Schwefelsäure-Zuleitung
   (3) Amid-Gemisch Austritt Stufe 1
   (4a) Abgas Amidierungsreaktoren Stufe 1
   (4b) Abgas Amidierungsreaktoren Stufe 2
   (5a) Optionales Abgas Amidierungsreaktoren Stufe 1 & 2
   (5b) Abgas Amidierungsreaktoren Stufe 1 & Stufe 2
   (6) Amid-Gemisch Austritt Stufe 2
   (7) Konvertiertes Amid-Gemisch
   (8) Entgastes Amid-Gemisch
   (9a) Abgas Gasabscheider zur Veresterung
   (9b) Optionales Abgas, abgeführt aus dem Verfahren
   (10a) Alkohol-Zuleitung (für MMA: Methanol)
   (10b) Alkohol-Zuleitung Abgas-Waschkolonne
   (11) Spaltsäure aus Veresterung
   (12) Brüdenstrom aus Veresterung
   (13) Flüssiger Rücklaufstrom Roh-Kolonne
   (14a) Destillatstrom Roh-Kolonne
   (14b) Brüdenstrom Roh-Kolonne
   (15a) Organische Phase Phasentrenner I (OP-1)
   (15b) Wässrige Phase Phasentrenner I (WP-1)
   (16a) VE-Wasser-Zuleitung Extraktion
   (16b) VE-Wasser-Zuleitung
   (16c) VE-Wasser-Zuleitung Phasentrenner II (N)
   (16d) Direktdampf
   (17a) Gewaschene organische Phase (OP-1)
   (17b) Wässrige Phase Extraktion
   (18) Vereinigte wässrige Phasen
   (19a) Brüdenstrom Roh-Stripper-Kolonne
   (19b) Sumpfstrom Roh-Stripper-Kolonne
   (19c) Organischer Seitenstrom Roh-Stripper-Kolonne / Rücklauf Rohkolonne
   (20a) Organische Phase Phasentrennung I (OP-1)
   (20b) Wässrige Phase Phasentrennung I (WP-1)
   (20c) vereinigter Rücklaufstrom / Produktwasser
   (21a) Abgas aus Abgas-Waschkolonne
   (21b) Sumpfstrom Abgas-Waschkolonne
   (22a) Brüden Azeotrop-Kolonne
   (22b) Sumpfprodukt Azeotrop-Kolonne / Roh-Alkylmethacrylat-Produkt
   (23a) Kondensat I zu Phasentrenner II
   (23b) Abgas Azeotrop-Kolonne/ Vakuumsystem
   (23c) Kondensat Kreislaufstrom
   (23d) Kondensat II, Vakuumpumpen-Kondensat
   (23e) Abgas/Inert-Anteil des Kondensations-/Vakuumsystems
   (24a) Organische Phase Phasentrenner II (OP-2)
   (24b) Wässrige Phase Phasentrenner II (WP-2)
   (25a) Kopfprodukt Rein-Kolonne / Rein-Alkylmethacrylat-Produkt
   (25b) Sumpfprodukt Rein-Kolonne
   (26a) Ausschleusung wässrige Phase aus Phasentrenner II
   (26b) Wässrige Phase zur Extraktion Pumpenkondensat
   (26c) Wässrige Phase zur Veresterung (Recycle)
   (27) Spaltsäure
   (28a) Organische Phase Extraktion Pumpenkondensat (OP-3)
   (28b) Wässrige Phase Extraktion Pumpenkondensat (WP-3) / Raffinat zur Spaltsäure
   (29) Methanol / Methylmethacrylat-Gemisch
   (30) Vakuumpumpenkondensat

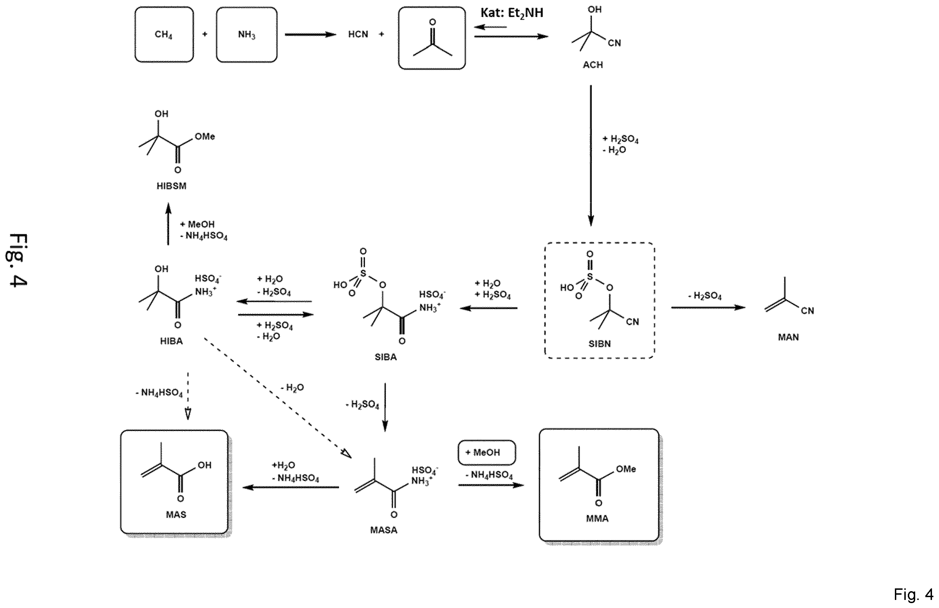

Figur 4 beschreibt das Reaktionsnetzwerk der Bildung von Methacrylsäure und / oder Methylmethacrylat ausgehend von Methan und Ammoniak sowie Aceton. Ausgehend von Methan (CH₄) und Ammoniak (NH₃) kann über das BMA-Verfahren (Blausäure aus Methan und Ammoniak) mittels katalytischer Dehydrierung Blausäure hergestellt werden (CH₄ + NH₃ → HCN + 3 H₂) (Variante 1). Alternativ kann über das Andrussow-Verfahren ausgehend von Methan und Ammoniak, unter Zugabe von Sauerstoff, Blausäure hergestellt werden (CH₄ + NH₃ + 1,5 O₂ → HCN + 3 H₂O) (Variante 2). Im nächsten Schritt wird ausgehend von Aceton und Blausäure, unter Zugabe eines basischen Katalysators (z.B. Diethylamin Et₂NH oder auch Alkalihydroxide), Acetoncyanhydrin (ACH) hergestellt. Die Hydroxygruppe des Acetoncyanhydrins wird im Folgenden mit Schwefelsäure verestert, wobei zunächst Sulfoxyisobutyronitril (SIBN) erhalten wird. Die Nitrilgruppe des Sulfoxyisobutyronitrils (SIBN) kann im nächsten Schritt unter Einwirkung von Schwefelsäure und Wasser verseift werden, wobei Sulfoxyisobuttersäureamid-Hydrogensulfat (SIBA·H₂SO₄) erhalten wird. Als Nebenreaktion kann die Bildung von Methacrylnitril (MAN) unter Eliminierung von Schwefelsäure aus SIBN erfolgen. Sulfoxyisobuttersäureamid-Hydrogensulfat (SIBA·H₂SO₄) kann zudem teilweise zum alpha-Hydroxyisobuttersäureamidhydrogensulfat (alpha-Hydroxyisobutyramidhydrogensulfat, HIBA·H₂SO₄) hydrolysiert werden. Ebenfalls ist die Rückreaktion zum Schwefelsäureester SIBA·H₂SO₄ möglich. Als Nebenprodukt kann alpha-Hydroxyisobuttersäure (HIBS) durch weitere Hydrolyse von HIBA·H₂SO₄ gebildet werden. Ausgehend von SIBA·H₂SO₄ wird unter Eliminierung von Schwefelsäure Methacrylamid-Hydrogensulfat (MASA·H₂SO₄) gebildet (Konvertierung). Ebenfalls kann die langsame Umsetzung von HIBA oder HIBS zu MAS oder MASA als Eliminierungsreaktion unter Abspaltung von NH₄HSO₄ oder Wasser ablaufen. Nachfolgend kann Methacrylamid-Hydrogensulfat (MASA·H₂SO₄) durch Hydrolyse zu Methacrylsäure (MAS) oder durch Veresterung mit Methanol (MeOH) zu Methylmethacrylat (MMA) umgewandelt werden. Wird alpha-Hydroxyisobuttersäure (HIBS) in die Veresterung eingetragen, kann es zu alpha-Hydroxyisobuttersäuremethylester (HIBSM) umgesetzt werden.

Die Abkürzungen in Figur 4 haben die folgenden Bedeutungen:
- ACH: Acetoncyanhydrin;
- SIBN: alpha-Sulfoxyisobuttersäurenitril, auch als Sulfoxyisobutyronitril bezeichnet;
- SIBA: alpha-Sulfoxyisobuttersäureamid, auch als Sulfoxyisobuttersäureamid oder Sulfoxyisobutyramid bezeichnet;
- SIBA·H₂SO₄: alpha-Sulfoxyisobuttersäureamid-Hydrogensulfat, auch als Sulfoxyisobuttersäureamid-Hydrogensulfat bezeichnet;
- MAN: Methacrylnitril;
- HIBA: alpha-Hydroxyisobuttersäureamid; alpha-Hydroxyisobutyramid;
- HIBA·H₂SO₄: alpha-Hydroxyisobuttersäureamid-Hydrogensulfat, alpha-Hydroxyisobutyramid-Hydrogensulfat;
- MASA: Methacrylsäureamid / Methacrylamid;
- MASA·H₂SO₄: Methacrylsäureamid-Hydrogensulfat;
- MAS: Methacrylsäure;
- MMA: Methylmethacrylat;
- HIBS: alpha-Hydroxyisobuttersäure;
- HIBSM: alpha-Hydroxyisobuttersäuremethylester

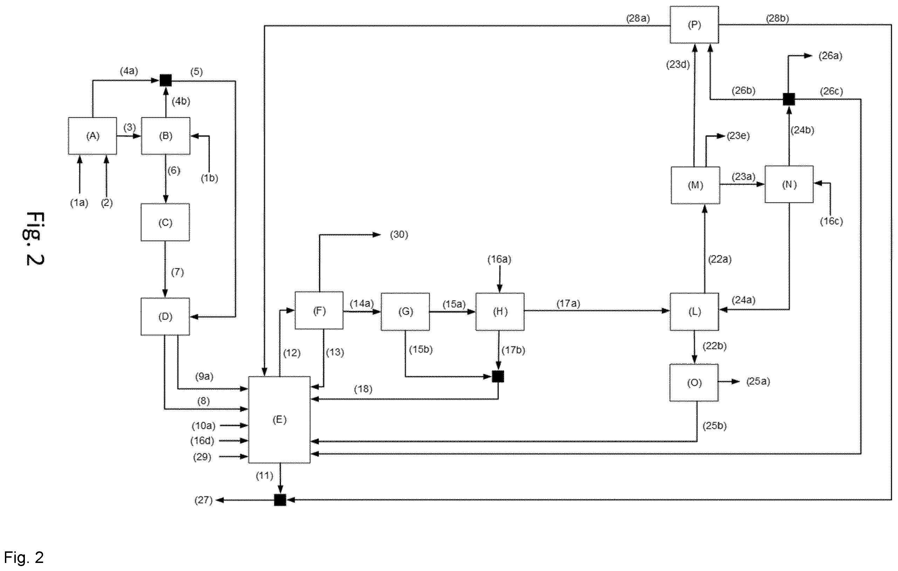

### Ausführungsform des Verfahrens nach Figur 2 (Variante A)

Eine mögliche Ausführungsform des Verfahrens (Variante A) betreffend die Herstellung von Alkylmethacrylat, insbesondere MMA, gemäß Fließbild in Figur 2 ist im Folgenden beschrieben:
In den Amidierungsreaktoren (A) und (B), welche als Schlaufenreaktor ausgebildet sind, erfolgt die Umsetzung von ACH und Schwefelsäure zu einer schwefelsauren Lösung enthaltend SIBA, HIBA und MASA (überwiegend jeweils in Form der Hydrogensulfate). In Abhängigkeit der Reaktionsbedingungen kann, insbesondere in den Reaktoren (A) und (B), als Nebenprodukt MAN aus ACH unter Freisetzung von Wasser entstehen. Die Schlaufenreaktoren (A) und (B) umfassen jeweils die Elemente Kreislaufpumpe, statischer Mischer, Wärmetauscher und Gasabscheider.

Der Amidierungsreaktor (A) der Stufe 1 weist eine ACH-Zuleitung (1a) und eine Schwefelsäure-Zuleitung (2) auf. Die ACH-Zuleitung (1a) mündet in den Kreislauf des Schlaufenreaktors (A) auf der Druckseite der Kreislaufpumpe, jedoch vor dem statischen Mischer. Die Schwefelsäure-Zuleitung (2) mündet in den Kreislauf des Schlaufenreaktors (A) vor der ACH-Zuleitung (1a) und auf der Saugseite der Kreislaufpumpe, wodurch bevorzugt die Pumpfähigkeit des gashaltigen Reaktionsgemischs verbessert werden kann.

Die Reaktionsmischung in Schlaufenreaktor (A) wird im Temperaturbereich von 70-130 °C und bei einem Kreislaufverhältnis (Verhältnis aus Umwälzvolumenstrom zu Zulaufvolumenstrom) im Bereich von 5 bis 110 umgepumpt und mittels Sekundärwassergekühlten Rohrbündelwärmetauscher temperiert. Insbesondere wird die Reaktionswärme der stark exothermen Reaktion zwischen Acetoncyanhydrin und Schwefelsäure abgeführt. Die statische Verweilzeit im Reaktorkreislauf des Amidierungsreaktors (A) liegt im Bereich von 5 bis 35 Minuten. Der Amidierungsreaktor (A) wird bei Normaldruck betrieben.

Das abgemischte und temperierte Reaktionsgemisch wird anschließend in einen Gasabscheider eingeleitet. Hier erfolgen die gezielte Abtrennung von gasförmigen Nebenkomponenten (wie Kohlenstoffmonoxid und anderen Inerten / Leichtsiedern) vom Amid-Kreislaufstrom und die Ausleitung des Abgas-Stroms (4a).

Ein Teilstrom (3) des umgepumpten Reaktionsgemischs wird mittels einer Austragspumpe, gravimetrisch oder mit Vordruck der Reaktor-Kreislaufpumpe selbst dem zweiten Schlaufenreaktor (B) zugeführt und bedarfsweise durch einen zusätzlichen Wärmetauscher aufgeheizt. Zur weiteren Umsetzung des Reaktionsgemischs (3) wird dem Amidierungsreaktor (B) frisches Acetoncyanhydrin über die ACH-Zuleitung (1b) zugeführt. Schlaufenreaktor (B) ist hinsichtlich Temperatur, Druck, Verweilzeit und Stoffstromführung vergleichbar mit Schlaufenreaktor (A) ausgestaltet.

Gasförmige Nebenprodukte werden aus Reaktor (B) in Form des Abgas-Stroms (4b) entfernt.

Die erhaltenen Abgas-Ströme (4a, 4b) werden durch Verschaltung zu (5) zusammengeführt und zwecks Verwertung dem nachfolgenden Gasabscheider / Zwischenbehälter (D) zugeführt. Alternativ können die Reaktionsabgase (4a, 4b) als gebündelter Abgasstrom (5a) aus dem Verfahren entfernt werden.

Das erhaltene, flüssige Reaktionsgemisch (6) wird zur maximalen Umsetzung zu MASA einer Konvertierung (C) unterzogen. Die Konvertierung setzt sich typischerweise aus einem oder mehreren Wärmetauschern zusammen, wobei durch gezieltes Aufheizen und anschließendes Verweilen des eintretenden Reaktionsgemischs (6) die Konzentration von MASA im austretenden Produktstrom der Amidierung, im konvertierten Amid-Gemisch (7), maximiert wird.

Das konvertierte Amid-Gemisch (7) wird beispielsweise gravimetrisch dem Gasabscheider / Zwischenbehälter (D) zugeführt. Hier wird das entstehende Abgas von dem viskosen und heißen konvertierten Amid-Gemisch (7) abgetrennt. Das freigesetzte Abgas enthält hauptsächlich Kohlenstoffmonoxid, welches durch Zersetzungsreaktionen entsteht, und daneben Feinsttröpfchen von Methacrylsäureamid-haltigem Reaktionsgemisch. Das Edukthaltige Gesamtabgas (9a) aus dem Gasabscheider / Zwischenbehälter (D) wird daher in die Veresterung (E) weitergeleitet. Das entgaste Amid-Gemisch (8) wird anschließend gepumpt oder gravimetrisch der Veresterung (E) zugeführt.

Der Abgasstrom (5b) der Amidierungsstufen kann gasseitig mit dem Gasabscheidebehälter (D) verbunden sein, und das Gesamtabgas (9a) der Verfahrensschritte (A, B, C, D) kann mit dem Dampfraum der Veresterung (E) verbunden sein. Alternativ kann das Abgas aus (D) zumindest teilweise als Abgas-Strom (9b) aus dem Verfahren entfernt werden.

In der Veresterung (E) werden die benötigten Edukte zur Umsetzung von Methacrylsäureamid zum entsprechenden Ester direkt oder indirekt in Form des entsprechenden Alkohols (10a, 10b) sowie von VE-Wasser (16a, 16b, 16c) zugeführt. Das entgaste Amid-Gemisch (8) wird hierbei durch Einleit- oder Tauchrohre, gepumpt oder gravimetrisch, der Reaktion (E) zugeführt. Eine direkte Alkohol-Zuleitung (10a) (z.B. Methanol für die Herstellung von MMA) erfolgt dabei meist durch abgetauchte Einleitrohre oder statische Mischer im Zulauf zur Veresterung (E).

Weiterhin werden verschiedene Kreislaufströme aus der thermischen Aufarbeitung (F, G, H, L, M, N, O, P) wie in Figur 2 gezeigt mit dem Veresterungsreaktor (E) verbunden.

Die Veresterung wird typischerweise in einem oder mehreren Veresterungsreaktoren (E) durchgeführt, welche mittels Rührwerks oder einer Pumpe durchmischt und gravimetrisch miteinander verbunden sind. Eine weitere Form der Durchmischung stellt sich durch Konvektion ein, welche durch die Zuführung von verdampfbaren Edukten hervorgerufen wird. Die Veresterungsreaktoren sind oftmals mit Wärmetauschern ausgerüstet, um den Wärmeeintrag für die Veresterungsreaktoren zu gewährleisten. Beispielsweise wird der Wärmeeintrag durch Mantelbeheizung, Zwangsumlaufverdampfer oder Direktdampfeinspeisung realisiert.

Die in der Veresterung (E) entstehende Reaktionsmischung (Roh-Ester) wird destillativ als kontinuierlicher Brüdenstrom (12) aus dem Veresterungsreaktor (E) geführt. Hierbei kann der Brüdenstrom (12) auch aus mehreren Reaktoren (E) zusammengeführt sein. Das in den Veresterungsreaktoren zurückbleibende Säuregemisch (11) wird nach intensiver destillativer Entfernung von Rest-Produkt aus der Veresterung ausgeschleust.

Der Brüdenstrom der Veresterung (12) wird in der Roh-Kolonne (F) einer Gegenstrom-Destillation unterzogen. Als Rücklauf der Roh-Kolonne (F) kann der Brüdenstrom am Kopf der Kolonne (F) kondensiert und teilweise zurückgefahren werden. Das hinter der Kondensation erhaltene Abgas (30), welches unter anderem durch die Zuführung von Stoffstrom (9a) generiert wird, kann aus dem Verfahren entfernt und beispielsweise einer Verbrennung zugeführt werden.

Das Sumpfprodukt (13), enthaltend MAS, wird kontinuierlich in den Veresterungsreaktor (E) zurückgefahren. Es ist möglich, das Sumpfprodukt (13) auf mehrere Veresterungskessel der Veresterung (E) zu verteilen.

Der Brüdenstrom (14a) am Kopf der Kolonne (F) enthält den überwiegenden Anteil des Alkylmethacrylats sowie Wasser, Alkohol, Aceton und MAN. Methacrylsäure bildet ein niedrig siedendes Azeotrop mit Wasser und ist ebenfalls im Brüdenstrom (12) enthalten.

Der wässrige und kondensierte Brüdenstrom (14a) am Kopf der Kolonne (F) wird einer Phasentrennung (G) im Phasentrenner I unterzogen, bei der eine organische Phase (15a), enthaltend Alkylmethacrylat, Methanol, Aceton und MAN, und eine wässrige Phase (15b) erhalten werden.

Die organische Phase (15a) wird einer Flüssig- / Flüssig-Extraktion (H) unterzogen, insbesondere, um einen großen Teil des enthaltenen Methanols in die Veresterung (E) zurückzufahren. Hierzu wird die organische Phase (15a) im Gegenstrom mit VE-Wasser (16a) in einer gerührten Extraktionskolonne (H) extrahiert. Die erhaltene wässrige Phase (17b) wird zusammen mit der wässrigen Phase (15b) des Phasentrenners (G) in Stoffstrom (18) vereinigt und in die Veresterung (E) zurückgeführt. Die im Extraktionsschritt (H) erhaltene organische Phase (17a), welche den überwiegenden Teil Alkylmethacrylat und signifikante Anteile an Leicht- und Schwersiedern enthält, wird der weiteren thermischen Aufarbeitung (L,M,N,O) zugeführt.

Die organische Phase (17a) aus Extraktionsschritt (H) wird in einem weiteren Schritt einer Azeotrop-Destillation (L) im Unterdruck unterzogen. Die Azeotrop-Kolonne ist als Abtriebskolonne realisiert, wobei der organische Zulauf (17a) vorgewärmt auf den Kopf der Kolonne (L) geführt wird, welche mit einem Verdampfer indirekt mit Niederdruck-Dampf beheizt wird. Am Kopf der Kolonne (L) wird ein heteroazeotropes Gemisch (22a) enthaltend MMA, Wasser, Methanol, Aceton, MAN und weitere Leichtsieder erhalten. Als Sumpfprodukt (22b) wird gereinigtes Alkylmethacrylat (Roh-Alkylmethacrylat) abgetrennt. Der Brüdenstrom (22a) verlässt die Kolonne (L) dampfförmig und wird im nachgeschalteten Kondensator (M) im Vakuum stufenweise kondensiert.

Die Hauptkondensation in (M) verläuft saugseitig der Vakuumeinheit, wobei ein flüssiges Kondensat (23a) entsteht, welches im Phasentrenner II (N) einer Phasentrennung unterzogen wird. Druckseitig der Kondensationseinheit (M) wird ein weiterer flüssiger Stoffstrom (23d, Vakuumpumpen-Kondensat) erzeugt, welcher als Extraktionsmittel im Extraktionsschritt (P) dient. Das bei der druckseitigen Kondensation entstehende Inertgashaltige Abgas (23e) wird aus dem Verfahren entfernt.

Das flüssige Kondensat (23a) aus (M) wird unter Zugabe von VE-Wasser (16c) in die Phasentrennung (N) geführt und in eine organische Phase (24a) und eine wässrige Phase (24b) getrennt. Die organische Phase (24a) enthält einen gewissen Anteil Alkylmethacrylat und wird über den Kopf der Kolonne (L) zurück in den Destillationsschritt (L) geführt.

Die wässrige Phase (24b) aus (N) ist, entsprechend des zugegebenen Frischwassers (16c), mit wasserlöslichen Komponenten wie Methanol, Aceton und MAN gesättigt und wird zur Vermeidung von Nebenprodukt-Anreicherung in zwei Stoffströme aufgeteilt. Ein Teilstrom (26c) wird in den Veresterungsreaktor (E) in Form eines Kreislaufstroms zurückgefahren. Ein Teilstrom (26b) wird nach einem Extraktionsschritt (P) über (28b) aus dem Verfahren ausgeschleust.

Alternativ zu Stoffstrom (26b, 26c) ist es ebenfalls möglich, den Stoffstrom (24b) vollständig in Form von Stoffstrom (26a) zu verwerfen und aus dem Verfahren auszuschleusen.

Als Auslass für angereicherte Nebenkomponenten dient Stoffstrom (26b), welcher zwecks Rückgewinnung von Alkylmethacrylat einer Extraktionskolonne (P) (PK-Extraktionskolonne) zugeführt wird. Im Extraktionsschritt (P) wird das Kondensat (23d) aus Kondensator (M) als Extraktionsmittel verwendet, wobei die Stoffströme (23d) und (26b) im Gegenstrom geführt werden. Im Extraktionsschritt (P) werden eine wässrige Phase (28b) und eine organische Phase (28a) erhalten, wobei die wässrige Phase (28b) mit der Abfallsäure (11) vermischt und als Spaltsäure (27) vollständig aus dem Verfahren entfernt wird, und wobei die organische Phase (28a) als Wertstoffstrom enthaltend Alkylmethacrylat in die Veresterung (E) zugeführt wird.

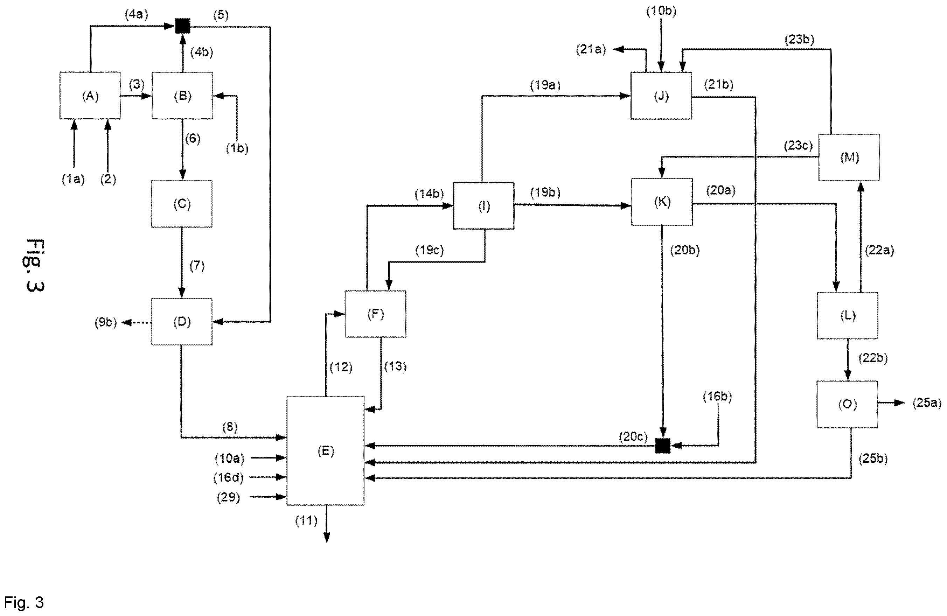

### Ausführungsform des Verfahrens nach Figur 3 (Variante B)

Eine mögliche Ausführungsform des Verfahrens (Variante B) betreffend die Herstellung von Alkylmethacrylat, insbesondere MMA, gemäß Fließbild in Figur 3 ist im Folgenden beschrieben:
Die Amidierung in (A) und (B), die Konvertierung in (C), die Gasabscheidung in (D) und die Veresterung in (E) erfolgen wie in der Ausführungsform nach Figur 2 (Variante A) beschrieben. Weiterhin werden verschiedene Kreislaufströme aus der thermischen Aufarbeitung (F, I, J, K, L, M, O), wie in Figur 3 gezeigt, mit dem Veresterungsreaktor (E) verbunden.

In der Ausführungsform nach Variante B (Figur 3) wird der in der Roh-Kolonne (F) entstehende Brüdenstrom unkondensiert als Brüdenstrom (14b) einem weiteren Destillationsschritt (I) (Roh-Stripper-Kolonne) zugeführt. In der Roh-Stripper-Kolonne (I) wird als Kopf-Fraktion (19a) ein leichtsiedendes Gemisch enthaltend Methanol, Aceton, Metacrylnitril, Methacrylsäureester und Wasser, und als Sumpf-Fraktion (19b) ein azeotropsiedendes Roh-Ester- / Wasser-Gemisch erhalten. Der Rücklauf für Kolonne (I) wird durch einen Partialkondensator erzeugt, welcher so eingestellt wird, dass das leichtsiedende Gemisch (19a) aus Methanol, Aceton, Alkylmethacrylat und Wasser in Form eines Brüdens ausgeschleust wird, während ein Alkylmethacrylat-reiches Gemisch in die Kolonne zurückgefahren werden kann.

Ein Teil der in der Roh-Stripper-Kolonne (I) ablaufenden Flüssigkeit wird im oberen Bereich der Kolonne (I) in Form eines flüssigen Seitenstroms (19c) abgezogen und als Rücklauf für die Roh-Kolonne (F) verwendet.

Das im Sumpf der Roh-Stripper-Kolonne (I) abgezogene azeotrope Gemisch (19b), enthaltend Methacrylatester, Wasser, geringe Mengen Leichtsieder (z.B. Methanol, Aceton) und Schwersieder (z.B. Hydroxyisobuttersäureester), wird abgekühlt und dem Phasentrenner II (K) zugeführt. Das Sumpfprodukt (19b) wird im Phasentrenner (K) in eine organische Phase (20a) und eine wässrige Phase (20b) aufgetrennt. Weiterhin erfolgt im Phasentrenner (K) die Auftrennung des Rücklaufstroms (23c), welcher aus dem Kondensator (M) und dem Kopfprodukt der Azeotrop-Kolonne (L) resultiert, in eine organische Phase (20a) und eine wässrige Phase (20b).

Die wässrige Phase (20b) der Phasentrennung (K) enthaltend Wasser, gesättigt mit Methanol, Aceton und Methacrylsäureester, wird mit VE-Wasser (16b) vermischt und in Form eines vereinigten Rücklaufstrom (20c) der Veresterung (E) zugeführt. Der Rücklaufstrom (20c) kann insbesondere dazu dienen, den Wasserbedarf der Veresterung zu decken und Edukte zurückzugewinnen.

Der Brüdenstrom (19a), welcher die Roh-Stripper-Kolonne (I) verlässt, wird in einen Waschschritt (J) (Abgas-Waschkolonne) geführt und mit frischem Alkohol (10b), z.B. Methanol, als Waschmedium gewaschen, wodurch der Gasstrom weitgehend von Alkylmethacrylat befreit wird. Die Abgas-Waschkolonne (J) wird im Gegenstrom betrieben. Alkylmethacrylat-haltige Abluft (23b) aus Kondensator (M) wird ebenfalls der Abgas-Waschkolonne (J) zugeführt, um Alkylmethacrylat zurückzugewinnen.

Im Sumpf der Abgas-Waschkolonne (J) wird der organische Strom (21b), enthaltend Methanol und Alkylmethacrylat, erhalten, welcher in die Veresterung (E) zurückgeführt wird. Der organische Rücklaufstrom (21b) kann hierbei auf verschiedene Veresterungsreaktoren verteilt werden.

Am Kopf der Abgas-Waschkolonne (J) wird ein Abgas-Strom (21a) erhalten, welcher MAN, Dimethylether und Methylformiat sowie Sättigungskonzentrationen an Methanol und Aceton aufweist und wenig Alkylmethacrylat enthält. Durch Einstellung des Verhältnisses Methanol (10b) zu Brüden (19a) und der Kopftemperatur in (J) kann die Zusammensetzung des Abgas-Stroms (21a) variiert werden. Der Abgas-Strom (19a) enthaltend Methanol, Aceton und MAN wird vollständig aus dem Verfahren ausgeschleust.

Die organische Phase (20a) der Phasentrennung (K) wird in die Azeotrop-Kolonne (L) geführt. Die organische Phase (20a), welche den überwiegenden Anteil Alkylmethacrylat und signifikante Anteile an Leicht- und Schwersiedern enthält, wird der weiteren thermischen Aufarbeitung (L, M, N, O) zugeführt.

Die thermische Aufarbeitung (L, M, N, O) erfolgt im Wesentlichen wie oben in der Ausführungsform nach Variante A (Figur 2) beschrieben. Im Unterschied zu Variante A werden die im Kondensator (M) entstehenden Stoffströme (23b) und (23c) auf die Verfahrensschritte (J) und (K) zurückgeführt. Es wird ein vollständig kondensiertes und azeotropes Leichtsieder-Gemisch (23c), welches aus der Kopf-Fraktion der Azeotrop-Kolonne (L) über Kondensator (M) erhalten wird, und welches typischerweise zwei flüssige Phasen aufweist, in die Phasentrennung (K) des vorgelagerten Schritts geführt. Der Stoffstrom (23c) setzt sich aus den Stoffströmen (23a) und (23d) der Variante A zusammen. Das in der Kondensations- / Vakuumeinheit (M) entstehende Abgas (23b) wird in die Waschkolonne (J) geführt, wobei insbesondere Wertstoffe, wie Alkylmethacrylat, zurückgewonnen werden.

Die Erfindung wird anhand der folgenden Beispiele weiter beschrieben. Dabei demonstriert Beispiel A1 (Vergleichsbeispiel) den Betrieb eines Verfahrens zur Herstellung von Methylmethacrylat mit einer nicht erfindungsgemäßen Schwefelsäurekonzentration (100.3 Gew.-%) und einer geringen Ausschleusung der Methacrylnitril-haltigen wässrigen Phase aus Phasentrenner II, verbunden mit einer niedrigen MMA-Gesamtausbeute unter Erhalt eines MMA-Produkts mit hohem Gehalt des Nebenprodukts MAN.

Das erfindungsgemäße Beispiel A2 beschreibt die Herstellung von Methylmethacrylat mit den beanspruchten Merkmalen unter Erzielung einer hohen Gesamtausbeute an MMA, gekennzeichnet durch ein verminderte MAN-Bildung in der Amidierung und Konvertierung sowie durch eine Moderierung der stationären MAN-Konzentration im Aufarbeitungsteil durch eine kontrollierte Ausschleusung, verbunden mit der Erzielung eines niedrigen MAN-Gehalts im Zielprodukt MMA.

Beispiel A3 (Vergleichsbeispiel) verdeutlicht den Betrieb analog Vergleichsbeispiel 1, jedoch zusätzlich ohne Extraktion des ausgeschleusten wässrigen MAN-haltigen Stroms, was nochmals erhöhte MMA-Verluste mit sich bringt.

Weitergehend beschreibt Beispiel B1 (erfindungsgemäß) die Herstellung von Methylmethacrylat mit den beanspruchten Merkmalen unter Erzielung einer hohen Gesamtausbeute an MMA, gekennzeichnet durch eine verminderte MAN-Bildung in der Amidierung und Konvertierung und eine ausschließlich destillative Abtrennung von Nebenprodukten (als Brüden).

### Beispiele

### Beispiele A1, A2 und A3 gemäß Fig. 2 (Variante A)

Die Herstellung von Methylmethacrylat, umfassend die Umsetzung von Acetoncyanhydrin mit Schwefelsäure in der Amidierung / Konvertierung ((A), (B), (C), (D)), der anschließenden Veresterung (E) mit Methanol sowie der destillativen und extraktiven Aufarbeitung ((F), (G) ,(H) ,(L) ,(O) ,(M) ,(N), (P)) des Methylmethacrylat-Produkts, erfolgte anhand der Ausführungsform nach Figur 2 wie oben beschrieben (Variante A).

Es erfolgte eine Bilanzierung und Betrachtung der Ausschleusung an Methacrylnitril (MAN) und Aceton über (28b), Abluft-Ströme (30) und (23e), und über das MMA-Produkt (25a) (siehe Fließschema nach Figur 2).

Im Folgenden werden Vergleichsbeispiele (Beispiel A1 und A3) unter Verwendung von Schwefelsäure mit einer Konzentration von 100,3 Gew.-% (formal 0,3 Gew.-% freies SO₃) und ein erfindungsgemäßes Beispiel (Beispiel A2) unter Verwendung von Schwefelsäure mit einer Konzentration von 99,7 Gew.-% (0,3 Gew.-% Wasser) beschrieben.

Der Wassergehalt der ACH-Zuleitungsströme (1a) und (1b) berechnet sich aus der Differenz zum ACH-Gehalt, der mittels HPLC ermittelt wird, respektive über eine spezifisch für Wasser quantitative und selektive Analyse mittels Gaschromatographie (mit Wärmeleitfähigkeitsdetektor).

Der Wassergehalt in der Schwefelsäure-Zuleitung (2) berechnet sich aus der Differenz zum Schwefelsäuregehalt, der durch Messung der Dichte und Schallgeschwindigkeit ermittelt wird.

Die allgemeine Durchführung des Verfahrens nach Figur 2 (Variante A) ist im Folgenden beschrieben, wobei Unterschiede und Ergebnisse in den Tabellen 1 bis 3 dargestellt sind.

Allgemeine Verfahrensdurchführung:
1a. Reaktionsstufen (A), (B), (C), (D) und (E)
5000 kg/h Acetoncyanhydrin, mit einer Zusammensetzung von 98,8 Gew.-% Acetoncyanhydrin; 0,25 Gew.-% Aceton; 0,65 Gew.-% Wasser sowie freier Schwefelsäure wurde im Massenverhältnis von 75 / 25 aufgeteilt, sodass ein Strom (1a) mit 3750 kg/h und ein Strom (1b) mit 1250 kg/h erhalten wird. Zulaufstrom (1a) wurde anschließend auf den ersten Amidierungsreaktor (A) aufgegeben.

Der Schlaufenreaktor (A) setzte sich aus den mittels Rohrleitung verbundenen Elementen Kreislaufpumpe, statischer Mischer, Wärmeübertrager, Kühler sowie einem Gasabscheider zusammen. Im Reaktor (A) wurde ein Umwälzvolumenstrom von 350 m³/h eingestellt, so dass ein effektiver Wärmeübergang sowie eine effektive Durchmischung und Gasabscheidung möglich waren. Der gesamte Reaktorkreislauf wurde bei etwa 95 °C und 990 mbar(a) im leichten Unterdruck betrieben.

Zulaufstrom (1a) wurde kontinuierlich und mit einer Temperatur von etwa 20 °C dem beschriebenen Reaktorkreislauf (A) zugeführt und eingemischt.

Die für die optimale Umsetzung des Reaktionsgemischs in den Reaktoren (A) und (B) notwendige Schwefelsäuremenge (2), welche eine Konzentration gemäß Tabelle 1 aufwies, wurde lastabhängig in dem angegebenen Massenverhältnis zur Gesamtmenge ACH (1a+1b) dem Reaktor (A) zugeführt. Hierdurch wurde in Reaktor (A) ein Schwefelsäureüberschuss (Schwefelsäure- / ACH-Verhältnis von 2,6 kg/kg) realisiert.

Das erhaltene Anrührgemisch (3), enthaltend Sulfoxyisobuttersäureamid, Methacrylsäureamid und Schwefelsäure, wurde anschließend in den zweiten Amidierungsreaktor (B) überführt, während das in Reaktor (A) abgetrennte Abgas (4a) in Richtung Konvertierung (D) geschickt wurde.

Reaktor (B) war analog zu Reaktor (A) aufgebaut und wurde bei den gleichen physikalischen Bedingungen und Parametern betrieben. Das durch Nebenreaktion gebildete Abgas (4b) wurde mittels eines Gasabscheiders von der Reaktionsmischung getrennt. Die Abgase der Amidierung (4a) und (4b) wurden anschließend zusammengeführt und in Form von Abgasstrom (5b), dessen Menge etwa 60 m³/h betrug, einem weiteren Gasabscheider / Zwischenbehälter (D) zugeführt.

Der ACH-Strom (1b) wurde anschließend in die Reaktionsmischung des zweiten Reaktors (B) aufgegeben. Das sich in der Reaktionsmischung einstellende Massenverhältnis H₂SO₄ / ACH ist in Tabelle 1 angegeben.

Im Ablauf der Amidierungsreaktoren (A, B) wurde ein 95 °C heißes Reaktionsgemisch (6), enthaltend Sulfoxyisobuttersäureamid (SIBA), Methacrylsäureamid (MASA) und Hydroxyisobuttersäureamid (HIBA), gelöst in schwefelsaurer Reaktionsmatrix erhalten. Dieses Gemisch wurde anschließend einem Konvertierungsschritt (C) unterzogen. In diesem wurde das Reaktionsgemisch in kurzer Zeit auf 155 °C erhitzt und anschließend in einer Verweilzeitstrecke thermisch umgewandelt.

Das Methacrylsäureamid-angereicherte Reaktionsgemisch (7) wurde nach der Konvertierung (C) gravimetrisch einem weiteren Gasabscheider bzw. Zwischenbehälter (D) zugeführt, welcher bei leichtem Unterdruck von ca. 950 mbara und in einer Temperatur von 155 °C betrieben wurde.

Im Gasabscheider / Zwischenbehälter (D) wurde das in der Reaktionsmischung enthaltende Gas abgetrennt und mit Abgas (5b) zusammengeführt. Es wurde ein Gesamtabgas (9a) erhalten, welches der Veresterung (E) in gasförmiger Form zugeführt wurde. Menge und Zusammensetzung von (9a), hinsichtlich der Nebenprodukte Aceton und MAN, sind in Tabelle 1 zusammengefasst.

Weiterhin wurde nach der Gasabscheidung (D) ein Stoffstrom (8) erhalten, dessen Massenstrom und Zusammensetzung in Tabelle 1 angegeben sind. Die erhaltene Menge an umsetzbaren Edukten (MASA + MAS), welche in die Veresterung geführt wurden, und die auf ACH bezogene Amidierungsausbeute (MASA + MAS) sind in Tabelle 1 dargestellt.

Der Methacrylsäureamid-haltige Stoffstrom (8) enthielt neben den genannten Hauptkomponenten als wichtige Nebenprodukte Aceton und MAN in den in Tabelle 1 angegeben Mengen. Die Gesamtstoffströme an Aceton und MAN, welche über (8) und den gasförmigen Zulaufstrom (9a) der Veresterung (E) zugeführt wurden, sind in Tabelle 1 angegeben.

Die flüssige und über 150 °C heiße Reaktionsmischung (8) wurde der Veresterung (E) zugeführt, wobei die Zuführung in eine Reaktorkaskade, bestehend aus drei mantelbeheizten und annähernd ideal durchmischten Reaktorkesseln durch freien Ablauf mittels eines Tauchrohrs erfolgte.

Stoffstrom (8) wurde im Verfahrensschritt (E) mit insgesamt 1540 kg/h Methanol (10a), 590 kg/h MMA- / MeOH-Gemisch (29), 500 kg/h Direktdampf (16d) sowie 1700 kg/h Wasser (Zulaufströme (16a) und (16c)) bei ca. 120 ± 5 °C und leichtem Überdruck von 50-150 mbarg zur Reaktion gebracht. Das sich stationär einstellende Stoffgemisch (29) bestand im Mittel aus 25 Gew.-% Methylmethacrylat und 75 Gew.-% Methanol sowie Wasser und wurde zur Verwertung von Methanol als Edukt in der Veresterungsreaktion und Methylmethacrylat als Produkt (E) zugeführt.

Durch geeignete Verschaltung der Eduktströme (10a), (16a), (16c); (16d), (29) und der Kreislaufströme (13), (18), (25b), (26c), (28a) in der Veresterung (E) wurde erreicht, dass in jedem der Veresterungskessel eine lokaler, stöchiometrischer Überschuss von Methanol und Wasser bezogen auf die zu Methylmethacrylat umsetzbaren Stoffe Methacrylsäureamid sowie Methacrylsäure vorlag.

In einer Nebenreaktion der Veresterung wurde ein Teil der zugeführten Menge des MAN durch Hydrolyse zu Methacrylsäureamid umgewandelt und somit dem Prozess entzogen. Je höher die zugeführte Konzentration an MAN, desto höher fällt auch der Hydrolysegrad aus. Auf diese Weise wurden in (E) die in Tabelle 1 angegebenen Mengen (MAN(hydrol.)) an MAN hydrolysiert.

Am Austritt der Reaktoreinheit (E) wurde eine mit Direktdampf betriebene Nachverdampfung geschaltet, welche den Anteil an Monomeren im Säuregemisch auf einen Gehalt von < 0,1 Gew.-% MMA, < 0,1 Gew.-% MAS, < 0,1 % Gew.-MASA sowie MAN und Aceton gemäß Tabelle 1 reduziert, während der Wassergehalt des ablaufenden Säuregemischs annähernd konstant gehalten wurde. In der Abfallsäure (11) waren ebenfalls weitere schwerflüchtige organische Nebenprodukte enthalten, welche gelöst als TOC (total organic carbon) und zu einem gewissen Anteil auch als polymerer Feststoff, ausgeschleust werden. Aufgrund von Nebenreaktionen in der Veresterung (E) waren die in Tabelle 1 angegebenen Mengen an sulfoniertem Aceton (sulfo Aceton) in Form von TOC in der Prozesssäure (11) enthalten. Die Abfallsäure enthielt im Wesentlichen NH₄HSO₄, H₂SO₄ und Wasser. Der TOC-Gehalt von (11) lag im Mittel bei 2-3 Gew.-%. Menge und Zusammensetzung der Abfallsäure (11) sind in Tabelle 1 zusammengefasst.

Der Energieeintrag in (E) zur kontinuierlichen Verdampfung der in (E) erhaltenen Produkte erfolgte mittels 10 barg Heizdampf.

### 1b. Vorreinigung (F), (G), (H)

Das bei der Veresterungsreaktion gebildete Roh-Produkt sowie das über (29) eingetragene Methylmethacrylat (MMA) wurde kontinuierlich in Form eines Brüdenstroms (12) der Veresterungskaskade (E) entzogen. Hierzu waren die Veresterungsreaktoren dampfseitig mit einer Brüden-Leitung miteinander verbunden, so dass der Brüdenstrom (12) als Summenstrom der Reaktoren II erhalten wurde. Gemäß Dampf / Flüssig-Gleichgewicht der Reaktionsmischungen stellte der Brüdenstrom (12) eine heteroazeotrope Zusammensetzung, enthaltend MMA, Wasser, MeOH, MAS, Aceton und MAN wie in Tabelle 2 angegeben, dar.

Zusätzlich zu Brüdenstrom (12) wurde auch der Abgasstrom der Amidierung (9a) dem Sumpf der Rohkolonne (F) zugeführt. Brüdenstrom (12) wurde anschließend einer Gegenstrom-Destillation unterzogen, indem der dampfförmige Strom (12) sowie der gasförmige Strom (9a) im Sumpfbereich einer Kolonne (F) aufgegeben wurden. Am Kopf der Kolonne (F) wurde in Kondensatoren, welche mittels Kühlwassers und Kaltwasser betrieben wurden, vollständig kondensiert. Die zweiphasigen Destillate wurden vereinigt und ein Teilstrom als Rücklauf in Rohkolonne (F) geführt.

Das hinter der Kondensation erhaltene Abgas (30), welches unter anderem durch die Zuführung von Stoffstrom (9a) generiert wurde, wurde entfernt und einer Verbrennung zugeführt. Über (30) wurden MAN und Aceton aus dem Prozess in den in Tabelle 2 angegebenen Mengen ausgetragen.

Entsprechend des Rücklaufverhältnisses wurden am Kopf der Kolonne (F) ein flüssiger Destillat-Strom (14a) sowie ein flüssiger Sumpfstrom (13) erhalten. Der Sumpfstrom (13) wurde kontinuierlich in die Veresterungsreaktion zurückgeführt. Der heteroazeotrope Destillatstrom (14a) wurde zwecks Flüssig- / Flüssig-Phasentrennung einem Phasentrenner (G) zugeführt, in welchem zwei Produktströme erhalten wurden. Die organische, leichte Phase (15a), enthaltend MMA, Wasser, Methanol, Aceton, MAS, MAN sowie Schwer- und Leichtsieder wurde einer Flüssig- / Flüssig-Extraktion (H) unterzogen.

Die wässrige, schwere Phase (15b) wurde zunächst mit der wässrigen Raffinat-Phase (17b) zu einem Rücklaufstrom (18) zusammengefasst, welcher Wasser, Methanol, MMA, Aceton, MAS, MAN sowie Schwer- und Leichtsieder aufweist. Stoffstrom (18) wurde analog zu Stoffstrom (13) kontinuierlich in die Veresterungsreaktion (E) zurückgeführt. Mengen und Zusammensetzung von (13), (14a), (15a), (15b) und (18) sind in Tabelle 2 zusammengefasst.

Im Extraktionsschritt (H) wurde dem Stoffstrom (15a) deionisiertes Wasser (16a) zugeführt, um weitere wasserlösliche Komponenten aus dem vorliegenden Roh-MMA zu entfernen. Zu diesem Zweck wurde am Kopf der Scheibenextraktionskolonne (H) deionisiertes Wasser kontinuierlich im Gegenstrom zu Stoffstrom (15a) zugeführt, so dass im Sumpf der Kolonne (H) ein wässriger Ablaufstrom (17b) sowie ein vorgereinigtes Roh-MMA (17a) erhalten wurde. Der Roh-MMA-Strom (17a) enthielt MMA, Wasser, Methanol, Aceton, MAS, MAN sowie Schwer- und Leichtsieder. Mengen und Zusammensetzung von (17a) und (17b) sind in Tabelle 2 zusammengefasst.

### 1c. Feinreinigung (L), (O), (M), (N), (P)

Stoffstrom (17a) wurde anschließend einer destillativen Aufbereitung (L) unterzogen. Zur Entfernung von Schwer- und Leichtsiedern wurde Stoffstrom (17a) dem Kopfbereich einer im Vakuum (300 mbara) betriebenen Azeotrop-Kolonne (L) zugeführt, welche mit Heizdampf indirekt beheizt wurde. Es wurde ein Leichtsieder-angereicherter, heteroazeotroper Brüdenstrom (22a) von einem Methylmethacrylat-angereicherten Sumpfstrom (22b) abgetrennt. Der Brüdenstrom (22a) enthielt MMA, Wasser, MAN, Aceton und weitere Leichtsieder. Der Sumpfstrom (22b), enthielt MMA, MAS, Schwersieder, MAN und Aceton. Die Mengen und Zusammensetzungen sind in Tabelle 3 zusammengefasst.

Der Brüdenstrom (22a) wurde am Austritt der Kolonne rohrseitig einer Kondensations- / Vakuum-Einheit (M) zugeführt, welche den Brüdenstrom (22a) zunächst vakuumseitig einer Hauptkondensation unterzog, anschließend das Restgas in einer Vakuumpumpe verdichtete und auf der Druckseite des Verdichtungsprozesses nochmals nachkondensierte.

Das nach der Hauptkondensation erhaltene heteroazeotrope Destillat (23a) wurde zur weiteren Verarbeitung einer Phasentrennung (N) unterzogen, während das druckseitig gewonnene Pumpendestillat (23d) der Veresterungsreaktion (E) zugeführt wurde. Das Pumpendestillat (23d) enthielt Leichtsieder, MMA, Aceton, Methanol, Wasser und MAN. Mengen und Zusammensetzungen sind in Tabelle 3 beschrieben.

Das gasseitig, hinter der Nachkondensation erhaltene Prozessabgas (23e) wurde kontinuierlich aus dem Prozess ausgeschleust. Das Prozessabgas (23e) enthielt Leichtsieder und inerte, unter gegebenen Bedingungen chemisch unreaktive Stoffe sowie MAN und Aceton. Mengen und Zusammensetzungen sind in Tabelle 3 beschrieben.

Zur Verbesserung der Phasentrennung wurde dem Destillatstrom (23a) im Phasentrenner (N) deionisiertes Wasser (16c) zugeführt, so dass eine organische Phase (24a) und eine wässrige Phase (24b) erhalten wurden. Die leichte, organische Phase (24a) wurde hierbei kontinuierlich als Rücklauf auf den Kopf der Kolonne (L) im Kreis geführt, während die wässrige Phase (24b), enthaltend Aceton und MAN, dem nächsten Prozessschritt zugeführt wurde. Mengen und Zusammensetzungen sind in Tabelle 3 beschrieben.

Im Vergleichsbeispiel A1 wurde der Stoffstrom (24b) anschließend in einem festen Massenverhältnis von 80 / 20 als Stoffstrom (26b) sowie Stoffstrom (26c) aufgeteilt. 80 % von Stoffstrom (24b) wurden als (26c) direkt in die Veresterungsreaktion zurückgeführt. 20 % von Stoffstrom (24b) wurden über den Zwischenschritt einer Flüssig- / Flüssig-Extraktion (P), zur Rückgewinnung von MMA, in Form von Stoffstrom (28b) aus dem Prozess ausgeschleust. Auf diesem Weg wurden MAN und Aceton aus dem Prozess ausgeschleust und damit deren Anreicherung im Prozess reduziert, kontrolliert und gesteuert. Mengen und Zusammensetzungen sind in Tabellen 3 und 4 beschrieben.

Im erfindungsgemäßen Beispiel A2 wurde Stoffstrom (24b) zunächst in einem Massenverhältnis von 50 / 50 in Stoffstrom (26b) und Stoffstrom (26c) aufgeteilt. Stoffstrom (26c) wurde direkt in die Veresterungsreaktion (E) zurückgeführt. Stoffstrom (26b) wurde über den Zwischenschritt einer Flüssig- / Flüssig-Extraktion (P) als (28b) teilweise aus dem Prozess ausgeschleust.

Im Vergleichsbeispiel A3 wurde der Stoffstrom (24b) in einem festen Massenverhältnis von 80 / 20 als Stoffstrom (26a) sowie Stoffstrom (26c) aufgeteilt. 80 % von Stoffstrom (24b) wurden als (26c) direkt in die Veresterungsreaktion zurückgeführt. 20 % von Stoffstrom (24b) wurden als Strom (26a) direkt aus dem Prozess entfernt und keinem Extraktionsschritt (P), zur Rückgewinnung von MMA, zugeführt. Auf diesem Weg wurden MAN und Aceton aus dem Prozess ausgeschleust. Mengen und Zusammensetzungen sind in Tabelle 3 beschrieben.

Alternativ kann Stoffstrom (24b) aber auch vor der Aufteilung in (26b) / (26c) vollständig oder teilweise in Form eines Austragstroms (26a) aus dem Prozess entfernt werden. In diesem Fall würde ein noch größerer Anteil des eingetragenen MAN sowie Acetons aus dem Prozess entfernt werden, als nachfolgend beschrieben ist.

In der Extraktion (P) wurde mit Hilfe des organischen Pumpendestillat (23d) als Extraktionsmittel der wässrige Produktstrom (26b) behandelt, um den Restgehalt an MMA in Stoffstrom (26b) vor der Ausschleusung zu reduzieren. Zu diesem Zweck wurde am Kopf der Scheibenextraktionskolonne (P) Stoffstrom (26b) und im Sumpf Stoffstrom (23d) zugeführt. In (P) wurden ein wässriges Raffinat (28b) im Sumpf der Kolonne (P) und ein organischer, mit Methylmethacrylat angereicherter Extrakt-Strom (28a) erhalten. Der Raffinat-Strom (28b) enthielt Wasser, Methanol, Aceton, Methylmethacrylat, MAN und Leicht- sowie Schwersieder. Raffinat-Strom (28b) wurde anschließend mit dem Abfallsäure-Strom (11) abgemischt und in Form des resultierenden Stoffstroms (27) aus dem Prozess ausgeschleust. Über (28b) wurden MAN und Aceton aus dem Prozess ausgeschleust. Der Extrakt-Strom (28a) enthielt Methylmethacrylat, Aceton, Methanol, MAN sowie weitere Leicht- und Schwersieder und wurde kontinuierlich in die Reaktionszone der Veresterung (E) zurückgeführt. Mengen und Zusammensetzungen sind in Tabelle 3 beschrieben.

Die weitere Aufarbeitung des Sumpfprodukts (22b) aus der Azeotrop-Kolonne (L) erfolgte in den Beispielen A1, A2 und A3 in gleicher Weise.

Das in der Azeotrop-Kolonne (L) erhaltene, Leichtsieder-freie Sumpfprodukt (22b) wurde zur weiteren Aufreinigung einer Unterdruck-Rektifikation (O) unterzogen, welche bei 180 mbar(a) arbeitete und einen Verstärkungs- sowie Abtriebsteil aufwies. Stoffstrom (22b) wurde mittig auf die Rein-Kolonne (O) aufgegeben, wobei dieser, entsprechend des sich einstellenden Gleichgewichts, in eine reine Destillatphase (25a) sowie einen Schwersieder-angereicherten Sumpfstrom (25b) aufgetrennt wurde.

Der Sumpfstrom (25b), welcher Methylmethacrylat, Methacrylsäure und Schwersieder enthielt, wurde kontinuierlich in den Veresterungs-Reaktionsschritt (E) zurückgeführt.

Der erhaltene Brüdenstrom in (O) wurde vollständig kondensiert. Das hierbei erhaltene Abgas von etwa 2 m³/h wurde dem Verfahrensschritt (M) zugeführt. Das Destillat wurde entsprechend des benötigten Rücklaufverhältnisses aufgeteilt, so dass die in Tabelle 3 angegebene Mengen MMA-Reinprodukt (25a) mit > 99,9 Gew.-% Reinheit erhalten wurden. Das MMA-Reinprodukt (25a) enthielt Aceton und MAN in den in Tabelle 3 angegeben Mengen. Im MMA-Produktstrom wurden MAN und Aceton aus dem Prozess entfernt.

### 1d. Verfahrensbedingungen und Ergebnisse

**Tabelle 1: Beispiele A1 - A3 - Daten für Amidierung, Konvertierung und Veresterung ((A), (B), (C), (D), (E))**

| | | | A1* | A2 | A3* |
|---|---|---|---|---|---|
| (1a) | ACH | kg/h | 3750 | 3750 | 3750 |
| (1b) | ACH | kg/h | 1250 | 1250 | 1250 |
| (1a) + (1b) | ACH ges. | kg/h | 5000 | 5000 | 5000 |
| (1a) : (1b) | | kg/kg | 75:25 | 75:25 | 75:25 |
| (1a) + (1b) | ACH Konz. | Gew% | 98,8 | 98,8 | 98,8 |
| (2) | H₂SO₄ Konz. | Gew% | 100,3 | 99,7 | 100,3 |
| (2) | Gesamt H₂SO₄ | kg/h | 8100 | 8100 | 8100 |
| (A) | H₂SO₄/ACH | kg/kg | 2,16 | 2,16 | 2,16 |
| (A)+(B) | H₂SO₄/ACH | kg/kg | 1,62 | 1,62 | 1,62 |
| (5b) | Gesamt | m³/h | 60 | 50 | 60 |
| (9a) | Gesamt | m³/h | 75 | 65 | 75 |
| (9a) | Aceton | g/m³ | 19,0 | 11,0 | 19,0 |
| (9a) | MAN | g/m³ | 2,0 | 1,1 | 2,0 |
| (9a) | Aceton | kg/h | 2 | 1 | 2 |
| (9a) | MAN | kg/h | 0,2 | 0,1 | 0,2 |
| (8) | Gesamt | kg/h | 12.914 | 13.015 | 12.914 |
| (8) | MASA | Gew.% | 35,6 | 35,4 | 35,6 |
| (8) | MAS | Gew.% | 0,4 | 1,0 | 0,4 |
| (8) | HIBA | Gew.% | 0,4 | 0,4 | 0,4 |
| (8) | Aceton | Gew.% | 0,25 | 0,2 | 0,25 |
| (8) | MAN | ppm | 500 | 354 | 500 |
| (8) | MASA+MAS (Edukte f. Veresterung) | kg/h | 4649 | 4734 | 4649 |
| (8) + (9a) | Aceton | kg/h | 34 | 26,5 | 34 |
| (8) + (9a) | MAN | kg/h | 6,5 | 4,7 | 6,5 |
| Amid-Ausbeute | MASA+MAS bez. auf ACH | % | 93,0 | 94,7 | 93,0 |
| (29) | Gesamt | kg/h | 590 | 590 | 590 |
| (10a) | Gesamt | kg/h | 1540 | 1570 | 1540 |
| (16d) | Gesamt | kg/h | 500 | 500 | 500 |
| (16a) + (16 c) | Wasser | kg/h | 1700 | 1700 | 1700 |
| MAN (hydrol.) | Verminderung MAN in (E) | kg/h | 1,5 | 1,0 | 1,5 |
| (11) | Gesamt | kg/h | 11.504 | 11.410 | 11.515 |
| (11) | MAN | ppm | 110 | 60 | 100 |
| (11) | TOC | Gew.% | 2,3 | 1,5 | 2,4 |
| (11) | Feststoff TOC | Gew.% | 0,2 | 0,1 | 0,2 |
| (11) | Sulfo Aceton in TOC | ppm | 470 | 360 | 465 |
| (11) | Aceton | kg/h | 5,0 | 4,0 | 5,0 |
| (11) | MAN | kg/h | 1,2 | 0,65 | 1,1 |

| | | | | | |
|---|---|---|---|---|---|
| *Vergleichsbeispiel | | | | | |

**Tabelle 2: Beispiele A1-A3 - Daten für Aufarbeitung / Vorreinigung (F), (G), (H))**

| | | | A1* | A2 | A3* |
|---|---|---|---|---|---|
| (12) | Gesamt | kg/h | 10810 | 9875 | 10698 |
| (12) | MMA | Gew% | 67 | 74 | 63 |
| (12) | Wasser | Gew% | 22 | 16 | 22 |
| (12) | MeOH | Gew% | 6,5 | 5 | 6,7 |
| (12) | MAS | Gew% | 3 | 3 | 2,8 |
| (12) | Aceton | Gew% | 1 | 0,8 | 1 |
| (12) | MAN | Gew% | 0,15 | 0,09 | 0,1 |
| (30) | Gesamt | kg/h | 83 | 74 | 85 |
| (30) | Aceton | kg/h | 1 | 0,5 | 1 |
| (30) | MAN | kg/h | 0,1 | 0,1 | 0,1 |
| (14a) | Gesamt | kg/h | 10.105 | 9.080 | 10.081 |
| (15a) | Gesamt | kg/h | 8.156 | 7.638 | 8.120 |
| (15a) | MMA | Gew.% | 83,9 | 88,8 | 84,1 |
| (15a) | Wasser | Gew.% | 4,1 | 2,9 | 4,1 |
| (15a) | MeOH | Gew.% | 5,4 | 3,4 | 5,4 |
| (15a) | MAS | Gew.% | 1,8 | 1,7 | 1,8 |
| (15a) | Aceton | Gew.% | 2,1 | 1,1 | 1,9 |
| (15a) | MAN | Gew.% | 0,4 | 0,1 | 0,3 |
| (15b) | Gesamt | kg/h | 1.949 | 1.447 | 1960 |
| (18) | Gesamt | kg/h | 3.251 | 2.583 | 3261 |
| (18) | Wasser | Gew.% | 75,0 | 82,0 | 74,7 |
| (18) | MeOH | Gew.% | 17,0 | 12,5 | 17,8 |
| (18) | MMA | Gew.% | 4,0 | 3,4 | 5,0 |
| (18) | MAS | Gew.% | 0,4 | 0,2 | 0,4 |
| (18) | Aceton | Gew.% | 1,5 | 0,8 | 1,4 |
| (18) | MAN | ppm | 470 | 100 | 330 |
| (17a) | Gesamt | kg/h | 7.754 | 7.398 | 7.719 |
| (17a) | MMA | Gew.% | 89,5 | 91,2 | 87,7 |
| (17a) | Wasser | Gew.% | 2,9 | 2,3 | 3,0 |
| (17a) | MeOH | Gew.% | 1,2 | 1,9 | 3,0 |
| (17a) | MAS | Gew.% | 1,8 | 1,4 | 1,8 |
| (17a) | Aceton | Gew.% | 1,9 | 1,0 | 1,7 |
| (17a) | MAN | Gew.% | 0,4 | 0,1 | 0,3 |

| | | | | | |
|---|---|---|---|---|---|
| *Vergleichsbeispiel | | | | | |

**Tabelle 3: Beispiele A1-A3 - Daten für Aufarbeitung / Feinreinigung (L), (O), (M), (N), (P)**

| | | | A1* | A2 | A3* |
|---|---|---|---|---|---|
| (22b) | Sumpf (L) | kg/h | // | // | // |
| (22b) | MMA | Gew.% | 96,0 | 96,7 | 95,8 |
| (22b) | MAS | Gew.% | 2,0 | 1,5 | 2,0 |
| (22b) | Schwersieder | Gew.% | <2,0 | 1,8 | <2,0 |
| (22b) | Aceton | ppm | 10 | <10 | 9 |
| (22b) | MAN | ppm | 138 | 37 | 98 |
| (23a) | Kondens. Brüden | kg/h | 7184 | 8238 | 7282 |
| (23a) | MMA | Gew.% | 61 | 75 | 64 |
| (23a) | Wasser | Gew.% | 8,1 | 5,4 | 7,8 |
| (23a) | Aceton | Gew.% | 11,5 | 7,3 | 10,6 |
| (23a) | MAN | Gew.% | 8,4 | 5,6 | 7,4 |
| (23a) | MAN | kg/h | 603 | 461 | nv |
| (23d) | PK | kg/h | // | // | // |
| (23d) | Leichtsieder | Gew.% | 33 | >30 | 33 |
| (23d) | MMA | Gew.% | 30 | 40 | 32 |
| (23d) | MeOH | Gew.% | 15 | 11 | 15 |
| (23d) | Wasser | Gew.% | 1,4 | 1,4 | 1,4 |
| (23d) | Aceton | Gew.% | 17,0 | 11,0 | 16,2 |
| (23d) | MAN | Gew.% | 5,9 | 3,0 | 5,2 |
| (23e) | Abgas | kg/h | 28 | 23 | 27 |
| (23e) | Aceton | kg/h | 1,0 | 1,0 | 1,1 |
| (23e) | MAN | kg/h | 0,1 | 0,1 | 0,1 |
| (24a) | Gesamt | kg/h | 6428 | 7795 | 6553 |
| (24a) | Wasser | Gew.% | 5,5 | 3,5 | 5,3 |
| (24a) | MeOH | Gew.% | 6,3 | 3,6 | 6,1 |
| (24a) | MMA | Gew.% | 66,7 | 78,6 | 70,0 |
| (24a) | Aceton | Gew.% | 10,7 | 6,9 | 9,9 |
| (24a) | MAN | Gew.% | 8,9 | 5,8 | 8,1 |
| (24a) | MAN | kg/h | 572 | 452 | nv |
| (24b) | Gesamt | kg/h | 1.355 | 1.043 | 1.328 |
| (24b) | Wasser | Gew.% | 60,5 | 73,7 | 61,9 |
| (24b) | MeOH | Gew.% | 18,6 | 13,8 | 18,5 |
| (24b) | MMA | Gew.% | 10,5 | 4,5 | 10,0 |
| (24b) | Aceton | Gew.% | 11,0 | 6,4 | 9,6 |
| (24b) | MAN | Gew.% | 1,9 | 0,8 | 1,7 |
| (24b) | MAN | kg/h | 25,7 | 8 | 22,5 |
| (26a) | Gesamt | kg/h | nv | nv | 266 |
| (26a) | MMA | kg/h | nv | nv | 26,8 |
| (26a) | Aceton | kg/h | nv | nv | 25,5 |
| (26a) | MAN | kg/h | nv | nv | 2,75 |
| (26b) | Gesamt | kg/h | 271 | 522 | nv |
| (26c) | Gesamt | kg/h | 1.084 | 522 | 1063 |
| (28a) | Gesamt | kg/h | 72 | 66 | nv |
| (28a) | Aceton | Gew.% | 19 | 14,5 | nv |
| (28a) | MAN | Gew.% | 3,2 | 2,0 | nv |
| (28a) | MAN | kg/h | 2,3 | 1,3 | nv |
| (28b) | Gesamt | kg/h | 252 | 508 | nv |
| (28b) | Wasser | Gew.% | 63,0 | 76,0 | nv |
| (28b) | MeOH | Gew.% | 19,6 | 14,0 | nv |
| (28b) | MMA | Gew.% | 6,7 | 2,3 | nv |
| (28b) | Aceton | Gew.% | 9,3 | 4,0 | nv |
| (28b) | MAN | Gew.% | 1,1 | 0,5 | nv |
| (28b) | Aceton | kg/h | 23 | 20 | nv |
| (28b) | MAN | kg/h | 2,65 | 2,7 | nv |
| (25a) | Gesamt | kg/h | 5453 | 5534 | 5440 |
| (25a) | MMA | Gew.% | >99,9 | >99,9 | >99,9 |
| (25a) | Aceton | ppm | 10 | <10 | 10 |
| (25a) | MAN | ppm | 176 | 47 | 118 |
| (25a) | Aceton | kg/h | 0,2 | <0,1 | 0,15 |
| (25a) | MAN | kg/h | 0,96 | 0,26 | 0,65 |

| | | | | | |
|---|---|---|---|---|---|
| *Vergleichsbeispiel, nv = nicht vorhanden | | | | | |

Die Ergebnisse in den Tabellen 1 bis 3 zeigen, dass im erfindungsgemäßen Beispiel A2 unter Verwendung von Schwefelsäure mit einer Konzentration von 99,7 Gew.-% (0,3 Gew.% Wasser) niedrigere Anteile an MAN und Aceton in der Amidierung und Konvertierung erhalten (siehe Stoff-Strom (8) in Tabelle 1) und in die Veresterung zugeführt werden als im Vergleich zu Beispielen A1 und A3 unter Verwendung von Schwefelsäure mit einer Konzentration von 100,3 Gew.-% (0,3 Gew.-% freies SO₃).

Zusätzlich zur verringerten Schwefelsäurekonzentration wird im erfindungsgemäßen Beispiel A2 eine erhöhte Menge an wässriger Phase aus Phasentrenner (N) ausgeschleust (Verhältnis 26c / 26b = 50 : 50 vs. 80 : 20 im Vergleichsbeispiel A1).

Zudem bewirkt die erfindungsgemäße Fahrweise nach Beispiel A2, dass das MMA-Reinprodukt deutlich geringer mit MAN verunreinigt ist, als es im Vergleichsbeispiel nach dem Stand der Technik der Fall ist. Der Anteil an MAN und Aceton im MMA-Endprodukt (25a) in Beispiel A2 ist deutlich verringert im Vergleich zu Beispiel A1 (siehe Tabelle 3).

In der folgenden Tabelle 4 sind die Stoffströme aus Tabelle 3 betreffend die Anreicherung von MAN in den Schritten (L), (M) und (N) sowie betreffend die Rückführung von MAN und die Ausschleusung von MAN über (M) und (P) für die Beispiele A1 und A3 (Vergleichsbeispiele) und A2 (erfindungsgemäß) zusammengestellt.

**Tabelle 4: Übersicht ausgewählter Prozessdaten sowie Übersicht zur MAN-Rückführung und MAN-Ausschleusung in (L), (M) (N) und (P)**

| Beispiel | | | A1* | A2 | A3* |
|---|---|---|---|---|---|
| Schwefelsäure-Konz. (2) | | Gew% | 100,3 | 99,7 | 100,3 |
| Verhältnis (26c) / (26b) | | kg/kg | 80/20 | 50/50 | nv |
| Verhältnis (26c) / (26a) | | kg/kg | nv | nv | 80/20 |
| Wässrige Phase zur Veresterung (Recycle) (26c) | GesamtMenge | kg/h | 1.084 | 522 | 1.063 |
| Wässrige Phase zur Veresterung (Recycle) (26c) | MAN-Menge | kg/h | 20,6 | 4,0 | 16,4 |
| Gesamte Rückführmenge in die Veresterung (26c) + (28a) bzw. nur (26c) | GesamtMenge | kg/h | 1.156 | 574 | 1.063 |
| Gesamte Rückführmenge in die Veresterung (26c) + (28a) | MAN-Menge | kg/h | 23,0 | 5,3 | 19,8 |
| Raffinat zur Spaltsäure (Ausschleusung nach Extraktion) (28b) | GesamtMenge | kg/h | 252 | 508 | nv |
| Ausschleusung wässrige Phase nach Phasentrenner II (26a) | Gesamt | kg/h | nv | nv | 266 |
| MAN-Ausschleusung in (28b) bzw. (26a) | MAN-Menge | kg/h | 2,65 | 2,70 | 2,75 |
| Quotient (MAN Rückführung / MAN Ausschleusung) | | kg/kg | 8,7 | 2,0 | 7,3 |
| MMA-Verlust in Ausschleusung (28b) bzw. (26a) | MMA-Menge | kg/h | 16,9 | 11,6 | 26,8 |
| Verlust MMA-Gesamtausbeute durch ausgeschleustes MMA | | % | 0,31 | 0,20 | 0,5 |
| MMA-Gesamtausbeute | MMA bez. auf ACH | % | 90,3 | 91,6 | 90,1 |

| | | | | | |
|---|---|---|---|---|---|
| *Vergleichsbeispiel | | | | | |

Anhand der Werte in Tabelle 4 zeigt sich, dass im Vergleichsbeispiel A1 eine größere Menge an MAN als störendes Nebenprodukt in den Prozess (Veresterungsreaktor E) zurückgeführt wird (über (28a)) und weniger störendes MAN aus dem Verfahren ausgeschleust werden kann (über (28b)) im Vergleich zum erfindungsgemäßen Beispiel A2. Der Quotient aus MAN (zurückgeführt) zu MAN (ausgeschleust) ist somit im erfindungsgemäßen Beispiel A2 deutlich geringer und somit vorteilhafter als im Vergleichsbeispiel A1. Der Verlust an MMA durch die Ausschleusung von wässriger Phase (28b) ist in den Vergleichsbeispielen A1 und A3 im Vergleich zum erfindungsgemäßen Beispiel A2 deutlich erhöht, wobei der MMA-Verlust durch Einsatz der Extraktion in Vergleichsbeispiel A1 geringer ausfällt als in Vergleichsbeispiel A3. Aufgrund der geringeren Anreicherung von Aceton und MAN im erfindungsgemäßen Beispiel A2 ist der MMA-Verlust am geringsten, obwohl die ausgeschleuste Menge an wässriger Phase größer ist als in den Vergleichsbeispielen A1 und A3.

Zudem wird experimentell gezeigt, dass die ausgeschleuste und optional einer weiteren Verwertung zugeführte Spaltsäure (11) weniger mit sulfoniertem Aceton belastet ist (siehe Tabelle 1).

### Beispiel B1 gemäß Figur 3 (Variante B)

Die Herstellung von Methylmethacrylat, umfassend die Umsetzung von Acetoncyanhydrin mit Schwefelsäure in der Amidierung / Konvertierung (A, B, C, D), der anschließenden Veresterung (E) mit Methanol sowie der destillativen und extraktiven Aufarbeitung (F, I ,J, K ,L ,O ,M) des Methylmethacrylat-Produkts erfolgte anhand der Ausführungsform nach Figur 3 wie oben beschrieben (Variante B).

Es erfolgte eine Bilanzierung und Betrachtung der Ausschleusung an MAN und Aceton über das Gesamt-Abgas (11), die Abluft-Ströme (21a) und (9b), und über das MMA-Produkt (25a) (siehe Fließschema nach Figur 3).

Im Folgenden wird ein erfindungsgemäßes Beispiel (Beispiele B1) unter Verwendung von Schwefelsäure mit einer Konzentration im Bereich von 99,7 Gew.-% (0,3 Gew.-% Wasser) beschrieben. Das Beispiel beschreibt die Herstellung von Methylmethacrylat mit den beanspruchten Merkmalen unter Erzielung einer hohen Gesamtausbeute an MMA, gekennzeichnet durch eine verminderte MAN-Bildung in der Amidierung und Konvertierung. Durch die ausschließlich destillative Aufarbeitung des MMA-Produkts (ohne Extraktion) und Ausschleusung von Nebenprodukten wie Aceton im Abgas gelingt es, analog zu Beispiel 2A, eine hohe MMA-Gesamtausbeute mit moderatem MAN-Gehalt zu realisieren.

Die Durchführung des erfindungsgemäßen Verfahrens nach Figur 3 (Variante B) ist im Folgenden beschrieben.

### 2a. Reaktionsstufen (A), (B), (C), (D) und (E)

Die Durchführung der Amidierung (A) und (B), der Konvertierung (C) und Gasabscheidung (D) erfolgte wie oben in Beispiel 1 beschrieben mit den im Folgenden genannten Unterschieden:
Der Gasraum der Amidierungsreaktoren (A) und (B) wurden bei ca. 970 mbara im leichten Unterdruck betrieben.
Der Zwischenbehälter (D) wurde bei einer Temperatur von 100 °C betrieben. Die Kühlung des Gemischs (7) erfolgte im Bereich des Behälters (D) mittels eines wassergekühlten Wärmetauschers, welcher von einem Amid-Kreislaufstrom, der an Behälter (D) angeschlossen war, durchströmt wurde.
Durch geeignete Verschaltung der Eduktströme (10a, 10b, 16b, 16d, 29) und der Kreislaufströme (13, 20c, 21b, 25b) wurde erreicht, dass in jedem der sechs Veresterungskessel ein lokaler, stöchiometrischer Überschuss von Methanol und Wasser, bezogen auf Methacrylsäureamid und Methacrylsäure, vorlag.
Im Gasabscheider (D) wird ein Gesamtabgas (9b) erhalten, welches Aceton und Methacrylsäureamid enthielt und welches kontinuierlich aus dem Prozess entfernt wurde.

Die Mengen und Zusammensetzungen sind in Tabelle 5 zusammengefasst.

### 2b. Vorreinigung (F), (I), (J)

Gemäß Dampf- / Flüssig-Gleichgewicht der Reaktionsmischungen verließ der Brüden-Strom (12) die Veresterung mit einer heteroazeotropen Zusammensetzung enthaltend MMA, Wasser, MeOH, MAS, Aceton und MAN wie in Tabelle 6 angegeben.

Brüdenstrom (12) wurde anschließend einer Gegenstrom-Destillation (F) unterzogen, wobei der dampfförmige Strom (12) im Sumpfbereich einer Kolonne (F) aufgegeben wurde. Die Destillation (F) wurde bei leichtem Überdruck von 100 mbar(g) betrieben.

Am Kopf der Kolonne wurde Brüdenstrom (12) partiell kondensiert und ein organischer, flüssiger Seitenstrom (19c) der Kolonne (I) als unterkühlter Rücklauf aufgegeben. Durch dieses Vorgehen wurde ein Methacrylsäure-haltiger Sumpfstrom (13) erhalten, welcher direkt in die Veresterung (E) zurückgeführt wird. Der Sumpfstrom enthält MMA, MAS und weitere Schwersieder gemäß Tabelle 6. Am Kopf der Kolonne (F) wurde ein dampfförmiger, heteroazeotroper Brüdenstrom (14b) erhalten, welcher zur weiteren destillativen Auftrennung in der Mitte einer Rektifikationskolonne (I) zugeführt wurde. Der heteroazeotrope Strom (14b) enthielt MMA, MeOH, Wasser, Aceton und MAN gemäß Tabelle 6.

Die Roh-Stripperkolonne (I) wurde mit Heizdampf beheizt und bei leichtem Überdruck von 100 mbarg betrieben. Es wurde ein von Leichtsiedern abgereichertes, heteroazeotropes Gemisch aus Methylmethacrylat, Wasser, Methanol, Aceton und MAN im Sumpfstrom (19b) von einem Leichtsieder-angereicherten Kopfstrom (19a) abgetrennt. Der Leichtsieder-Gehalt und damit unter anderem auch der Gehalt an Aceton und MAN konnte über den Energieeintrag des Verdampfers gesteuert werden.

Am Kopf der Kolonne (I) wurde der erhaltene Brüden-Strom partiell kondensiert, das Kondensat als flüssiger Rücklauf zurück auf Kolonne (I) gegeben und die verbleibende, leichtsiederhaltige Dampfphase (19a), welche MeOH, MMA, Wasser Aceton und MAN sowie weitere Leichtsieder gemäß Tabelle 6 enthielt, einer Abgaswäsche (J) zugeführt. Hierbei wurde die Anreicherung an Leichtsiedern im Kopfbereich der Kolonne (I) durch die Temperatur im Partialkondensator gesteuert.

Ein Teil des flüssigen Leichtsieder-Gemischs am Kopf von Kolonne (I) wurde kontinuierlich aus der Kolonne (I) in Form eines flüssigen Seitenstrom-Abzugs (19c) entfernt. Seitenstrom-Abzug (19c) enthielt MMA, MeOH, Wasser, Aceton und MAN gemäß Tabelle 6 und diente, unterkühlt, als Rücklauf für Waschkolonne (F).

Der Brüdenstrom (19a) wurde anschließend zusammen mit Abgas (23b) aus der Azeotrop-Kolonne (L) auf den Sumpfbereich einer Abgaswaschkolonne (J) zugeführt, welche am Kopf einen Partialkondensator aufwies. Die zugeführten Brüden (19a) stiegen in Kolonne (J) auf und wurden durch den Schwersieder-haltigen Rücklauf, welcher durch Kondensation am Kopf entsteht, gewaschen, so dass ein MMA-abgereichertes Abgas (21a) am Kopf von Kolonne (J) sowie ein flüssiges, MMA-angereichertes Gemisch (21b) im Sumpf von Kolonne (J) erhalten wurden. Der Waschprozess in (J) wurde durch Zugabe von frischem Methanol (10b), welches am Kopf von Kolonne (J) aufgegeben wurde, unterstützt. Der Sumpfstrom (21b) aus (J), enthaltend MeOH und MMA, wurde der Veresterung (E) zugeführt. Das Abgas (21a) enthielt Leichtsieder und Inerte, MeOH, Aceton und MAN und wurde einer Verbrennung zugeführt. Mengen und Zusammensetzungen sind in Tabelle 6 angegeben.

Der am Roh-Stripper (I) erhaltene, heteroazeotrope Sumpfstrom (19b) wurde zur Separation der Flüssigphasen zunächst auf 30 °C gekühlt und anschließend dem Phasentrennbehälter (K) zugeführt. Zusätzlich zu Stoffstrom (19b) wird in Phasentrenner (K) ein MMA-haltiges, heteroazeotropes Destillat (23c) der Kondensations- / Vakuum-Einheit (M) zugeführt. Die wässrige, schwere Phase (20b) enthielt Wasser, Methanol, MMA, Aceton, MAS sowie Schwer- und Leichtsieder gemäß Tabelle 6 und wurde kontinuierlich einer Mischstrecke zugeführt. Hier wurde durch die Zugabe von deionisiertem Wasser (16b) ein verdünnter Strom (20c) erhalten, welcher vollständig der Veresterung (E) zugeführt wurde. Die organische, leichte Phase (20a) enthielt MMA, Wasser, Methanol, Aceton, MAS, MAN und Schwer- und Leichtsieder gemäß Tabelle 6 und wurde zur Feinreinigung der Azeotrop-Kolonne (L) zugeführt.

### 2c. Feinreinigung (L), (O), (M)

Die organische, leichte Phase (20a) wurde zur Entfernung von Leichtsiedern dem Kopfbereich einer im Vakuum betriebenen Azeotrop-Kolonne (L) zugeführt, welche mit Heizdampf indirekt beheizt wurde und einen Leichtsieder-angereicherten, heteroazeotropen Brüdenstrom (22a) vom einem Methylmethacrylat-angereicherten Sumpfstrom (22b) separiert.

Der Brüdenstrom (22a) enthielt MMA, Wasser, MeOH, Aceton und MAN gemäß Tabelle 7. Der Sumpfstrom (22b), welcher in gewünschter Weise von Leichtsiedern befreit wurde, enthielt MMA, MAS, Schwersieder und die Nebenprodukte MAN und Aceton gemäß Tabelle 7.

Das in der Azeotrop-Kolonne (L) erhaltene Sumpfprodukt (22b) wurde zur weiteren Aufreinigung einer Unterdruck-Rektifikation (O) unterzogen, welche bei 180 mbar(a) durchgeführt wurde und durch einen Verstärkungs- sowie einen Abtriebsteil gekennzeichnet war. Stoffstrom (22b) wurde mittig auf die Rein-Kolonne (O) aufgegeben, wobei entsprechend des sich einstellenden Gleichgewichtes ein Rein-MMA-Produkt (25a) und ein Schwersieder-angereicherter Sumpfstrom (25b) erhalten wurden. Der erhaltene Brüdenstrom wurde vollständig kondensiert, das erhaltene Abgas von 4 m³/h wurde vollständig dem Verfahrensschritt (M) zugeführt, und das Destillat wurde entsprechend des benötigten Rücklaufverhältnisses aufgeteilt, so dass, bezogen auf die Veresterungsstraße (E), die in Tabelle 7 angegebene Menge MMA mit > 99,9 % Reinheit erhalten wurde. Der Anteil an Aceton und MAN sowie die ausgeschleusten Mengen sind in Tabelle 7 angeführt.

Der erhaltene Sumpfstrom (25b), welcher MMA, MAS und Schwersieder gemäß Tabelle 7 enthält, wurde kontinuierlich in den Reaktionsschritt (E) zurückgefahren.

### 2d. Verfahrensbedingungen und Ergebnisse

**Tabelle 5: Beispiel B1 (erfindungsgemäß) - Daten für Amidierung, Konvertierung und Veresterung ((A), (B), (C), (D), (E))**

| | | | B1 |
|---|---|---|---|
| (1a) | ACH | kg/h | 6.845 |
| (1b) | ACH | kg/h | 3.685 |
| (1a) + (1b) | ACH ges | kg/h | 10.530 |
| (1a) : (1b) | | kg/kg | 65:35 |
| (1a) + (1b) | ACH Konz. | Gew% | 99,0 |
| (2) | H₂SO₄ Konz. | Gew.% | 99,7 |
| (2) | Gesamt | kg/h | 17.060 |
| (A) | H₂SO₄/ ACH | kg/kg | 2,55 |
| (A)+(B) | H₂SO₄ / ACH | kg/kg | 1,62 |
| (5b) | Gesamt | m³/h | 85 |
| (9b) | Gesamt | m³/h | 105 |
| (9b) | Aceton | g/m³ | 15 |
| (9b) | MAN | g/m³ | 1 |
| (9b) | Aceton | kg/h | 2 |
| (9b) | MAN | kg/h | 0,1 |
| (8) | Gesamt | kg/h | 27.475 |
| (8) | MASA | Gew.% | 35,7 |
| (8) | MAS | Gew.% | 0,9 |
| (8) | HIBA | Gew.% | 0,2 |
| (8) | Aceton | Gew.% | 0,2 |
| (8) | MAN | ppm | 322 |
| (8) | Aceton | kg/h | 53 |
| (8) | MAN | kg/h | 8,6 |
| (8) | MASA + MAS (Edukte f. Veresterung) | kg/h | 10.080 |
| Amid-Ausbeute | MASA + MAS bez. Auf ACH | % | 95,7 |
| (29) | Gesamt (MMA/MeOH) | kg/h | 700 |
| (10a) | Gesamt (MeOH) | kg/h | 3.300 |
| (16d) | Gesamt (Direktdampf) | kg/h | 550 |
| (16b) | Wasser | kg/h | 3450 |
| MAN (hydrol.) | Verminderung MAN in (E) | kg/h | 4,8 |
| (11) | Gesamt | kg/h | 23570 |
| (11) | TOC | Gew.% | 3,2 |
| (11) | Feststoff TOC | Gew.% | 0,2 |
| TOC/(11) | Sulfo Aceton | ppm | 265 |
| TOC/(11) | MAN | ppm | 120 |
| (11) | Aceton | kg/h | 7 |
| (11) | MAN | kg/h | 2,9 |

**Tabelle 6: Beispiel B1 (erfindungsgemäß) - Daten für Aufarbeitung / Vorreinigung (F), (I), (J))**

| | | | B1 |
|---|---|---|---|
| (12) | Gesamt | kg/h | 32304 |
| (12) | MMA | Gew.% | 87,9 |
| (12) | Wasser | Gew.% | 5,3 |
| (12) | MeOH | Gew.% | 3,6 |
| (12) | MAS | Gew.% | 1,3 |
| (12) | Aceton | Gew.% | 0,6 |
| (12) | MAN | ppm | 52 |
| (14b) | Gesamt | kg/h | 23445 |
| (14b) | MMA | Gew.% | 73,9 |
| (14b) | Wasser | Gew.% | 10,3 |
| (14b) | MeOH | Gew.% | 13,8 |
| (14b) | Aceton | Gew.% | 1,5 |
| (14b) | MAN | ppm | 105 |
| (19a) | Gesamt | kg/h | 1.684 |
| (19a) | MeOH | Gew.% | > 60,0 |
| (19a) | MMA | Gew.% | 25,0 |
| (19a) | Wasser | Gew.-% | 2,0 |
| (19a) | Aceton | Gew.% | 9,3 |
| (19a) | MAN | ppm | 179 |
| (19c) | Gesamt | kg/h | - |
| (19c) | MMA | Gew.% | 60 |
| (19c) | Wasser | Gew.% | 10 |
| (19c) | MeOH | Gew.% | 28 |
| (19c) | Aceton | Gew.% | 2 |
| (19c) | MAN | ppm | 200 |
| (21a) | Gesamt | kg/h | 220 |
| (21a) | Leichtsieder/ Inerte | Gew.% | >60 |
| (21a) | MeOH | Gew.% | 20 |
| (21a) | Aceton | Gew.% | 18 |
| (21a) | MAN | ppm | 330 |
| (21a) | Aceton | kg/h | 37 |
| (21a) | MAN | kg/h | 0,1 |
| (20b) | Gesamt | kg/h | 1.833 |
| (20b) | Wasser | Gew.% | 88,0 |
| (20b) | MeOH | Gew.% | 8,1 |
| (20b) | MMA | Gew.% | 2,9 |
| (20b) | MAS | Gew.% | 0,02 |
| (20b) | Aceton | Gew.% | 0,03 |
| (20b) | MAN | ppm | 15 |
| (20a) | Gesamt | kg/h | 17.998 |
| (20a) | MMA | Gew.% | 95,7 |
| (20a) | Wasser | Gew.% | 2,2 |
| (20a) | MeOH | Gew.% | 1,6 |
| (20a) | MAS | Gew.% | 0,1 |
| (20a) | Aceton | ppm | 250 |
| (20a) | MAN | ppm | 127 |

**Tabelle 7: Beispiel B1 (erfindungsgemäß) - Daten für Aufarbeitung / Feinreinigung (L), (O), (M)**

| | | | B1 |
|---|---|---|---|
| (22a) | Gesamt | kg/h | 5.814 |
| (22a) | MMA | Gew.% | 88,6 |
| (22a) | Wasser | Gew.% | 6,8 |
| (22a) | MeOH | Gew.% | 5,0 |
| (22a) | Aceton | Gew.% | 0,08 |
| (22a) | MAN | ppm | 280 |
| (22b) | Gesamt | kg/h | - |
| (22b) | MMA | Gew.% | >99,0 |
| (22b) | MAS | Gew.% | 0,2 |
| (22b) | Schwersieder | Gew.% | 0,3 |
| (22b) | Aceton | ppm | < 10 |
| (22b) | MAN | ppm | 64 |
| (23c) | Gesamt | kg/h | ~5.780 |
| (23b) | Gesamt Abgas | kg/h | - |
| (25a) | Gesamt MMA Rein | kg/h | 11.390 |
| (25a) | MMA | Gew.% | > 99,9 |
| (25a) | Aceton | ppm | < 10 |
| (25a) | MAN | ppm | 70 |
| (25a) | Aceton | kg/h | < 0,1 |
| (25a) | MAN | kg/h | 0,8 |
| MMA-Ausbeute | MMA bez. auf ACH | % | 91,1 |
| (25b) | Sumpf (O) | kg/h | 800 |
| (25b) | MMA | Gew.% | 87,0 |
| (25b) | MAS | Gew.% | 4,0 |
| (25b) | Schwersieder | Gew.% | 9,0 |

Die Ergebnisse in den Tabellen 4 bis 6 zeigen, dass im erfindungsgemäßen Beispiel B1 unter Verwendung von Schwefelsäure mit einer Konzentration von 99,7 Gew.-% (0,3 Gew.% Wasser) ein niedriger Anteil an MAN und Aceton in der Amidierung und Konvertierung erhalten und in die Veresterung zugeführt werden. Im Zusammenspiel mit einer ausschließlich destillativen Aufreinigung des Roh-Produkts sowie der gasförmigen Ausschleusung von Nebenkomponenten, bewirkt diese Fahrweise, dass das MMA-Reinprodukt deutlich geringer mit MAN verunreinigt ist.

## Patentansprüche

1. Verfahren zur Herstellung von Alkylmethacrylat, bevorzugt Methylmethacrylat, umfassend
a. die Umsetzung von Acetoncyanhydrin und Schwefelsäure in ein oder mehreren Reaktoren I in einer ersten Reaktionsstufe (Amidierung) bei einer Temperatur im Bereich von 70 bis 130 °C, wobei eine erste Reaktionsmischung, enthaltend Sulfoxyisobuttersäureamid und Methacrylsäureamid, erhalten wird;
b. das Konvertieren der ersten Reaktionsmischung, umfassend das Erhitzen auf eine Temperatur im Bereich von 130 bis 200 °C in ein oder mehreren Reaktoren II in einer zweiten Reaktionsstufe (Konvertierung), wobei eine zweite Reaktionsmischung, enthaltend überwiegend Methacrylsäureamid und Schwefelsäure, erhalten wird;
c. die Umsetzung der zweiten Reaktionsmischung mit Alkohol und Wasser, bevorzugt Methanol und Wasser, in ein oder mehreren Reaktoren III in einer dritten Reaktionsstufe (Veresterung), wobei eine dritte Reaktionsmischung, enthaltend Alkylmethacrylat, erhalten wird;
d. und die Abtrennung von Alkylmethacrylat aus der dritten Reaktionsmischung erhalten aus der dritten Reaktionsstufe;
wobei die in der ersten Reaktionsstufe eingesetzte Schwefelsäure eine Konzentration im Bereich von 98,0 Gew.-% bis 100,0 Gew.-% aufweist;
wobei die Abtrennung von Alkylmethacrylat aus der dritten Reaktionsmischung mindestens zwei Destillationsschritte umfasst, bei denen die Nebenprodukte Methacrylnitril und Aceton zumindest teilweise in der Kopf-Fraktion als wasserhaltiges Heteroazeotrop erhalten werden,
wobei das wasserhaltige Heteroazeotrop, enthaltend Methacrylnitril und Aceton, aus mindestens einem dieser Destillationsschritte zumindest teilweise aus dem Verfahren ausgeschleust wird,
und wobei mindestens ein Strom, enthaltend Methacrylnitril und Aceton, zumindest teilweise in die dritte Reaktionsstufe zurückgeführt wird.

2. Verfahren nach Anspruch 1, **dadurch gekennzeichnet, dass** es sich bei dem mindestens einen Strom, enthaltend Methacrylnitril und Aceton, welcher zumindest teilweise in die dritte Reaktionsstufe zurückgeführt wird, um wasserhaltiges Heteroazeotrop, enthaltend Methacrylnitril und Aceton, aus mindestens einem der Destillationsschritte handelt.

3. Verfahren nach Anspruch 1 oder 2, **dadurch gekennzeichnet, dass** mindestens eine wässrige Phase, welche mittels Kondensation und Phasentrennung des wasserhaltigen Heteroazeotrops aus mindestens einem der Destillationsschritte erhalten wird, vollständig oder teilweise in die dritte Reaktionsstufe zurückgeführt wird und dort mit der zweite Reaktionsmischung, enthaltend überwiegend Methacrylsäureamid und Schwefelsäure, in Kontakt gebracht wird.

4. Verfahren nach einem der Ansprüche 1 bis 3, **dadurch gekennzeichnet, dass** mindestens eine wässrige Phase, welche mittels Kondensation und Phasentrennung des wasserhaltigen Heteroazeotrops aus mindestens einem der Destillationsschritte erhalten wird, vollständig oder teilweise, optional nach einem Extraktionsschritt, aus dem Verfahren ausgeschleust wird.

5. Verfahren nach einem der Ansprüche 1 bis 4, **dadurch gekennzeichnet, dass** das wasserhaltigen Heteroazeotrops aus mindestens einem der Destillationsschritte zumindest teilweise, optional nach einem Waschschritt, in Form eines gasförmigen Stroms vollständig oder teilweise aus dem Verfahren ausgeschleust wird.

6. Verfahren nach einem der Ansprüche 1 bis 5, **dadurch gekennzeichnet, dass** die Abtrennung von Alkylmethacrylat aus der dritten Reaktionsmischung mindestens einen Phasentrennungsschritt umfasst, bei dem das wasserhaltige Heteroazeotrop aus mindestens einem der Destillationsschritte in eine wässrige Phase, enthaltend Methacrylnitril und Aceton, und eine organische Phase, enthaltend überwiegend Alkylmethacrylat, getrennt wird, wobei die wässrige Phase teilweise aus dem Verfahren ausgeschleust wird und / oder teilweise in die dritte Reaktionsstufe zurückgeführt wird, und wobei die organische Phase, enthaltend überwiegend Alkylmethacrylat, vollständig oder teilweise in den mindestens einen Destillationsschritt zurückgeführt wird.

7. Verfahren nach einem der Ansprüche 1 bis 6, **dadurch gekennzeichnet, dass** die dritte Reaktionsmischung erhalten in der dritten Reaktionsstufe kontinuierlich verdampft wird, wobei der hierbei entstehenden Brüdenstrom einem ersten Destillationsschritt K1 zugeführt wird, bei dem eine Kopf-Fraktion, enthaltend Alkylmethacrylat, Wasser und Alkohol, und eine Sumpf-Fraktion enthaltend schwerer siedenden Komponenten, erhalten werden, und wobei die Sumpf-Fraktion vollständig oder teilweise in die dritte Reaktionsstufe zurückgeführt wird.

8. Verfahren nach einem der Ansprüche 1 bis 7, **dadurch gekennzeichnet, dass** die Abtrennung von Alkylmethacrylat aus der dritten Reaktionsmischung umfasst, dass
(i) die dritte Reaktionsmischung erhalten in der dritten Reaktionsstufe zunächst in einem ersten Destillationsschritt K1 destilliert wird, wobei ein erstes wasserhaltiges Heteroazeotrop, enthaltend Methacrylnitril und Aceton, als Kopf-Fraktion erhalten wird;
(ii) das erste wasserhaltige Heteroazeotrop als Kondensat in einem Phasentrennungsschritt (Phasentrenner I) in eine wässrige Phase WP-1 und eine organische Phase OP-1, enthaltend den überwiegenden Teil des Alkylmethacrylats, getrennt wird;
(iii) die organische Phase OP-1 in einen zweiten Destillationsschritt K2 geführt wird, wobei als Kopf-Fraktion ein zweites wasserhaltiges Heteroazeotrop, enthaltend Methacrylnitril und Aceton, erhalten wird;
(iv) zumindest ein Teil des zweiten wasserhaltigen Heteroazeotrops in einem Phasentrennungsschritt (Phasentrenner II) in eine wässrige Phase WP-2, enthaltend Methacrylnitril und Aceton, und eine organische Phase OP-2 getrennt wird,
wobei die organische Phase OP-2 vollständig oder teilweise in den zweiten Destillationsschritt K2 zurückgeführt wird,
und wobei die wässrige Phase WP-2, enthaltend Methacrylnitril und Aceton, teilweise in die dritte Reaktionsstufe zurückgeführt wird und teilweise, optional nach einem Extraktionsschritt, aus dem Verfahren ausgeschleust wird.

9. Verfahren nach Anspruch 8, **dadurch gekennzeichnet, dass** die wässrige Phase WP-1 vollständig oder teilweise in die dritte Reaktionsstufe zurückgeführt wird und die organische Phase OP-1, enthaltend den überwiegenden Teil des Alkylmethacrylats, einer Extraktion unter Verwendung von Wasser als Extraktionsmittel unterzogen wird, wobei die wässrige Phase dieser Extraktion in die dritte Reaktionsstufe zurückgeführt wird und die organische Phase dieser Extraktion in den zweiten Destillationsschritt K2 geführt wird.

10. Verfahren nach Anspruch 8 oder 9, **dadurch gekennzeichnet, dass** ein Teil der wässrige Phase WP-2, enthaltend Methacrylnitril und Aceton, einer Extraktion unterzogen wird, wobei eine wässrige Phase WP-3 und eine organische Phase OP-3 erhalten werden, wobei die wässrige Phase WP-3 vollständig oder teilweise aus dem Verfahren ausgeschleust wird, und wobei die organische Phase OP-3 vollständig oder teilweise in die dritte Reaktionsstufe zurückgeführt wird.

11. Verfahren nach Anspruch 8 oder 9, **dadurch gekennzeichnet, dass** ein Teil der wässrige Phase WP-2, enthaltend Methacrylnitril und Aceton, einer Extraktion unterzogen wird, wobei eine wässrige Phase WP-3 und eine organische Phase OP-3 erhalten werden, wobei die wässrige Phase WP-3 einem weiteren Destillationsschritt K4 unterzogen wird, wobei im Destillationsschritt K4 eine Kopf-Fraktion, enthaltend Methacrylnitril, erhalten wird, welche aus dem Verfahren ausgeschleust wird, und wobei im Destillationsschritt K4 eine Sumpf-Fraktion, enthaltend Wasser, erhalten wird, welche vollständig oder teilweise in die Extraktion zurückgeführt wird, und wobei die organische Phase OP-3 vollständig oder teilweise in die dritte Reaktionsstufe zurückgeführt wird.

12. Verfahren nach einem der Ansprüche 1 bis 7, **dadurch gekennzeichnet, dass** die Abtrennung von Alkylmethacrylat aus der dritten Reaktionsmischung umfasst, dass
(i) die dritte Reaktionsmischung erhalten in der dritten Reaktionsstufe zunächst in einem ersten Destillationsschritt K1 destilliert wird, wobei ein erstes wasserhaltiges Heteroazeotrop, enthaltend Methacrylnitril und Aceton, als Kopf-Fraktion erhalten wird;
(ii) das erste wasserhaltige Heteroazeotrop als Brüdenstrom in einen weiteren Destillationsschritt K4 geführt wird, bei dem ein weiteres wasserhaltiges Heteroazeotrop, enthaltend Methacrylnitril und Aceton, als Kopf-Fraktion und eine Sumpf-Fraktion, enthaltend Alkylmethacrylat, erhalten werden,
(iii) die Kopf-Fraktion aus Destillationsschritt K4, optional nach einem Waschschritt, in Form eines gasförmigen Stroms vollständig oder teilweise aus dem Verfahren ausgeschleust wird;
(iv) die Sumpf-Fraktion aus Destillationsschritt K4 in einem Phasentrennungsschritt (Phasentrenner II) in eine wässrige Phase WP-2, enthaltend Methacrylnitril und Aceton, und eine organische Phase OP-2 getrennt wird, wobei die wässrige Phase WP-2, enthaltend Methacrylnitril und Aceton, vollständig oder teilweise in die dritte Reaktionsstufe zurückgeführt wird,
(v) die organische Phase WP-2 vollständig oder teilweise in einen zweiten Destillationsschritt K2 geführt wird, bei dem als Kopf-Fraktion ein zweites wasserhaltiges Heteroazeotrop, enthaltend Methacrylnitril und Aceton, erhalten wird, welches vollständig oder teilweise kondensiert wird und in den Phasentrennungsschritt (Phasentrenner II) nach (iv) geführt wird.

13. Verfahren nach einem der Ansprüche 1 bis 12, **dadurch gekennzeichnet, dass** die zweite Reaktionsmischung kleiner oder gleich 3 Gew.-% Methacrylsäure, kleiner oder gleich 1,5 Gew.-% alpha-Hydroxyisobuttersäureamid und kleiner oder gleich 0,3 Gew.% Methacrylnitril, jeweils bezogen auf die gesamte zweite Reaktionsmischung, enthält.

14. Verfahren nach einem der Ansprüche 1 bis 13, **dadurch gekennzeichnet, dass** die zweite Reaktionsmischung 30 bis 40 Gew.-% Methacrylsäureamid, bezogen auf die gesamte zweite Reaktionsmischung, enthält.

15. Verfahren nach einem der Ansprüche 1 bis 14, **dadurch gekennzeichnet, dass** in der ersten Reaktionsstufe die Umsetzung von Acetoncyanhydrin und Schwefelsäure in mindestens zwei getrennten Reaktionszonen oder in mindestens zwei voneinander getrennten Reaktoren erfolgt.

16. Verfahren nach einem der Ansprüche 1 bis 15, **dadurch gekennzeichnet, dass** in der ersten Reaktionsstufe die Umsetzung von Acetoncyanhydrin und Schwefelsäure in mindestens zwei voneinander getrennten Reaktoren erfolgt, wobei Schwefelsäure und Acetoncyanhydrin im ersten Reaktor in einem molaren Verhältnis von Schwefelsäure zu ACH im Bereich von 1,6 bis 3,0 eingesetzt werden, und wobei Schwefelsäure und Acetoncyanhydrin im zweiten Reaktor in einem molaren Verhältnis von Schwefelsäure zu ACH im Bereich von 1,2 bis 2,0 eingesetzt werden.

17. Verfahren nach einem der Ansprüche 1 bis 16, **dadurch gekennzeichnet, dass** die Reaktionsmischung aus Acetoncyanhydrin (ACH) und Schwefelsäure in der ersten Reaktionsmischung eine Gesamtwassermenge im Bereich von 0,1 mol-% bis 20 mol%, bezogen das gesamte der ersten Reaktionsstufe zugeführte ACH, aufweist.

18. Verfahren nach einem der Ansprüche 8 bis 17, **dadurch gekennzeichnet, dass** im zweiten Destillationsschritt K2 als Sumpf-Fraktion ein Alkylmethacrylat-Rohprodukt erhalten wird, welches bevorzugt mindestens 99 Gew.-% Alkylmethacrylat enthält, wobei das Alkylmethacrylat-Rohprodukt in einem weiteren Destillationsschritt K3 gereinigt wird, wobei als Kopf-Fraktion ein Alkylmethacrylat-Reinprodukt erhalten wird, welches einen Gehalt an Methacrylnitril im Bereich von 10 bis 300 ppm, bezogen auf das Alkylmethacrylat-Reinprodukt, aufweist.

19. Verfahren nach einem der Ansprüche 1 bis 18, **dadurch gekennzeichnet, dass** das Verfahren eine Regenerierung von Schwefelsäure umfasst, wobei ein Teil der dritten Reaktionsmischung erhalten in der dritten Reaktionsstufe und mindestens ein wässriger oder organischer Abfallstrom, enthaltend Schwefelsäure, Ammoniumhydrogensulfat und sulfonierte Aceton-Derivate, welcher aus der Ausschleusung des wasserhaltigen Heteroazeotrops, enthaltend Methacrylnitril und Aceton, resultiert, einem thermischen Regenerierungsschritt zugeführt werden, bei dem Schwefelsäure erhalten wird, welche in die erste Reaktionsstufe zurückgeführt wird.

20. Verfahren nach einem der Ansprüche 1 bis 18, **dadurch gekennzeichnet, dass** das Verfahren eine Gewinnung von Ammoniumsulfat umfasst, wobei ein Teil der dritten Reaktionsmischung erhalten in der dritten Reaktionsstufe und mindestens ein wässriger oder organischer Abfallstrom, enthaltend Schwefelsäure, Ammoniumhydrogensulfat und sulfonierte Aceton-Derivate, welcher aus der Ausschleusung des wasserhaltigen Heteroazeotrops, enthaltend Methacrylnitril und Aceton, resultiert, einem thermischen Regenerierungsschritt zugeführt wird, bei dem mittels Kristallisation Ammoniumsulfat erhalten wird, welches als Nebenprodukt abgetrennt wird.

## Claims

1. Process for preparing alkyl methacrylate, preferably methyl methacrylate, comprising
a. the reacting of acetone cyanohydrin and sulfuric acid in one or more reactors I in a first reaction stage (amidation) at a temperature in the range from 70 to 130°C to obtain a first reaction mixture comprising sulfoxyisobutyramide and methacrylamide;
b. the converting of the first reaction mixture, comprising heating to a temperature in the range from 130 to 200°C in one or more reactors II in a second reaction stage (conversion) to obtain a second reaction mixture comprising predominantly methacrylamide and sulfuric acid;
c. the reacting of the second reaction mixture with alcohol and water, preferably methanol and water, in one or more reactors III in a third reaction stage (esterification) to obtain a third reaction mixture comprising alkyl methacrylate;
d. and the separating of alkyl methacrylate from the third reaction mixture obtained from the third reaction stage;
wherein the sulfuric acid used in the first reaction stage has a concentration in the range from 98.0% by weight to 100.0% by weight;
wherein the separation of alkyl methacrylate from the third reaction mixture comprises at least two distillation steps in which the methacrylonitrile and acetone by-products are obtained at least partly in the tops fraction as a water-containing heteroazeotrope,
wherein the water-containing heteroazeotrope comprising methacrylonitrile and acetone from at least one of these distillation steps is at least partly discharged from the process,
and wherein at least one stream comprising methacrylonitrile and acetone is at least partly recycled into the third reaction stage.

2. Process according to Claim 1, **characterized in that** the at least one stream comprising methacrylonitrile and acetone which is at least partly recycled into the third reaction stage is water-containing heteroazeotrope comprising methacrylonitrile and acetone from at least one of the distillation steps.

3. Process according to Claim 1 or 2, **characterized in that** at least one aqueous phase which is obtained by means of condensation and phase separation of the water-containing heteroazeotrope from at least one of the distillation steps is recycled fully or partly into the third reaction stage, where it is contacted with the second reaction mixture comprising predominantly methacrylamide and sulfuric acid.

4. Process according to any of Claims 1 to 3, **characterized in that** at least one aqueous phase which is obtained by means of condensation and phase separation of the water-containing heteroazeotrope from at least one of the distillation steps is discharged fully or partly from the process, optionally after an extraction step.

5. Process according to any of Claims 1 to 4, **characterized in that** the water-containing heteroazeotrope from at least one of the distillation steps is discharged fully or partly from the process, at least partly in the form of a gaseous stream, optionally after a scrubbing step.

6. Process according to any of Claims 1 to 5, **characterized in that** the separation of alkyl methacrylate from the third reaction mixture comprises at least one phase separation step in which the water-containing heteroazeotrope from at least one of the distillation steps is separated into an aqueous phase comprising methacrylonitrile and acetone, and an organic phase comprising predominantly alkyl methacrylate, wherein the aqueous phase is partly discharged from the process and/or is partly recycled into the third reaction stage, and wherein the organic phase comprising predominantly alkyl methacrylate is recycled fully or partly into the at least one distillation step.

7. Process according to any of Claims 1 to 6, **characterized in that** the third reaction mixture obtained in the third reaction stage is evaporated continuously, wherein the resultant vapour stream is fed to a first distillation step K1 in which a tops fraction comprising alkyl methacrylate, water and alcohol, and a bottoms fraction comprising higher-boiling components are obtained, and wherein the bottoms fraction is recycled fully or partly into the third reaction stage.

8. Process according to any of Claims 1 to 7, **characterized in that** the separation of alkyl methacrylate from the third reaction mixture comprises
(i) first distilling the third reaction mixture obtained in the third reaction stage in a first distillation step K1 to obtain a first water-containing heteroazeotrope comprising methacrylonitrile and acetone as tops fraction;
(ii) separating the first water-containing heteroazeotrope as condensate in a phase separation step (phase separator I) into an aqueous phase WP-1 and an organic phase OP-1 comprising the predominant portion of the alkyl methacrylate;
(iii) guiding the organic phase OP-1 into a second distillation step K2, wherein the tops fraction obtained is a second water-containing heteroazeotrope comprising methacrylonitrile and acetone;
(iv) separating at least a portion of the second water-containing heteroazeotrope in a phase separation step (phase separator II) into an aqueous phase WP-2 comprising methacrylonitrile and acetone, and an organic phase OP-2,
wherein the organic phase OP-2 is recycled fully or partly into the second distillation step K2,
and wherein the aqueous phase WP-2 comprising methacrylonitrile and acetone is partly recycled into the third reaction stage and is partly discharged from the process, optionally after an extraction step.

9. Process according to Claim 8, **characterized in that** the aqueous phase WP-1 is recycled fully or partly into the third reaction stage and the organic phase OP-1 comprising the predominant portion of the alkyl methacrylate is subjected to an extraction using water as extractant, wherein the aqueous phase of this extraction is recycled into the third reaction stage and the organic phase of this extraction is guided into the second distillation step K2.

10. Process according to Claim 8 or 9, **characterized in that** a portion of the aqueous phase WP-2 comprising methacrylonitrile and acetone is subjected to an extraction to obtain an aqueous phase WP-3 and an organic phase OP-3, wherein the aqueous phase WP-3 is discharged fully or partly from the process, and wherein the organic phase OP-3 is recycled fully or partly into the third reaction stage.

11. Process according to Claim 8 or 9, **characterized in that** a portion of the aqueous phase WP-2 comprising methacrylonitrile and acetone is subjected to an extraction to obtain an aqueous phase WP-3 and an organic phase OP-3, wherein the aqueous phase WP-3 is subjected to a further distillation step K4, wherein a tops fraction comprising methacrylonitrile is obtained in distillation step K4, which is discharged from the process, and wherein a bottoms fraction comprising water is obtained in distillation step K4, which is recycled fully or partly into the extraction, and wherein the organic phase OP-3 is recycled fully or partly into the third reaction stage.

12. Process according to any of Claims 1 to 7, **characterized in that** the separation of alkyl methacrylate from the third reaction mixture comprises
(i) first distilling the third reaction mixture obtained in the third reaction stage in a first distillation step K1 to obtain a first water-containing heteroazeotrope comprising methacrylonitrile and acetone as tops fraction;
(ii) guiding the first water-containing heteroazeotrope as a vapour stream into a further distillation step K4 in which a further water-containing heteroazeotrope comprising methacrylonitrile and acetone is obtained as tops fraction, and a bottoms fraction comprising alkyl methacrylate is obtained,
(iii) discharging the tops fraction from distillation step K4, optionally after a scrubbing step, fully or partly from the process in the form of a gaseous stream,
(iv) separating the bottoms fraction from distillation step K4 in a phase separation step (phase separator II) into an aqueous phase WP-2 comprising methacrylonitrile and acetone, and an organic phase OP-2, wherein the aqueous phase WP-2 comprising methacrylonitrile and acetone is recycled fully or partly into the third reaction stage,
(v) guiding the organic phase WP-2 fully or partly into a second distillation step K2 in which the tops fraction obtained is a second water-containing heteroazeotrope comprising methacrylonitrile and acetone, which is condensed fully or partly and is guided into the phase separation step (phase separator II) according to (iv).

13. Process according to any of Claims 1 to 12, **characterized in that** the second reaction mixture contains not more than 3% by weight of methacrylic acid, not more than 1.5% by weight of alphahydroxyisobutyramide and not more than 0.3% by weight of methacrylonitrile, based in each case on the overall second reaction mixture.

14. Process according to any of Claims 1 to 13, **characterized in that** the second reaction mixture contains 30% to 40% by weight of methacrylamide, based on the overall second reaction mixture.

15. Process according to any of Claims 1 to 14, **characterized in that** the conversion of acetone cyanohydrin and sulfuric acid in the first reaction stage is effected in at least two separate reaction zones or in at least two separate reactors.

16. Process according to any of Claims 1 to 15, **characterized in that**, in the first reaction stage, the conversion of acetone cyanohydrin and sulfuric acid is effected in at least two separate reactors, wherein sulfuric acid and acetone cyanohydrin are used in the first reactor in a molar ratio of sulfuric acid to ACH in the range from 1.6 to 3.0, and wherein sulfuric acid and acetone cyanohydrin are used in the second reactor in a molar ratio of sulfuric acid to ACH in the range from 1.2 to 2.0.

17. Process according to any of Claims 1 to 16, **characterized in that** the reaction mixture of acetone cyanohydrin (ACH) and sulfuric acid in the first reaction mixture includes a total amount of water in the range from 0.1 mol% to 20 mol%, based on the entirety of ACH supplied to the first reaction stage.

18. Process according to any of Claims 8 to 17, **characterized in that** a crude alkyl methacrylate product is obtained as bottoms fraction in the second distillation step K2 and contains preferably at least 99% by weight of alkyl methacrylate, wherein the crude alkyl methacrylate product is purified in a further distillation step K3 to obtain a pure alkyl methacrylate product as tops fraction, having a methacrylonitrile content in the range from 10 to 300 ppm, based on the pure alkyl methacrylate product.

19. Process according to any of Claims 1 to 18, **characterized in that** the process comprises a regeneration of sulfuric acid, wherein a portion of the third reaction mixture obtained in the third reaction stage and at least one aqueous or organic waste stream comprising sulfuric acid, ammonium hydrogensulfate and sulfonated acetone derivatives that results from the discharge of the water-containing heteroazeotrope comprising methacrylonitrile and acetone are sent to a thermal regeneration step in which sulfuric acid is obtained, which is recycled into the first reaction stage.

20. Process according to any of Claims 1 to 18, **characterized in that** the process comprises obtaining ammonium sulfate, wherein a portion of the third reaction mixture obtained in the third reaction stage and at least one aqueous or organic waste stream comprising sulfuric acid, ammonium hydrogensulfate and sulfonated acetone derivatives that results from the discharge of the water-containing heteroazeotrope comprising methacrylonitrile and acetone is sent to a thermal regeneration step in which ammonium sulfate is obtained by means of crystallization, which is separated off as a by-product.

## Revendications

1. Procédé pour la préparation de méthacrylate d'alkyle, de préférence de méthacrylate de méthyle, comprenant :
a. la transformation de cyanhydrine d'acétone et d'acide sulfurique dans un ou plusieurs réacteurs I dans un premier étage de réaction (amidation) à une température dans la plage de 70 à 130°C, un premier mélange réactionnel, contenant de l'amide de l'acide sulfoxyisobutyrique et de l'amide de l'acide méthacrylique, étant obtenu ;
b. la conversion du premier mélange réactionnel, comprenant le chauffage à une température dans la plage de 130 à 200°C dans un ou plusieurs réacteurs II dans un deuxième étage de réaction (conversion), un deuxième mélange réactionnel, contenant principalement de l'amide de l'acide méthacrylique et de l'acide sulfurique, étant obtenu ;
c. la transformation du deuxième mélange réactionnel avec de l'alcool et de l'eau, de préférence du méthanol et de l'eau, dans un ou plusieurs réacteurs III dans un troisième étage de réaction (estérification), un troisième mélange réactionnel, contenant du méthacrylate d'alkyle, étant obtenu ;
d. et la séparation de méthacrylate d'alkyle à partir du troisième mélange réactionnel obtenu à partir du troisième étage de réaction ;
- l'acide sulfurique utilisé dans le premier étage de réaction présentant une concentration dans la plage de 98,0% en poids à 100,0% en poids ;
- la séparation de méthacrylate d'alkyle à partir du troisième mélange réactionnel comprenant au moins deux étapes de distillation, lors desquelles les produits secondaires méthacrylonitrile et acétone sont obtenus au moins partiellement dans la fraction de tête sous forme d'hétéroazéotrope aqueux,
- l'hétéroazéotrope aqueux, contenant du méthacrylonitrile et de l'acétone, provenant d'au moins l'une de ces étapes de distillation étant soutiré au moins partiellement du procédé et
- au moins un flux, contenant du méthacrylonitrile et de l'acétone, étant recyclé au moins partiellement dans le troisième étage de réaction.

2. Procédé selon la revendication 1, **caractérisé en ce qu'**il s'agit, pour ledit au moins un flux, contenant du méthacrylonitrile et de l'acétone, qui est recyclé au moins partiellement dans le troisième étage de réaction, d'hétéroazéotrope aqueux, contenant du méthacrylonitrile et de l'acétone, provenant d'au moins l'une des étapes de distillation.

3. Procédé selon la revendication 1 ou 2, **caractérisé en ce qu'**au moins une phase aqueuse, qui est obtenue par condensation et séparation de phases de l'hétéroazéotrope aqueux provenant d'au moins l'une des étapes de distillation, est recyclée complètement ou partiellement dans le troisième étage de réaction et y est mise en contact avec le deuxième mélange réactionnel, contenant principalement de l'amide de l'acide méthacrylique et de l'acide sulfurique.

4. Procédé selon l'une quelconque des revendications 1 à 3, **caractérisé en ce qu'**au moins une phase aqueuse, qui est obtenue par condensation et séparation de phases de l'hétéroazéotrope aqueux provenant d'au moins l'une des étapes de distillation, est soutirée complètement ou partiellement, éventuellement après une étape d'extraction, du procédé.

5. Procédé selon l'une quelconque des revendications 1 à 4, **caractérisé en ce que** l'hétéroazéotrope aqueux provenant d'au moins l'une des étapes de distillation est soutiré complètement ou partiellement du procédé, au moins partiellement, éventuellement après une étape de lavage, sous forme d'un flux gazeux,.

6. Procédé selon l'une quelconque des revendications 1 à 5, **caractérisé en ce que** la séparation de méthacrylate d'alkyle à partir du troisième mélange réactionnel comprend au moins une étape de séparation de phases, dans laquelle l'hétéroazéotrope aqueux provenant d'au moins l'une des étapes de distillation est séparé en une phase aqueuse, contenant du méthacrylonitrile et de l'acétone, et en une phase organique, contenant principalement du méthacrylate d'alkyle, la phase aqueuse étant soutirée partiellement du procédé et/ou recyclée partiellement dans le troisième étage de réaction et la phase organique, contenant principalement du méthacrylate d'alkyle, étant recyclée complètement ou partiellement dans ladite au moins une étape de distillation.

7. Procédé selon l'une quelconque des revendications 1 à 6, **caractérisé en ce que** le troisième mélange réactionnel obtenu dans le troisième étage de réaction est évaporé en continu, le flux de vapeur ainsi formé étant introduit dans une première étape de distillation K1, lors de laquelle une fraction de tête, contenant du méthacrylate d'alkyle, de l'eau et de l'alcool, et une fraction de fond, contenant des composants à point d'ébullition plus élevé, sont obtenues et la fraction de fond étant recyclée complètement ou partiellement dans le troisième étage de réaction.

8. Procédé selon l'une quelconque des revendications 1 à 7, **caractérisé en ce que** la séparation de méthacrylate d'alkyle à partir du troisième mélange réactionnel comprend les faits selon lesquels
(i) le troisième mélange réactionnel, obtenu dans le troisième étage de réaction, est d'abord distillé dans une première étape de distillation K1, un premier hétéroazéotrope aqueux, contenant du méthacrylonitrile et de l'acétone, étant obtenu comme fraction de tête ;
(ii) le premier hétéroazéotrope aqueux est séparé en tant que condensat dans une étape de séparation de phases (séparateur de phases I) en une phase aqueuse WP-1 et en une phase organique OP-1, contenant la partie principale du méthacrylate d'alkyle ;
(iii) la phase organique OP-1 est introduite dans une deuxième étape de distillation K2, un deuxième hétéroazéotrope aqueux, contenant du méthacrylonitrile et de l'acétone, étant obtenu comme fraction de tête ;
(iv) au moins une partie du deuxième hétéroazéotrope aqueux étant séparée dans une étape de séparation de phases (séparateur de phases II) en une phase aqueuse WP-2, contenant du méthacrylonitrile et de l'acétone, et en une phase organique OP-2,
la phase organique OP-2 étant recyclée complètement ou partiellement dans la deuxième étape de distillation K2 et la phase aqueuse WP-2, contenant du méthacrylonitrile et de l'acétone, étant recyclée partiellement dans le troisième étage de réaction et soutirée partiellement, éventuellement après une étape d'extraction, du procédé.

9. Procédé selon la revendication 8, **caractérisé en ce que** la phase aqueuse WP-1 est recyclée complètement ou partiellement dans le troisième étage de réaction et la phase organique OP-1, contenant la partie principale du méthacrylate d'alkyle, est soumise à une extraction à l'aide d'eau comme agent d'extraction et la phase aqueuse de cette extraction est recyclée dans le troisième étage de réaction et la phase organique de cette extraction est guidée dans la deuxième étape de distillation K2.

10. Procédé selon la revendication 8 ou 9, **caractérisé en ce qu'**une partie de la phase aqueuse WP-2, contenant du méthacrylonitrile et de l'acétone, est soumise à une extraction, une phase aqueuse WP-3 et une phase organique OP-3 étant obtenues, la phase aqueuse WP-3 étant soutirée complètement ou partiellement du procédé et la phase organique OP-3 étant recyclée complètement ou partiellement dans le troisième étage de réaction.

11. Procédé selon la revendication 8 ou 9, **caractérisé en ce qu'**une partie de la phase aqueuse WP-2, contenant du méthacrylonitrile et de l'acétone, est soumise à une extraction, une phase aqueuse WP-3 et une phase organique OP-3 étant obtenues, la phase aqueuse WP-3 étant soumise à une autre étape de distillation K4, une fraction de tête, contenant du méthacrylonitrile, étant obtenue dans l'étape de distillation K4, laquelle fraction de tête est soutirée du procédé et une fraction de fond, contenant de l'eau, étant obtenue dans l'étape de distillation K4, laquelle fraction de fond est recyclée complètement ou partiellement dans l'extraction et la phase organique OP-3 étant recyclée complètement ou partiellement dans le troisième étage de réaction.

12. Procédé selon l'une quelconque des revendications 1 à 7, **caractérisé en ce que** la séparation de méthacrylate d'alkyle à partir du troisième mélange réactionnel comprend les faits selon lesquels
(i) le troisième mélange réactionnel, obtenu dans le troisième étage de réaction, est d'abord distillé dans une première étape de distillation K1, un premier hétéroazéotrope aqueux, contenant du méthacrylonitrile et de l'acétone, étant obtenu comme fraction de tête ;
(ii) le premier hétéroazéotrope aqueux est guidé en tant que flux de vapeur dans une autre étape de distillation K4, lors de laquelle un autre hétéroazéotrope aqueux, contenant du méthacrylonitrile et de l'acétone, est obtenu comme fraction de tête et une fraction de fond, contenant du méthacrylate d'alkyle, est obtenue,
(iii) la fraction de tête provenant de l'étape de distillation K4 est soutirée, éventuellement après une étape de lavage, sous forme d'un flux gazeux, complètement ou partiellement du procédé.
(iv) la fraction de fond provenant de l'étape de distillation K4 est séparée dans une étape de séparation de phases (séparateur de phase II) en une phase aqueuse WP-2, contenant du méthacrylonitrile et de l'acétone, et en une phase organique OP-2, la phase aqueuse WP-2, contenant du méthacrylonitrile et de l'acétone, étant recyclée complètement ou partiellement dans le troisième étage de réaction,
(v) la phase organique WP-2 est introduite complètement ou partiellement dans une deuxième étape de distillation K2, dans laquelle on obtient, comme fraction de tête, un deuxième hétéroazéotrope aqueux, contenant du méthacrylonitrile et de l'acétone, qui est condensé complètement ou partiellement et guidé dans l'étape de séparation de phases (séparateur de phases II) selon (iv).

13. Procédé selon l'une quelconque des revendications 1 à 12, **caractérisé en ce que** le deuxième mélange réactionnel contient 3% en poids ou moins d'acide méthacrylique, 1,5% en poids ou moins d'amide de l'acide alpha-hydroxyisobutyrique et 0,3% en poids ou moins de méthacrylonitrile, à chaque fois par rapport à la totalité du deuxième mélange réactionnel.

14. Procédé selon l'une quelconque des revendications 1 à 13, **caractérisé en ce que** le deuxième mélange réactionnel contient 30 à 40% en poids d'amide de l'acide méthacrylique, par rapport à la totalité du deuxième mélange réactionnel.

15. Procédé selon l'une quelconque des revendications 1 à 14, **caractérisé en ce que**, dans le premier étage de réaction, la transformation de cyanhydrine d'acétone et d'acide sulfurique a lieu dans au moins deux zones de réaction séparées ou dans au moins deux réacteurs séparés l'un de l'autre.

16. Procédé selon l'une quelconque des revendications 1 à 15, **caractérisé en ce que**, dans le premier étage de réaction, la transformation de cyanhydrine d'acétone et d'acide sulfurique a lieu dans au moins deux réacteurs séparés l'un de l'autre, l'acide sulfurique et la cyanhydrine d'acétone étant mis en œuvre dans le premier réacteur dans un rapport molaire d'acide sulfurique à ACH dans la plage de 1,6 à 3,0 et l'acide sulfurique et la cyanhydrine d'acétone étant mis en œuvre dans le deuxième réacteur dans un rapport molaire d'acide sulfurique à ACH dans la plage de 1,2 à 2,0.

17. Procédé selon l'une quelconque des revendications 1 à 16, **caractérisé en ce que** le mélange réactionnel de cyanhydrine d'acétone (ACH) et d'acide sulfurique dans le premier mélange réactionnel présente une quantité totale d'eau dans la plage de 0,1% en mole à 20% en mole, par rapport à la totalité de l'ACH introduite dans le premier étage de réaction.

18. Procédé selon l'une quelconque des revendications 8 à 17, **caractérisé en ce que**, dans la deuxième étape de distillation K2, on obtient comme fraction de fond un produit brut de méthacrylate d'alkyle, qui contient de préférence au moins 99% en poids de méthacrylate d'alkyle, le produit brut de méthacrylate d'alkyle étant purifié dans une autre étape de distillation K3, un produit pur de méthacrylate d'alkyle étant obtenu comme fraction de tête, lequel produit pur présente une teneur en méthacrylonitrile dans la plage de 10 à 300 ppm, par rapport au produit pur de méthacrylate d'alkyle.

19. Procédé selon l'une quelconque des revendications 1 à 18, **caractérisé en ce que** le procédé comprend une régénération de l'acide sulfurique, une partie du troisième mélange réactionnel obtenu dans le troisième étage de réaction et au moins un flux résiduaire aqueux ou organique, contenant de l'acide sulfurique, de l'hydrogénosulfate d'ammonium et des dérivés sulfonés d'acétone, qui provient du soutirage de l'hétéroazéotrope aqueux, contenant du méthacrylonitrile et de l'acétone, étant introduits dans une étape de régénération thermique, dans laquelle de l'acide sulfurique est obtenu qui est recyclé dans le premier étage de réaction.

20. Procédé selon l'une quelconque des revendications 1 à 18, **caractérisé en ce que** le procédé comprend une production de sulfate d'ammonium, une partie du troisième mélange réactionnel obtenu dans le troisième étage de réaction et au moins un flux résiduaire aqueux ou organique, contenant de l'acide sulfurique, de l'hydrogénosulfate d'ammonium et des dérivés sulfonés d'acétone, qui provient du soutirage de l'hétéroazéotrope aqueux, contenant du méthacrylonitrile et de l'acétone, étant introduits dans une étape de régénération thermique, dans laquelle du sulfate d'ammonium est obtenu par cristallisation, lequel sulfate d'ammonium est séparé en tant que produit secondaire.
